# EUROPEAN PATENT APPLICATION

(11) **EP 3 527 209 A1**
(43) Date of publication of application: **21.08.2019**
(21) Application number: 18157247.0
(22) Date of filing: 16.02.2018
(51) Int. Cl.: A61K 31/437, A61K 31/4375, A61K 31/519, A61K 31/4365, C07D 471/04, C07D 498/04, C07D 513/04, A61P 29/00

(54) **PHARMACEUTICAL 6,5 HETEROBICYCLIC RING DERIVATIVES**

(71) Applicant: UCB Biopharma SPRL, 1070 Brussels (BE)
(72) Inventor: The designation of the inventor has not yet been filed
(74) Representative: UCB Intellectual Property

(57) **Abstract**

The invention relates to 6,5 heterobicyclic ring derivatives, processes for preparing them, pharmaceutical compositions containing them and their use for the treatment of inflammatory conditions driven by STING activation, such as SLE and geographic atrophy.

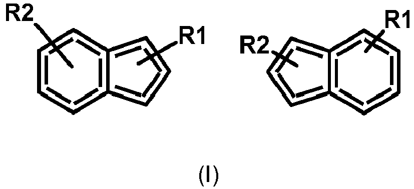

## Description

### FIELD OF THE INVENTION

The invention relates to 6,5 heterobicyclic ring derivatives, processes for preparing them, pharmaceutical compositions containing them and their use for the treatment of inflammatory conditions driven by STING activation, such as, but not confined to, SLE and geographic atrophy. 6,5 heterobicyclic ring derivatives may be useful as STING (Stimulator of Interferon Genes) antagonists that inhibit the STING pathway.

### BACKGROUND OF THE INVENTION

The response of the body to tissue damage is to mount an inflammatory response. Usually this is self-limiting and functions to remove damaged tissue, counter any infectious microorganisms and restore the tissue to normal function. This innate immune response utilises pattern recognition receptors (PRRs) that can be divided into those that recognise specific microbial products (pathogen associated molecular patterns; PAMPs) and those that recognise host molecules (damage associated molecular patterns; DAMPs). The value in responding to PAMPs is clear but responding to DAMPs is equally important as it amplifies the inflammatory response and allows infections to be controlled early before they have a chance to overwhelm the host. DNA from viruses and bacteria and self DNA (nuclear or mitochondrial) can serve as PAMPs and DAMPs respectively (Paludan SR & Bowie AG (2013). Immune sensing of DNA. Immunity, 38:870-880). A number of DNA PRRs are recognised including cyclic GMP AMP synthase (cGAS) in the cytoplasmic compartment. cGAS appears to show little discrimination between microbial or self DNA although the extent of activation depends upon the length of the DNA, its' structure and whether or not it is oxidised (Andreeva L et al (2017). cGAS Senses Long and HMGB/TFAM-bound U-Turn DNA by Forming Protein-DNA Ladders. Nature 549:394-398). In the absence of infection or tissue damage, cGAS is silent, partly because self DNA is compartmentalised into the nucleus and mitochondria, and partly because of the activity of DNase enzymes that take care of physiological low levels of DNA leakage from cells and organelles.

When cGAS binds DNA it undergoes a conformational change and acquires enzyme activity utilising ATP and GTP as substrates and producing the cyclic dinucleotide 2',3'-cGAMP as a product. 2',3'-cGAMP engages the adapter protein STING that exists as a dimer on the endoplasmic reticulum. A conformational change to STING triggers a series of events including translocation to the trans Golgi network, recruitment of the tank binding kinase, TBK1, and phosphorylation of substrates IRF3, IKK and STAT 6 leading to the type I interferon response, proinflammatory cytokines and chemokines (Li, Y (2017). Regulating STING in health and disease. Journal of Inflammation 14:11). Activation is also linked to cell death pathways for example through inflammasome activation (Gaidt MM et al (2017). The DNA Inflammasome in Human Myeloid Cells Is Initiated by a STING-Cell Death Program Upstream of NLRP3. Cell 171:1110-1124).

There are instances where artificially stimulating the cGAS STING pathway may have value. For instance, strengthening the host immune response to infection or boosting immunological responses to tumour cells. STING agonists are being developed for these purposes (Mullard A (2018). Can innate immune system targets turn up the heat on 'cold' tumours? Nat Rev Drug Discov. 17:3-5).

The reverse is also true. For whilst activation of the cGAS STING pathway is an important response to tissue injury and infection, inflammatory changes induced by excessive activation of the pathway may be detrimental. Mutations in human STING that give rise to a constitutive activation of the protein cause SAVI (STING associated vasculopathy of infants) (Liu, Y (2014). Activated STING in a Vascular and Pulmonary Syndrome. The New England Journal of Medicine, 371:507-518) with affected children having skin rash, skin ulceration and interstitial lung disease (all symptoms that can be found in severe SLE). And in SLE, measurement of 2',3'-cGAMP in peripheral blood mononuclear cells was associated with patients with higher disease scores (An J et al (2017). Expression of Cyclic GMP-AMP Synthase in Patients With Systemic Lupus Erythematosus. Arthritis Rheumatol. 69:800-807) indicating the cGAS STING pathway to be active in patients with more severe SLE and suggesting the therapeutic potential of STING inhibitors.

There are other conditions where DNA sensing pathways and the cGAS STING pathway in particular, may drive pathological changes and where STING inhibitors may have utility. Extensive tissue damage, as seen in systemic inflammatory response syndrome (SIRS) and in critical care patients, is associated with symptoms similar to sepsis that may be explained by extensive engagement of innate immune receptors by DNA derived from tissue damage (Boyapati, RK et al. (2017). Advances in the understanding of mitochondrial DNA as a pathogenic factor in inflammatory diseases. F1000Research 6: 169). And in tissues where repair processes are limited, such as the heart and the brain, the inflammatory response to tissue injury can contribute to tissue damage. Experimental evidence suggests a damaging role for the cGAS STING pathway in myocardial infarction (King, KR et al (2017). IRF3 and type I interferons fuel a fatal response to myocardial infarction. Nat. Med. 23:1481-1487) and expression of cGAS and STING in CNS cells (Jeffries AM & Marriott I (2017). Human microglia and astrocytes express cGAS-STING viral sensing components. Neurosci Lett. 658:53-56) suggests similar mechanisms could be active in stroke.

Although the inflammatory response is essential to host defence, there are instances when the vigour of the host response to an infectious microorganism is itself a problem. For example the high mortality of N5N1 infection (avian flu) is caused by the local accumulation of exudate in the lungs from damaged cells attacked by the patient's own immune response (Short KR et al (2016). Influenza virus damages the alveolar barrier by disrupting epithelial cell tight junctions. Eur Respir J. 47:954-66). In such circumstances there may be a need to limit the extent of the innate immune response and inhibitors, that may include STING inhibitors, could have value in those circumstances.

Another area receiving attention relates to tissue degeneration. Many diseases are associated with mitochondrial stress and this has been linked to the release into the cytoplasm of mitochondrial DNA that can activate the cGAS STING pathway. This mechanism leads to the death of retinal pigmented epithelial cells and causes blindness in geographic atrophy (Kerur N et al (2018). cGAS drives noncanonical-inflammasome activation in age-related macular degeneration. Nat. Med. 24:50-61). The mechanism may also be active in other neurodegenerative diseases such as Parkinson's disease, amyotrophic lateral sclerosis, Alzheimer's disease and peripheral degenerative diseases such as chondrocyte death in osteoarthritis, loss of pancreatic islet cells etc.

Thus there is a clear need to develop STING antagonists to explore their therapeutic potential in a range of human conditions with high unmet medical need.

### SUMMARY OF THE INVENTION

The present invention relates to 6,5 heterobicyclic ring derivatives, processes for preparing them, pharmaceutical compositions containing them and their use for the treatment of inflammatory conditions driven by STING activation, such as, but not confined to, SLE and geographic atrophy. A further aspect of the present invention consists of novel compounds that demonstrate the ability to functionally antagonise STING activation.

Further aspects of the invention will become apparent from the detailed specification.

### DESCRIPTION

In one aspect, the present invention relates to compounds of general formula I, or a pharmaceutically acceptable acid addition salt, a racemic mixture or its corresponding enantiomer and/or optical isomers thereof, 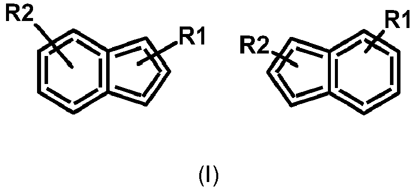 wherein
- the central core A which is the 6,5 heterobicyclic rings contains at least one heteroatom from O,N,S and C atoms can be optionally substituted by halogen, cyano, C1-6 alkyl, trifluoromethyl, difluromethyl, (C2-6) alkenyl, hydroxy, (C1-6) alkoxy, difluoromethoxy, trifluoromethoxy, trifluoroethoxy, (C1-6) alkylthio, (C1-6) alkylsulphonyl, amino, (C1-6) alkylamino, di(C1-6)alkylamino, (C1-6)alkoxy(C1-6)alkyl-amino, N-[(C1-6)alkyl]-N-[hydroxy(C1-6)alkyl]amino, (C2-6) alkylcarbonylamino, (C2-6) alkoxycarbonylamino, (C1-6) alkylsulphonylamino, formyl, (C2-6) alkylcarbonyl, carboxy, (C2-6) alkoxycarbonyl, aminocarbonyl, (C1-6) alkylaminocarbonyl, di(C1-6)alkylaminocarbonyl, aminosulphonyl, (C1-6) alkylaminosulphonyl or di(C1-6)alkylaminosulphonyl; (C3-7)heterocycloalkyl or (C3-7)spiroheterocycloalkyl, either of which groups may be optionally substituted by one or more substituents;
- R1 represents alkyl or cycloalkyl amines optionally substituted including (C1-3) aminoalkyl, (C3-7) aminocycloalkyl, (C1-3)alkylimidazole, (C1-3)alkyl isoindoline, (C1-3)alkylpiperazine, (C1-3)alkylpiperidine, (C1-3)alkyl imidazopiperazine, (C1-3)alky(C4-7)aminocycloalkyl, (C1-3)alky(C4-7)aminodicycloalkyl; and
- R2 represents aryl, heteroaryl, heterobicyclic, (C4-7) aminocycloalkyl, cycloalkyl, heterocycloalkyl, (C6-8) diaminocycloalkyl, morpholino, (C4-7)cylcoalkylmethyl, piperzinyl, piperdinyl. R2 is optionally substituted with groups including hydroxyl, (C1-6)alkyl, acetyl, halogen, cyano, C1-6 alkyl, trifluoromethyl, difluromethyl, (C2-6) alkenyl, hydroxy, (C1-6) alkoxy, difluoromethoxy, trifluoromethoxy, trifluoroethoxy, (C1-6) alkylthio, (C1-6) alkylsulphonyl, amino, (C1-6) alkylamino, di(C1-6)alkylamino, (C1-6)alkoxy(C1-6)alkylamino, N-[(C1-6)alkyl]-N-[hydroxy(C1-6)alkyl]amino, (C2-6) alkylcarbonylamino, (C2-6) alkoxycarbonylamino, (C1-6) alkylsulphonylamino, formyl, (C2-6) alkylcarbonyl, carboxy, (C2-6) alkoxycarbonyl, aminocarbonyl, (C1-6) alkylaminocarbonyl, di(C1-6)alkylaminocarbonyl, aminosulphonyl, (C1-6) alkylaminosulphonyl or di(C1-6)alkylaminosulphonyl; (C3-7)heterocycloalkyl or (C3-7)spiroheterocycloalkyl, either of which groups may be optionally substituted by one or more substituents.

Examples of the central core A are

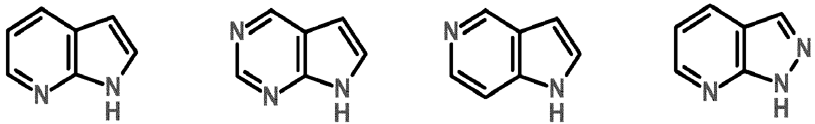

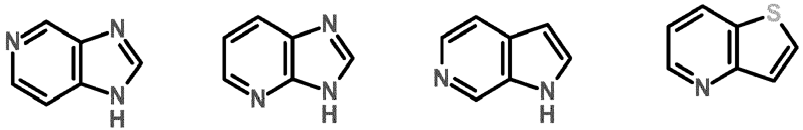

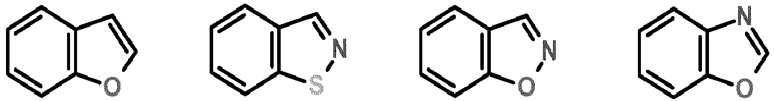

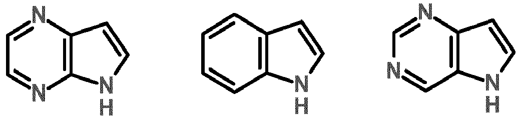

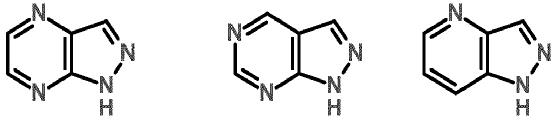

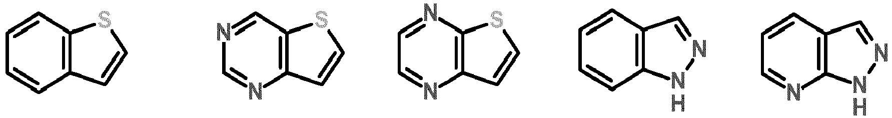

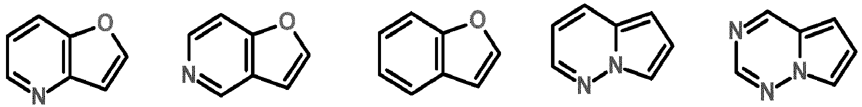

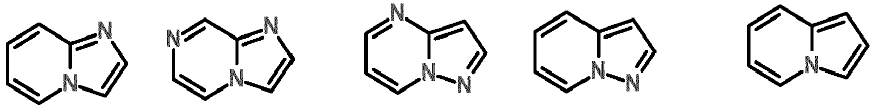

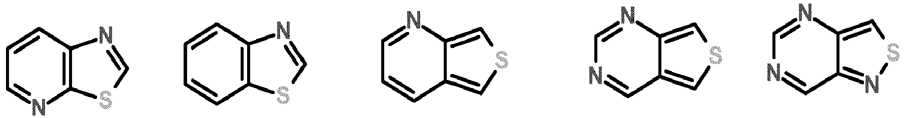

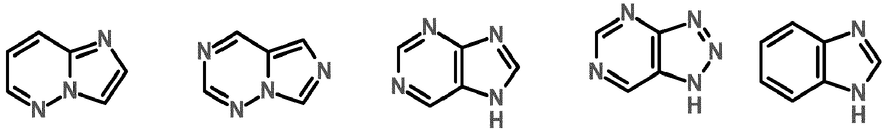

Preferably the central core A is

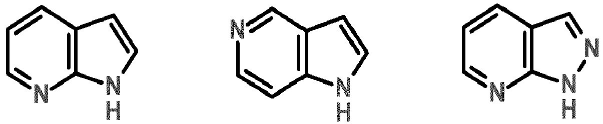

More preferred central core A is

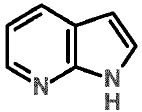

Preferably R1 is ethyl-azabicyclo[3.2.0]heptane; or ethyl-2-methyl-pyrrolidine or (C4-7) cycloalkylamines including 3-substituted-N-methyl-cyclobutanamine and piperazine.

More preferred R1 is 3-substituted-N-methyl-cyclobutanamine.
Preferably R2 is 1-substituted-4-aryl-piperazine or 1-substituted-4-heteroaryl-piperazine or 1-(4-substituted-aryl)piperazine or 1-(4-substituted-heteroaryl)piperazine or 1-substituted-4-aryl-piperidine or 1-substituted4-heteroaryl-piperidine or 1-(4-substituted-aryl)piperidine or 1-(4-substituted-heteroaryl)piperidine with optional substitution on aryl, heteroaryl, piperazine or piperidine groups.
More preferred R2 is 1-substituted-4-aryl-piperazine or 1-substituted-4-heteroaryl-piperazine, 1-substituted-4-aryl-piperidine or 1-substituted-4-heteroaryl-piperidine.

In a specific embodiment, compounds of formula I are those wherein:
- the central core A is 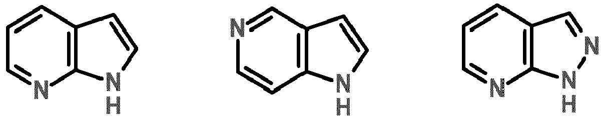
- R1 is ethyl-azabicyclo[3.2.0]heptane; or ethyl-2-methyl-pyrrolidine or (C4-7) cycloalkylamines including 3-substituted-N-methyl-cyclobutanamine and piperazine;
- R2 is 1-substituted-4-aryl-piperazine or 1-substituted-4-heteroaryl-piperazine or 1-(4-substituted-aryl)piperazine or 1-(4-substituted-heteroaryl)piperazine or 1-substituted-4-aryl-piperidine or 1-substituted4-heteroaryl-piperidine or 1-(4-substituted-aryl)piperidine or 1-(4-substituted-heteroaryl)piperidine with optional substitution on aryl, heteroaryl, piperazine or piperidine groups.
In a further specific embodiment, compounds of formula I are those wherein:
- the central core A is 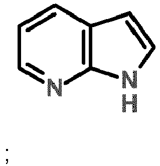
- R1 is 3-substituted-N-methyl-cyclobutanamine;
- R2 is 1-substituted-4-aryl-piperazine or 1-substituted-4-heteroaryl-piperazine, 1-substituted-4-aryl-piperidine or 1-substituted-4-heteroaryl-piperidine.
Specific compounds of the present invention are those selected from the group consisting of: 6-[(2S)-2-methyl-4-(2-methylphenyl)piperazin-1-yl]-1-[2-(pyrrolidin-1-yl)ethyl]-1H-pyrrolo[2,3-b]pyridine,
6-{4-[3-(methylsulfonyl)phenyl]piperazin-1-yl}-1-[2-(pyrrolidin-1-yl)ethyl]-1H-pyrrolo[2,3-b]pyridine,
6-[4-(4-acetylphenyl)piperazin-1-yl]-1-[trans-3-(methylamino)cyclobutyl]-1H-pyrrolo[2,3-b]pyridine-3-carbonitrile,
5-(4-{1-[2-(6-azabicyclo[3.2.0]hept-6-yl)ethyl]-1H-pyrrolo[2,3-b]pyridin-6-yl}piperazin-1-yl)imidazo[1,2-a]pyridine,
6-{4-[4-(methylsulfonyl)phenyl]piperazin-1-yl}-1-[2-(pyrrolidin-1-yl)ethyl]-1H-pyrrolo[2,3-b]pyridine-3-carbonitrile,
N-methyl-3-[6-[4-(4-pyridin-2-ylphenyl)piperazin-1-yl]pyrrolo[2,3-b]pyridin-1-yl]cyclobutan-1-amine,
N-methyl-3-[6-[4-(4-pyridin-2-ylphenyl)piperazin-1-yl]pyrrolo[2,3-b]pyridin-1-yl]cyclobutan-1-amine.

The following paragraphs provide definitions of the various chemical moieties that make up the compounds according to the invention and are intended to apply uniformly throughout the specification and claims unless an otherwise expressly set out definition provides a broader definition.

As used herein, the term "C1-C6 alkyl" refers to a saturated, aliphatic hydrocarbon group including a straight or branched carbon chain with 1 - 6 carbon atoms. Examples for "alkyl" are methyl, ethyl, n-propyl, and isopropyl.

The term "C1-C6 alkoxy" refers to a group -O-R' wherein R' is C1-C6 alkyl as defined above. The term "halogen" refers to fluorine, chlorine, bromine or iodine.

The term "C1-C6 alkyl substituted by halogens or hydroxy or C1-C6 alkoxy" refers to an alkyl group as defined above, wherein at least one hydrogen atom is replaced by a halogen atom, a hydroxyl or a C1-C6 alkoxy.

The term "C1-C6 alkoxy substituted by halogens or hydroxy or C1-C6 alkoxy" refers to an alkoxy group as defined above, wherein at least one hydrogen atom is replaced by a halogen atom, a hydroxyl or a C1-C6 alkoxy.

The term "heterocyclyl" refers to a saturated ring, containing 1-3 heteroatoms, selected from N, O or S, for example morpholinyl, piperazinyl, piperidinyl or pyrrolidinyl or azetidinyl.

The term "pharmaceutically acceptable salt" or "pharmaceutically acceptable acid addition salt" according to the invention embraces therapeutically active, non-toxic acid or base salt forms which the compounds of formula **I** are able to form.

The acid addition salt form of a compound of formula **I** that occurs in its free form as a base can be obtained by treating the free base with an appropriate acid such as an inorganic acid, for example, a hydrohalic such as hydrochloric or hydrobromic, sulfuric, nitric, phosphoric and the like; or an organic acid, such as, for example, acetic, trifluoroacetic, oxalic, hydroxyacetic, propanoic, lactic, pyruvic, malonic, succinic, maleic, fumaric, malic, tartaric, citric, methanesulfonic, ethanesulfonic, benzenesulfonic, p-toluenesulfonic, cyclamic, salicylic, p-aminosalicylic, pamoic and the like.

The invention also relates to all stereoisomeric forms such as enantiomeric and diastereoisomeric forms of the compounds of formula **I** or mixtures thereof (including all possible mixtures of stereoisomers).

With respect to the present invention reference to a compound or compounds is intended to encompass that compound in each of its possible isomeric forms and mixtures thereof, unless the particular isomeric form is referred to specifically.

Some of the compounds of formula I may also exist in tautomeric forms. Such forms although not explicitly indicated in the above formula are intended to be included within the scope of the present invention.

It is to be understood that each individual atom present in formula (I), or in the formulae depicted hereinafter, may in fact be present in the form of any of its naturally occurring isotopes, with the most abundant isotope(s) being preferred. Thus, by way of example, each individual hydrogen atom present in formula (I), or in the formulae depicted hereinafter, may be present as a 1 H, 2H (deuterium) or 3H (tritium) atom, preferably 1H. Similarly, by way of example, each individual carbon atom present in formula (I), or in the formulae depicted hereinafter, may be present as a 12C, 13C or 14C atom, preferably 12C.

Another embodiment of the present invention concerns a pharmaceutical composition comprising a detectable amount of a compound of formula **I** or a pharmaceutically acceptable salt thereof in combination with a pharmaceutically acceptable diluent or carrier.

In another embodiment, the present invention concerns a compound as listed above for use as a medicament.

In a specific embodiment, the present invention concerns a compound as listed above for use as a medicament in the treatment of inflammatory conditions driven by STING, such as SLE (Systemic lupus erythematosus) and geographic atrophy.

In another embodiment, the present invention concerns a pharmaceutical composition containing a compound as listed above as well as pharmaceutically acceptable excipients.

In another embodiment, the invention concerns the synthesis of intermediates, acid addition salt, a racemic mixture or its corresponding enantiomer and/or optical isomers thereof.

In another embodiment, the present invention concerns synthesis intermediates of general formula II 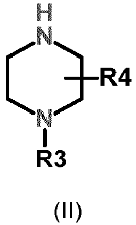 wherein R4 represents (C1-6)alkyl, (C1-6)alkoxy or oxo and R3 represents optionally substituted aryl or heteroaryl.

In another embodiment, the present invention concerns synthesis intermediates of general formula III 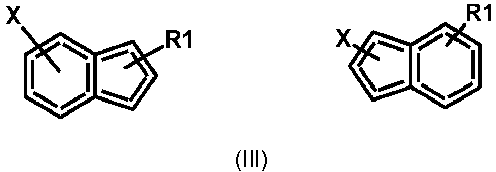 wherein
- X represents a halogen (Br, Cl, I) suitable for cross-coupling with intermediates of formula (II) or cross-coupling with an aryl or heteroaryl boronic acid derivative ; and
- R1 represents alkyl or cycloalkyl amines optionally substituted including (C1-3) aminoalkyl, (C3-7) aminocycloalkyl, (C1-3)alkylimidazole, (C1-3)alkyl isoindoline, (C1-3)alkylpiperazine, (C1-3)alkylpiperidine, (C1-3)alkyl imidazopiperazine, (C1-3)alky(C4-7)aminocycloalkyl, (C1-3)alky(C4-7)aminodicycloalkyl.

In another embodiment, the present invention concerns synthesis intermediates of general formula IV 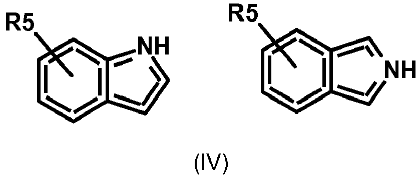 wherein R5 can be either X or R2 .

In another embodiment, the present invention concerns synthesis intermediates of general formula V 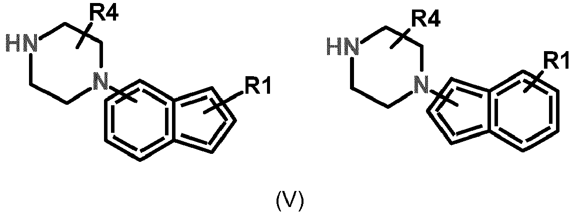 wherein
- R4 represents (C1-6)alkyl, (C1-6)alkoxy or oxo ; and
- R1 represents alkyl or cycloalkyl amines optionally substituted including (C1-3) aminoalkyl, (C3-7) aminocycloalkyl, (C1-3)alkylimidazole, (C1-3)alkyl isoindoline, (C1-3)alkylpiperazine, (C1-3)alkylpiperidine, (C1-3)alkyl imidazopiperazine, (C1-3)alky(C4-7)aminocycloalkyl, (C1-3)alky(C4-7)aminodicycloalkyl.

In another embodiment, the present invention concerns synthesis intermediates of general formula VI 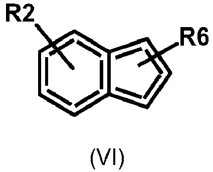 wherein
- R6 represents an alkyl or cycloalkyl substituent bearing a functional group suitable for displacement by an amine, for example 4-methylbenzenesulfonate; and
- R2 represents aryl, heteroaryl, heterobicyclic, (C4-7) aminocycloalkyl, cycloalkyl, heterocycloalkyl, (C6-8) diaminocycloalkyl, morpholino, (C4-7)cylcoalkylmethyl, piperzinyl, piperdinyl. R2 is optionally substituted with groups including hydroxyl, (C1-6)alkyl, acetyl, halogen, cyano, C1-6 alkyl, trifluoromethyl, difluromethyl, (C2-6) alkenyl, hydroxy, (C1-6) alkoxy, difluoromethoxy, trifluoromethoxy, trifluoroethoxy, (C1-6) alkylthio, (C1-6) alkylsulphonyl, amino, (C1-6) alkylamino, di(C1-6)alkylamino, (C1-6)alkoxy(C1-6)alkylamino, N-[(C1-6)alkyl]-N-[hydroxy(C1-6)alkyl]amino, (C2-6) alkylcarbonylamino, (C2-6) alkoxycarbonylamino, (C1-6) alkylsulphonylamino, formyl, (C2-6) alkylcarbonyl, carboxy, (C2-6) alkoxycarbonyl, aminocarbonyl, (C1-6) alkylaminocarbonyl, di(C1-6)alkylaminocarbonyl, aminosulphonyl, (C1-6) alkylaminosulphonyl or di(C1-6)alkylaminosulphonyl; (C3-7)heterocycloalkyl or (C3-7)spiroheterocycloalkyl, either of which groups may be optionally substituted by one or more substituents.

In another embodiment, the present invention concerns synthesis intermediates of general formula VII 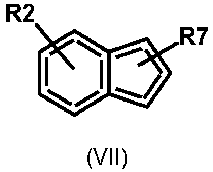 wherein
- R7 represents an primary or secondary amine ; and
- R2 represents aryl, heteroaryl, heterobicyclic, (C4-7) aminocycloalkyl, cycloalkyl, heterocycloalkyl, (C6-8) diaminocycloalkyl, morpholino, (C4-7)cylcoalkylmethyl, piperzinyl, piperdinyl. R2 is optionally substituted with groups including hydroxyl, (C1-6)alkyl, acetyl, halogen, cyano, C1-6 alkyl, trifluoromethyl, difluromethyl, (C2-6) alkenyl, hydroxy, (C1-6) alkoxy, difluoromethoxy, trifluoromethoxy, trifluoroethoxy, (C1-6) alkylthio, (C1-6) alkylsulphonyl, amino, (C1-6) alkylamino, di(C1-6)alkylamino, (C1-6)alkoxy(C1-6)alkylamino, N-[(C1-6)alkyl]-N-[hydroxy(C1-6)alkyl]amino, (C2-6) alkylcarbonylamino, (C2-6) alkoxycarbonylamino, (C1-6) alkylsulphonylamino, formyl, (C2-6) alkylcarbonyl, carboxy, (C2-6) alkoxycarbonyl, aminocarbonyl, (C1-6) alkylaminocarbonyl, di(C1-6)alkylaminocarbonyl, aminosulphonyl, (C1-6) alkylaminosulphonyl or di(C1-6)alkylaminosulphonyl; (C3-7)heterocycloalkyl or (C3-7)spiroheterocycloalkyl, either of which groups may be optionally substituted by one or more substituents.

### SYNTHETIC METHODS

The compounds of formula I according to the invention can be prepared analogously to conventional methods as understood by the person skilled in the art of synthetic organic chemistry.

According to one embodiment, some compounds of general formula I may be prepared by reacting intermediate (II) with intermediate (III) under Buchwald cross-coupling conditions with a suitable transition metal catalyst and base. Compounds of general formula (I) may also be prepared by reacting intermediate (III) with an approporiate aryl or heteroaryl boronic acid or boronic ester under Suzuki cross-coupling conditions. Alternatively, some compounds of general formula (I) may be prepared by reacting intermediate (IV) with a suitable R1 group bearing either a halogen (Cl, Br,I) for direct alkylation or an alcohol for Mitsunobu coupling. In this instance if R5 is a halogen in intermediate (IV), Buchwald or Suzuki cross-couping with a suitable R2 group bearing eitheran amine, boronic acid or boronic ester will also be required to yield compounds of formula (I).

Compounds of general formula (I) may also be prepared from intermediate (V) by Buchwald cross-coupling with a suitable arylhalide or heteroaryl halide.

Compounds of general formula (I) may also be prepared from intermediate (V) by amide bond formation or urea formation by reaction with a suitable acid, acid chloride or isocyanate under appropriate coupling conditions.

Alternatively, compounds of general formula (I) may also be prepared from intermediate (VI) by amine displacement of a leaving group on the R6 substituent.

Compounds of general formula (I) may also be prepared from intermediate (VII) by reductive amination involving condensation with an aldehyde or ketone followed by reduction of the resulting imine.

### EXAMPLES

The following examples illustrate how the compounds covered by formulas I and II may be synthesized. They are provided for illustrative purposes only and are not intended, nor should they be construed, as limiting the invention in any manner. Those skilled in the art will appreciate that routine variations and modifications of the following examples can be made without exceeding the spirit or scope of the invention.

### Abbreviations

BOC: tert-butyloxycarbonyl QC: quality control
DCM: dichloromethane EtOAc: ethyl acetate
DMF: *N,N-*dimethylformamide MeOH: methanol
DMSO: dimethylsulfoxide EtOH: ethanol
Et₂O: diethyl ether MeCN: acetonitrile
THF: tetrahydrofuran DIPEA: *N,N-*diisopropylethylamine
H₂O: water LDA: lithium diisopropylamide
NH₃: ammonia Pd(OAc)₂: palladium(II) acetate
MgSO₄: magnesium sulphate Na₂SO₄: sodium sulphate
K₂CO₃: potassium carbonate NBS: N-bromosuccinimide
eq.: equivalents DBU: 1,8-diazabicyclo[5.4.0]undec-7-ene
TFA: trifluoroacetic acid DME: 1,2-dimethoxyethane
pTSA: *p*-toluenesulfonic acid TBAF: tetra-*n*-butylammonium fluoride
min.: minutes h: hour
r.t.: room temperature RT: retention time
br: broad M: molar
N: Normal ES+: Electrospray Positive Ionisation
SCX: solid phase cation exchange N₂: nitrogen
HPLC: High Performance Liquid Chromatography
LCMS: Liquid Chromatography Mass Spectrometry
brine: aqueous sodium chloride solution
PdCl₂(dppf)-DCM: [1,1'-bis(diphenylphosphino)ferrocene]dichloropalladium(II)
Pd-Ruphos G3: (2-Dicyclohexylphosphino-2',6'-diisopropoxy-1,1'-biphenyl)[2-(2'-amino-1,1'-biphenyl)]palladium(II) methanesulfonate
BINAP: (2,2'-bis(diphenylphosphino)-1,1'-binaphthyl)
Pd₂dba₃: Tris(dibenzylideneacetone)dipalladium(0)
HATU: 1-[Bis(dimethylamino)methylene]-1H-1,2,3-triazolo[4,5-b]pyridinium 3-oxid hexafluorophosphate

### Nomenclature

Compounds were named with the aid of ACD-ConsoleVersion 14.03 and/or Biovia Draw 2016.

### Analytical Conditions

All NMRs were obtained either at 300 MHz or at 400 MHz.

All reactions involving air- or moisture-sensitive reagents were performed under a nitrogen atmosphere using dried solvents and glassware unless otherwise noted.

Except where otherwise stated, analytical LCMS data were obtained by using *Method 1, 2, 3, 4* or 5 below.

All quoted LCMS Retention Time (RT) and QC Retention Time (RT) values are in minutes.

### Method 1:

### pH 10

### Gradient:

| Time | A% | B% |
|---|---|---|
| 0.00 | 95.00 | 5.00 |
| 4.00 | 5.00 | 95.00 |
| 5.00 | 5.00 | 95.00 |
| 5.10 | 95.00 | 5.00 |

| | |
|---|---|
| Flow: | 1 mL/min |
| Solvent A: | 10 mM Ammonium Formate in water + 0.1 % Ammonia Solution |
| Solvent B: | Acetonitrile + 5 % water + 0.1 % Ammonia Solution |
| Column: | XBridge C18, 2.1 x 20 mm, 2.5 µm |

### Method 2:

### pH 10

### Gradient:

| Time | A% | B% |
|---|---|---|
| 0.00 | 95.00 | 5.00 |
| 1.50 | 5.00 | 95.00 |
| 2.25 | 5.00 | 95.00 |
| 2.50 | 95.00 | 5.00 |

### Flow 1 mL/min

Solvent A: 10 mM Ammonium Formate in water + 0.1% Ammonia Solution
Solvent B: Acetonitrile + 5 % water + 0.1% Ammonia Solution
Column: XBridge C18, 2.1 x 20 mm, 2.5 □m

### Method 3:

### pH 3

### Gradient:

| Time | A% | B% |
|---|---|---|
| 0.00 | 98.00 | 2.00 |
| 0.3 | 98.00 | 2.00 |
| 3.00 | 5.00 | 95.00 |
| 4.00 | 5.00 | 95.00 |
| 4.10 | 98.00 | 2.00 |
| 5.10 | 98.00 | 2.00 |

### Flow 0.8 mL/min

Solvent A: Water + Acetonitrile + Formic Acid (95/5/750µl/L)
Solvent B: Water + Acetonitrile + Formic Acid (5/95/500µl/L)
Column: Acquity UPLC HSS T3 C18 column (1.8µm, 2.1 x 50 mm)

### Method 4:

### pH 10

### Gradient:

| Time | A% | B% |
|---|---|---|
| 0.0 | 95 | 5 |
| 0.1 | 95 | 5 |
| 2.6 | 5 | 95 |
| 2.75 | 5 | 95 |
| 2.8 | 95 | 5 |
| 3.0 | 95 | 5 |

### Flow: 1 mL/min

Solvent A = 10 mM Ammonium formate + 0.1 % Ammonia Solution (pH10)
Solvent B = MeCN + 5 % H2O + 0.1 % Ammonia Solution (pH10)
Column: Waters XBridge BEH C18 XP 2.5 µm 2.1 x 50 mm

### Method 5:

HPLC: Shimadzu Analytical 10Avp series with quadroon pump and UV-detector SPD-20AV (190 to 360 nm),
autosampler: Gilson 215, ELSD (evaporative light scattering detector): Sedex 75,
mass-spectrometer: PE SCIEX API 150.

Data are acquired in a full MS scan from m/z 80 to 1000 in positive mode with an acidic elution and both in positive and negative modes with a basic elution. Full flow in MS.
XBridge C18 column (3.5 µm, 100 x 4.6 mm);
detection - 220 nm, 254 nm
sample injection, uL -3.0 - 5.0
solvent A - Ammonium Formate (HCOONH4) in water 630 mg/L + 500 µL/L NH4OH 30 % (pH ∼ 8.5);
solvent B - acetonitrile;
- flow rate - 1,0 mL/min;
- UV detection wave-length, nM - 220, 254;
- mass range, m/z - 100... 1000 positive mode

### General Procedure 1

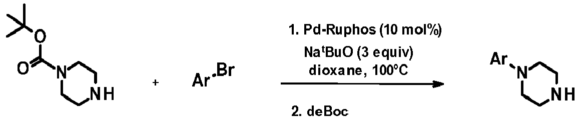

A mixture of 1-BOC-piperazine (1 equiv.), sodium tert-butoxide (1.5 - 3 equiv.), arylhalide (1.2 equiv.) and Pd-Ruphos G3 (0.1 equiv.) were dissolved in pre-degassed 1,4-dioxane (0.15 - 0.3 M). The reaction mixture was then heated at 100 °C until complete. The reaction mixture was cooled to ambient temperature and then either treated with an aqueous work up or with filtration through celite. The solvent was dried (using either MgSO₄, Na₂SO₄ or a phase separator) and subsequently removed under vacuum. The residue was purified by flash silica chromatography to give the product or used directly in the next step. The product was then dissolved in DCM (4-5 mL) and TFA (0.5 - 1 ml) added (either at 0 °C or at room temperature). The reaction mixture was stirred at ambient temperature until complete (LCMS monitoring). Then, the reaction was filtered through an SCX cartridge eluting first with methanol then with ammonia (7 M, 4 M or 2 M) in methanol. The ammonia in methanol solution was concentrated under reduced pressure to give the desired product.

### General Procedure 2

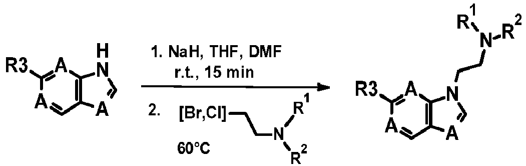

The appropriate heterocycle (1 equiv.) was dissolved in a mixture of DMF and THF (1:1 or 1:1.5 respectively, 0.2 M). The solution was cooled to 0 °C and sodium hydride (1.2 or 2.2 equiv.) carefully added portion wise. After approximately 5 min the alkyl halide (1.2 equiv.) was carefully added portion-wise. The reaction mixture was stirred at 0 °C or at RT for 15 min. to an hour then heated at 80 °C for a period ranging between 30 min to 6 h. The reaction mixture was cooled to 0 °C and quenched with water and treated with EtOAc and the organic layer separated. The organic solvent was dried (using either MgSO₄, Na₂SO₄ or a phase separator) and subsequently removed under vacuum. The residue was purified by flash silica column chromatography or reverse phase column chromatography to give the product.

### General Procedure 3

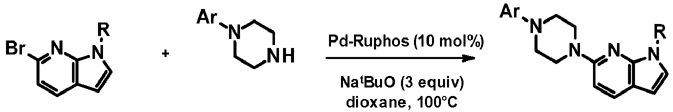

A mixture of the heterocycle (1 eq.), amine (1.2 eq.), Pd-Ruphos G3 (0.1 eq.) and sodium tert-butoxide (1.5-3 eq.) was dissolved in pre-degassed 1,4-dioxane (0.1 - 0.2 M). The reaction mixture was then heated between 90 °C -110 °C until reaction was complete. The reaction mixture was cooled to ambient temperature and filtered through celite or treated with an aqueous work up. The solvent was dried (using either MgSO₄, Na₂SO₄ or a phase separator) and subsequently removed under vacuum. Residues were then either purified by filtration through an SCX cartridge (washing first with methanol then eluting with approx. 2 M ammonia in methanol) followed by reverse phase column chromatography or directly purified by reverse phase column chromatography to give the desired product.

### General Procedure 4

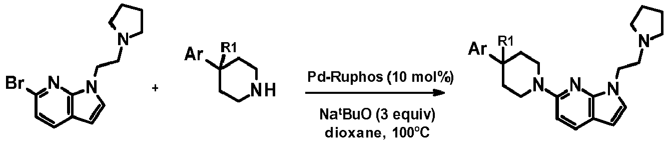

6-bromo-1-(2-pyrrolidin-1-ylethyl)pyrrolo[2,3-b]pyridine (100 mg, 0.3 mmol), sodium tert-butoxide (3 equiv., 98 mg, 1 mmol), Pd-Ruphos G3 (0.1 eq., 0.03399 mmol), and the corresponding piperidine (1.2 eq., 0.34 mmol) were dissolved in degassed 1,4-dioxane (0.05 M, 7 mL). The reaction mixture was stirred at 100 °C overnight. The reaction was then cooled down to ambient temperature and filtered through a cartridge of celite, concentrated under vacuum, dissolved in DMSO and subjected to reverse phase column chromatography.

### General Procedure 5

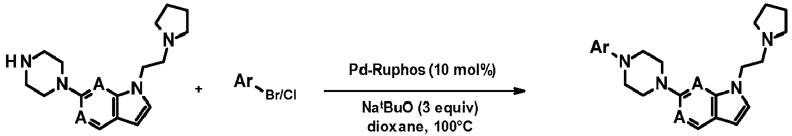

A mixture of Intermediate 7 (1 eq.), the appropriate aryl bromide or aryl chloride (approx. 1.2 or 1.5 eq.), sodium tert-butoxide (2.5-3 eq.) and Pd-Ruphos G3 (0.10 or 0.15 eq.), was suspended in pre-degassed anhydrous 1,4-dioxane. The reaction mixture was then stirred at 100 °C overnight and cooled to room temperature. Small scale reactions (0.05 mmol) were filtered and diluted with DMF (0.6 mL) and purified directly by reverse phase column chromatography to yield the desired product. Larger scale reactions (> 0.05 mmol) were treated with an aqueous work up and purified by the following sequence: filtration though celite followed by filtration through an SCX cartridge washing first with methanol then eluting with 2 M ammonia in methanol. The ammonia in methanol solution was concentrated under reduced pressure and the residue purified by reverse phase column chromatography.

### General Procedure 6

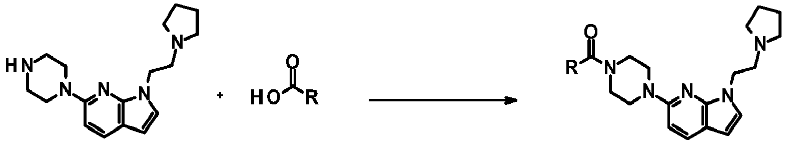

The appropriate carboxylic acid (0.05 mmol, 1 eq.) was added to a solution of Intermediate 7 (15 mg, 0.05 mmol, 1 eq.) and 2,4,6-tripropyl-1,3,5,2,4,6-trioxatriphosphorinane-2,4,6-trioxide (38 mg, 0.06 mmol, 1.20 eq) in DCM (0.5 mL). The reaction mixture was stirred at r.t. overnight. Reaction mixture filtered and diluted with DMF (0.45 mL) and purified by reverse phase column chromatography to yield the desired product.

### General Procedure 7

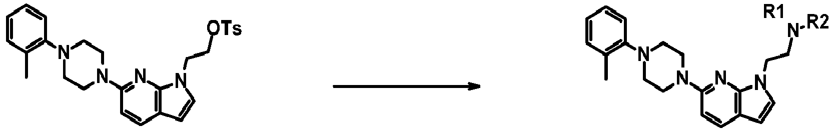

The appropriate amine (1.4 eq.), potassium carbonate (6 eq.) and Intermediate 20 (1.0 eq.) in 1,4-dioxane were stirred at 100 °C over a period of 6-12 hours. Small scale reactions (0.05 mmol) were filtered and purified by preparative HPLC to yield the desired product. Larger scale reactions (> 0.08 mmol) were passed through an SCX cartridge washing first with MeOH then eluting with approx. 2 M NH₃ in MeOH. The ammonia in methanol solution was concentrated under reduced pressure and the crude residue purified by reverse phase HPLC to yield the desired product.

### General Procedure 8

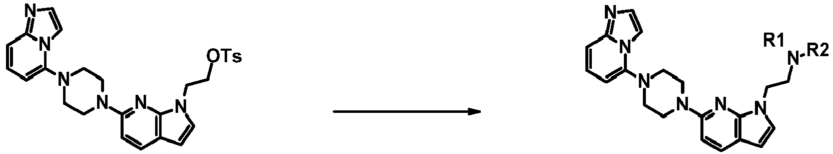

A mixture of the appropriate amine (1.5 eq.) and Intermediate 18 (20 mg, 0.03 mmol, 1 eq.) in acetonitrile and triethylamine (0.02 mL, 0.14 mmol, 4 eq.) were stirred at 80°C overnight. The reaction mixture was filtered and purified by HPLC to yield the desired product.

### General Procedure 9

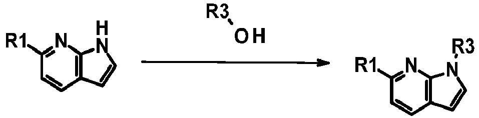

A mixture of the alcohol (1.5 eq.) and heterocycle (1 eq.) was dissolved in toluene (0.25 M). Cyanomethylenetributylphophorane (1.5 eq) was then added and the reaction mixture heated at 90 °C for 3-12 hours. The crude reaction mixture was loaded onto a silica column and eluted with a gradient of ethyl acetate in hexane to give the desired product.

### General Procedure 10

The corresponding arylbromide (1 eq.), boronic acid (1.5 eq.), K₂CO₃ (2 eq.) were dissolved in 1,4-dioxane (0.1 M). H₂O (1 M) was added and the mixture degassed before PdCl₂(dppf)-DCM (0.01 eq.) was added and heated at 90°C until the starting material was consumed. The reaction mixture was cooled to ambient temperature and EtOAc and H₂O were added. The layers were separated and the organic layer dried over Na₂SO₄. The solvent was removed under vacuum and the residue purified by flash silica column chromatography fusing hexane to EtOAc as eluent to afford the desired product.

### General Procedure 11

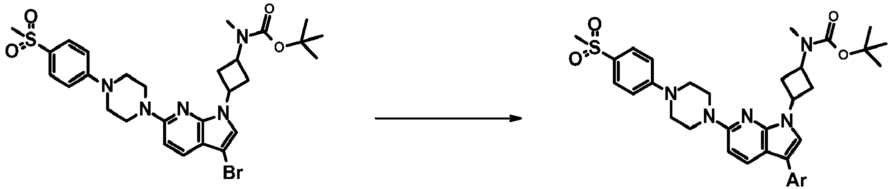

Intermediate 26 (70 mg, 0.11 mmol), boronic ester (2 eq., 0.22 mmol), K₂CO₃ (2 eq., 0.22 mmol) and PdCl₂(dppf)-DCM complex (0.1 equiv., 0.01132 mmol) were dissolved in 1,4-dioxane (0.1 M, 1 mL). H₂O (0.1 mL) was added and the mixture was degassed for 1 min. The mixture was stirred overnight at 100°C. The mixture was then cooled to ambient temperature and DCM (3 mL) and H₂O (2 mL) were added and the layers separated. TFA (1 mL) was added to the DCM layer and the reaction mixture was stirred at room temperature for 2 h. reaction mixture was filtered through an SCX column, rinsed with MeOH and DCM, and the product eluted with NH₃ (7N in MeOH). The volatiles were removed under vacuum and the residue was purified by reverse phase HPLC to afford the desired product.

### General Procedure 12

The amine (1 eq.) was dissolved in a 1:1 mixture of tetrahydrofuran and ethanol (2 mL). The aldehyde (10 eq.) was then added, followed by acetic acid (10 eq.) and sodium triacetoxyborohydride (4 eq.). The reaction mixture was stirred at room temperature overnight then concentrated under reduced pressure, dissolved in DCM and washed with aq. sodium hydroxide (2 M). The organic solvent was separated, dried (Na₂SO₄) and concentrated under reduced pressure. The residue was then purified by reverse phase column chromatography to give the desired product.

### General Procedure 13

Boc-protected amine was dissolved in DCM (4-5 mL) and TFA (0.5-1 mL) added. The reaction mixture was the stirred until completion. The crude reaction mixture was then purified by filtration through SCX washing first with methanol then eluting with approx. 2 M ammonia in methanol. The ammonia in methanol solution was concentrated under reduced pressure to give the desired product.

### General Procedure 14

A mixture of amine (1.0-1.5 equiv.), sodium tert-butoxide (1.4 equiv.), arylhalide (1.0 equiv.) and BINAP (0.15 equiv.) were dissolved in 1,4-dioxane (0.2 M) and the mixture was evacuated and backfilled with N₂ three times to degas the solvent. Pd₂dba₃ (0.05 equiv.) was then added and the reaction mixture heated at 80-100 °C until complete. The reaction mixture was cooled to r.t. and then either treated with an aqueous work up or with filtration through Celite. The solvent was dried (using either MgSO₄, Na₂SO₄ or a phase separator) and subsequently removed under vacuum. The residue was purified by flash silica chromatography to give the product or used directly in the next step.

### INTERMEDIATE 1

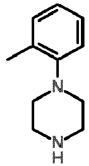

### 1-(o-tolyl)piperazine

1-(o-tolyl)piperazine was prepared following General Procedure 1 using 1-BOC-piperazine (6 g, 32 mmol) and 2-bromotoluene (5 mL, 41 mmol) to afford intermediate 1 (7.2 g, 82% yield)
LCMS (Method 2, ES⁺) 1.56 min, 177 *m*/*z* (M+H)⁺.

### INTERMEDIATE 2

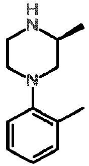

### (3S)-3-methyl-1-(o-tolyl)piperazine

(3S)-3-methyl-1-(o-tolyl)piperazine was prepared following General Procedure 1 using 2-bromotoluene (170 mg, 1 mmol) and (S)-4-N-Boc-2-methylpiperazine (240 mg, 1.2 eq., 1.2 mmol) to afford intermediate 2 (110 mg, 47% yield).
LCMS (Method 2, ES⁺) 1.12 min, 191 *m*/*z* (M+H)⁺*.*

### INTERMEDIATE 3

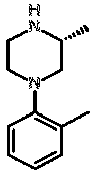

### (3R)-3-methyl-1-(o-tolyl)piperazine

(3R)-3-methyl-1-(o-tolyl)piperazine was prepared following General Procedure 1 using 2-bromotoluene (170 mg, 1 mmol) and (R)-4-N-Boc-2-methylpiperazine (240 mg, 1.2 eq., 1.2 mmol) to afford intermediate 3 (122 mg, 52% yield).
LCMS (Method 2, ES⁺) 1.10 min, 191 *m*/*z* (M+H)⁺*.*

### INTERMEDIATE 4

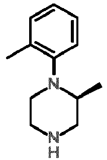

### (2S)-2-methyl-1-(o-tolyl)piperazine

(2S)-2-methyl-1-(o-tolyl)piperazine was prepared following General Procedure 1 using 2-bromotoluene (170 mg, 1 mmol) and (R)-1-N-Boc-2-methylpiperazine (240 mg, 1.2 eq., 1.2 mmol) to afford intermediate 4 (180 mg, 77% yield).
LCMS (Method 2, ES⁺) 1.08 min, 191 *m*/*z* (M+H)⁺*.*

### INTERMEDIATE 5

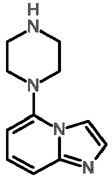

### 5-piperazin-1-ylimidazo[1,2-a]pyridine

5-piperazin-1-ylimidazo[1,2-a]pyridine was prepared following General Procedure 1 using 5-bromoimidazo[1,2-A]pyridine (5 g, 25 mmol) and 1-boc-piperazine (4.7 g, 25 mmol) to afford intermediate 5 (1.1 g, 55% yield).
LCMS (Method 2, ES⁺) 0.35 min, 203 *m*/*z* (M+H)⁺*.*

### INTERMEDIATE 6

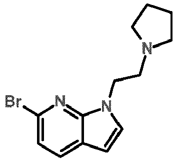

### 6-bromo-1-(2-pyrrolidin-1-ylethyl)pyrrolo[2,3-b]pyridine

6-bromo-1-(2-pyrrolidin-1-ylethyl)pyrrolo[2,3-b]pyridine was prepared following General Procedure 2 using 6-bromo-1H-pyrrolo[2,3-b]pyridine (10 g, 50 mmol) and 1-(2-chloroethyl)pyrrolidine hydrochloride (11 g, 63.4 mmol). The reaction was heated to 80 °C for 2 hours. The residue was purified by flash silica column chromatography using gradient elution from hexane to 1:1 hexane:EtOAc to afford the intermediate 6 (12 g, 82% yield).
LCMS (Method 2, ES⁺) 1.30 min, 295 & 297 *m*/*z* (M+H)⁺*.*

### INTERMEDIATE 7

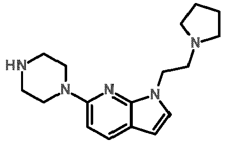

### 6-piperazin-1-yl-1-(2-pyrrolidin-1-ylethyl)pyrrolo[2,3-b]pyridine

Intermediate 6 (7 g, 23.8 mmol) was treated according to General Procedure 1 followed by General Procedure 13 to afford Intermediate 7 (4.5 g, 67% yield).
LCMS (Method 2, ES⁺) 1.02 min, 300 *m*/*z* (M+H)⁺*.*

### INTERMEDIATE 8

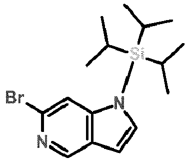

### 6-bromopyrrolo[3,2-c]pyridin-1-yl)-triisopropyl-silane

6-Bromo-1H-pyrrolo[2,3-b]pyridine (4 g, 20.3 mmol) was dissolved in 1,4-dioxane (100 mL) and DIPEA (11 mL, 63.1 mmol) added followed by triisopropylsilyl trifluromethanesulfonate (6.5 mL, 24 mmol). The reaction mixture was then stirred at r.t. for 30 min. before it was heated at 80°C overnight. Triisopropylsilyl trifluromethanesulfonate (1.2 equiv., 24.4 mmol) was added and stirred at 80°C for an additional 3 hours. The reaction was cooled down to ambient temperature and NH₄Cl aq. solution and EtOAc were added. The layers were separated and the aq. phase was extracted with EtOAc. The combined organic layers were dried over MgSO₄ and the solvent was removed under vacuum. The residue was purified by flash chromatography using gradient elution from hexane to a 1:1 mixture hexane:EtOAc to yield Intermediate 8 (5.2 g, 72% yield).
LCMS (Method 2, ES⁺) 1.96 min, 353 & 355 *m*/*z* (M+H)⁺*.*

### INTERMEDIATE 9

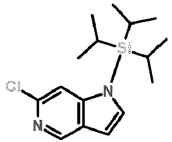

### (6-chloropyrrolo[3,2-c]pyridin-1-yl)-triisopropyl-silane

6-Chloro-5-azaindole (1 g, 6.42 mmol) was partially suspended in 1,4-dioxane (30 mL). DIPEA (3.5 mL, 20 mmol) was then added followed by triisopropylsilyl trifluoromethanesulfonate (2.5 mL, 9.3 mmol). The reaction mixture was stirred at room temperature for 5 h 30 min and then treated with an aq. work up. The organic solvent was dried (Na₂SO₄) and concentrated under reduced pressure to give a residue that was purified by flash silica column chromatography to give the title compound (1.68 g, 84%) which was used crude in the next step.

### INTERMEDIATE 10

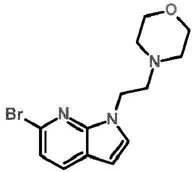

### 4-[2-(6-bromopyrrolo[2,3-b]pyridin-1-yl)ethyl]morpholine

4-[2-(6-bromopyrrolo[2,3-b]pyridin-1-yl)ethyl]morpholine was prepared following General Procedure 2 using 6-bromo-1H-pyrrolo[2,3-b]pyridine (400 mg, 2 mmol) and 2-(4-morpholine)ethylbromide (1.2 equiv., 2.39 mmol). The residue was purified by flash silica chromatography using gradient elution from hexane to EtOAc (30%) in hexane to give intermediate 10 (450 mg, 73% yield).
LCMS (Method 2, ES⁺) 1.90 min, 310 & 312 *m*/*z* (M+H)⁺*.*

### INTERMEDIATE 11

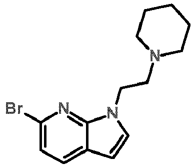

### 6-bromo-1-[2-(1-piperidyl)ethyl]pyrrolo[2,3-b]pyridine

6-bromo-1-[2-(1-piperidyl)ethyl]pyrrolo[2,3-b]pyridine was prepared following General Procedure 2 using 6-bromo-1H-pyrrolo[2,3-b]pyridine (400 mg, 2 mmol) and 1-(2-bromoethyl)piperidine hydrobromide (1.2 equiv., 2.39 mmol). The product was purified by flash silica column chromatography using gradient elution from hexane to hexane:EtOAc 1:1 to give intermediate 11 (420 mg, 69% yield).
LCMS (Method 2, ES⁺) 1.45 min, 308 & 310 *m*/*z* (M+H)⁺*.*

### INTERMEDIATE 12

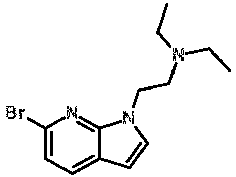

### 2-(6-bromopyrrolo[2,3-b]pyridin-1-yl)-N,N-diethyl-ethanamine

2-(6-bromopyrrolo[2,3-b]pyridin-1-yl)-N,N-diethyl-ethanamine was prepared following General Procedure 2 using 6-bromo-1H-pyrrolo[2,3-b]pyridine (400 mg, 2 mmol) and 2-bromo-N,N-diethylamine hydrobromide (1.2 equiv., 2.4 mmol). The crude product was purified by flash silica column chromatography using gradient elution from hexane to EtOAc to afford Intermediate 12 (360 mg, 61% yield).
LCMS (Method 2, ES⁺) 1.42 min, 296 &298 *m*/*z* (M+H)⁺*.*

### INTERMEDIATE 13

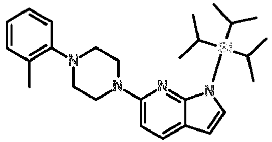

### triisopropyl-[6-[4-(o-tolyl)piperazin-1-yl]pyrrolo[2,3-b]pyridin-1-yl]silane.

Triisopropyl-[6-[4-(o-tolyl)piperazin-1-yl]pyrrolo[2,3-b]pyridin-1-yl]silane was prepared using General Procedure 3 using Intermediate 1 (0.5 g, 3 mmol) and Intermediate 8 (1.2 equiv., 3 mmol). The residue was purified by flash silica column chromatography using gradient elution from hexane to 20% EtOAc in hexane to give Intermediate 13 (1.1 g, 90% yield).
LCMS (Method 2, ES⁺) 2.20 min, 449 *m*/*z* (M+H)⁺.

### INTERMEDIATE 14

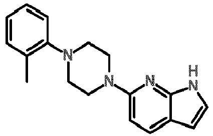

### 6-[4-(o-tolyl)piperazin-1-yl]-1H-pyrrolo[2,3-b]pyridine

Intermediate 13 (1.1 g, 2.5 mmol) was dissolved in THF (10 mL, 0.25 M) and TBAF (2.9 mL, 1.2 eq., 2.9 mmol, 1 mmol/mL) was dropwise added. The mixture was stirred at ambient temperature for 2 hours before Et₂O and H₂O were added. The layers were separated and the aq. phase extracted with Et₂O. The layers were dried over Na₂SO₄ and the solvent removed under vacuum. The solution was filtered through an SCX column and washed with MeOH and DCM, and then eluted with NH₃ (7N in MeOH). The residue was purified by flash chromatography to afford Intermediate 14 (325 mg, 45% yield).
LCMS (Method 2, ES⁺) 1.49 min, 293 *m*/*z* (M+H)⁺*.*

### INTERMEDIATE 15

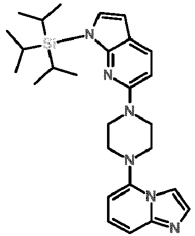

### [6-(4-imidazo[1,2-a]pyridin-5-ylpiperazin-1-yl)pyrrolo[2,3-b]pyridin-1-yl]-triisopropyl-silane

[6-(4-imidazo[1,2-a]pyridin-5-ylpiperazin-1-yl)pyrrolo[2,3-b]pyridin-1-yl]-triisopropyl-silane was prepared following General Procedure 3 using Intermediate 8 (1.2 eq., 13 mmol) and Intermediate 5 (2.2 g, 11 mmol). The residue was purified by flash silica column chromatography with gradient elution from hexane to EtOAc to yield Intermediate 15 (3.2 g, 61% yield).
LCMS (Method 2, ES⁺) 1.90 min, 475 *m*/*z* (M+H)⁺*.*

### INTERMEDIATE 16

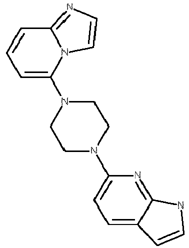

### 5-[4-(1H-pyrrolo[2,3-b]pyridin-6-yl)piperazin-1-yl]imidazo[1,2-a]pyridine

Intermediate 15 (3.15 g, 6.64 mmol) was dissolved in THF (0.25 M, 26 mL) and TBAF (8 mL, 1.2 eq., 7.96 mmol, 1 mmol/mL) added. The reaction mixture was stirred at ambient temperature for 3 hours and then H₂O and Et₂O were added. The layers were separated and the aqueous phase extracted with Et₂O. The solvent was removed under vacuum and the residue was redissolved in MeOH and filtered through an SCX column and washed with MeOH and then eluted with NH₃ (7N in MeOH) to give Intermediate 16 (1.0 g, 44%).
LCMS (Method 2, ES⁺) 1.13 min, 319 *m*/*z* (M+H)⁺*.*

### INTERMEDIATE 17

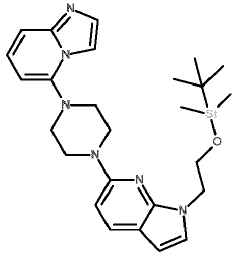

### tert-butyl-[2-[6-(4-imidazo[1,2-a]pyridin-5-ylpiperazin-1-yl)pyrrolo[2,3-b]pyridin-1-yl]ethoxy]-dimethyl-silane

Intermediate 16 (2 g, 6.3 mmol) was dissolved in a mixture of THF (0.25 M) and DMF (0.25 M) and cooled at 0 °C before sodium hydride (1.5 eq., 400 mg, 9.95 mmol) was added in portions. The mixture was stirred at ambient temperature for 30 min. before (2-bromoethoxy)-tert-butyldimethylsilane (1.2 eq., 8 mmol) was added. The mixture was warmed to 60 °C and stirred until the reaction was complete. The reaction mixture was quenched with H₂O and EtOAc added. The layers were separated and the aq. phase extracted with EtOAc. The combined organic layers were dried over Na₂SO₄ and the solvent removed under vacuum. The residue was purified by column chromatography, gradient elution from EtOAc to 1:1 MeOH:EtOAc to afford Intermediate 17 as a white solid (1 g, 44% yield).
LCMS (Method 2, ES⁺) 1.78 min, 477 *m*/*z* (M+H)⁺*.*

### INTERMEDIATE 18

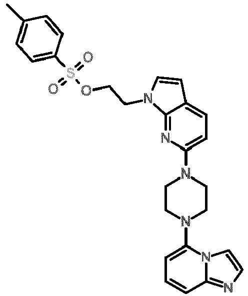

### 2-[6-(4-imidazo[1,2-a]pyridin-5-ylpiperazin-1-yl)pyrrolo[2,3-b]pyridin-1-yl]ethyl 4-methylbenzenesulfonate

Intermediate 17 (1 g, 2.098 mmol) was dissolved in THF (0.25 M, 102.3 mmol) and TBAF (3.1 mL, 3.15 mmol, 1 mmol/mL) was added. The reaction mixture was stirred at ambient temperature for 3 hours and then H₂O and Et₂O added. The layers were separated and the aqeous phase was extracted with Et₂O. The combined organic layers were dried over Na₂SO₄. The solvent was removed under vacuum and the residue was dissolved in MeOH and filtered through an SCX column and washed with MeOH and then eluted with NH₃ (7 N) in MeOH. The resulting residue was dissolved in DCM (0.25 M) and triethylamine (1.5 eq., 3 mmol) added. The solution was cooled to 0 °C, and p-toluensulfonyl chloride (1.2 eq., 2 mmol) was added and the reaction mixture stirred at ambient temperature overnight. The reaction mixture was quenched with H₂O and DCM and the layers separated. The solvent was removed under vacuum and the residue purified by flash silica column chromatography using gradient elution from hexane to EtOAc, and then to 20% MeOH in EtOAc to afford Intermediate 18 (900 mg, 69% yield)
LCMS (Method 2, ES⁺) 1.50 min, 517 *m*/*z* (M+H)⁺*.*

### INTERMEDIATE 19

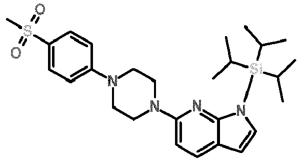

### triisopropyl-[6-[4-(4-methylsulfonylphenyl)piperazin-1-yl]pyrrolo2,3-b]pyridin-1-yl]silane

Triisopropyl-[6-[4-(4-methylsulfonylphenyl)piperazin-1-yl]pyrrolo[2,3-b]pyridin-1-yl]silane was prepared following General Procedure 3 using Intermediate 8 (5 g, 14.2 mmol) and 1-[4-(methylsulfonyl)phenyl]piperazine (1.2 eq., 17 mmol). The residue was purified by flash column chromatography with gradient elution from hexane to EtOAc and then until 30% MeOH in EtOAc to give Intermediate 19 (5.3 g, 73% yield).
LCMS (Method 2, ES⁺) 1.98 min, 513 *m*/*z* (M+H)⁺

### INTERMEDIATE 20

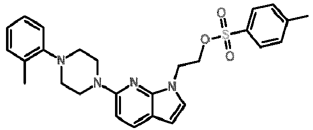

### 2-[6-[4-(o-tolyl)piperazin-1-yl]pyrrolo[2,3-b]pyridin-1-yl]ethyl 4-methylbenzenesulfonate

Intermediate 24 (0.3 g, 0.89 mmol) was dissolved in dichloromethane (5 mL) and triethylamine (0.2 mL, 1 mmol) was added followed by p-toluenesulfonylchloride (171 mg, 0.88 mmol). After 2 h a second portion of triethylamine (0.2 mL, 1 mmol) was added followed by a second portion of intermediate 24 (0.3 g, 0.89 mmol) in dichloromethane (5 mL) and a second portion of p-toluenesulfonylchloride (171 mg, 0.88 mmol). The reaction mixture was then stirred at rt overnight and treated with an aqueous work up. The organic solvent was dried (Na₂SO₄) and concentrated under reduced pressure. The crude residue was then purified by flash silica column chromatography to give the title compound (0.51 g, quant.)
LCMS (Method 2, ES⁺) 1.63 min, 491 *m*/*z* (M+H)⁺*.*

### INTERMEDIATE 21

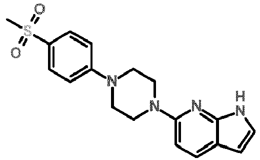

### 6-[4-(4-methylsulfonyphenyl)piperazin-1-yl]-1H-pyrrolo[2,3-b]pyridine

Intermediate 19 (3 g, 5.9 mmol) was dissolved in THF (0.5 M) and TBAF (8.8 mL, 1.5 eq., 8.8 mmol, 1 mmol/mL) was added at ambient temperature. The mixture was stirred for 1 hour then H₂O and Et₂O were added. The layers were separated and the aq. phase was extracted with Et₂O. The solvent was removed under vacuum and the residue purified by flash chromatography gradient elution from hexane to EtOAc and then to a mixture of 1:1 EtOAc:MeOH to yield intermediate 21 (950 mg, 46% yield).
LCMS (Method 2, ES⁺) 1.20 min, 357 *m*/*z* (M+H)⁺.

### INTERMEDIATE 22

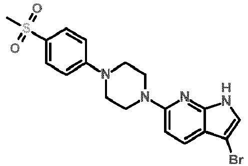

### 3-bromo-6-[4-(4-methylfonylphenyl)piperazin-1-yl]-1H-pyrrolo[2,3-b]pyridine

Intermediate 19 (730 mg, 1.4 mmol) was dissolved in DCM (30 mL) and cooled to 0 °C. N-bromosuccinimide (165 mg, 0.92 mmol) was added portion-wise over 20 min. The reaction was stirred at that temperature for an additional 10 minutes before it was quenched with saturated NaHCO₃ and the layers separated. The aqueous phase was then extracted with DCM. The solvent was removed under vacuum and the residue directly dissolved in THF (10 mL) TBAF (1.6 mL, 1.2 eq., 1.6 mmol, 1 mmol/mL) was added and the mixture was stirred at ambient temperature for 20 minutes. The crude reaction mixture was quenched with saturated aq. NH₄Cl solution and EtOAc added. The layers were separated. The aq. phase was then extracted with EtOAc. The combined organic layers were combined, dried over Na₂SO₄ and the solvent removed under vacuum. The residue was purified by chromatography gradient elution from DCM to 30% MeOH in DCM to yield Intermediate 22 (500 mg, 32% yield).
LCMS (Method 2, ES⁺) 1.34 min, 434 & 436 *m*/*z* (M+H)⁺.

### INTERMEDIATE 23

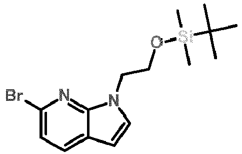

### 2-(6-bromopyrrolo[2,3-b]pyridin-1-yl)ethoxy-tert-butyl-dimethyl-silane

2-(6-bromopyrrolo[2,3-b]pyridin-1-yl)ethoxy-tert-butyl-dimethyl-silane was prepared following General Procedure 2 using 6-bromo-1H-pyrrolo[2,3-b]pyridine (2 g, 9.9 mmol) and (2-bromoethoxy)-tert-butyldimethylsilane (2.7 mL, 12 mmol) to afford intermediate 23 (3.13 g, 88% yield).
LCMS (Method 2, ES⁺) 1.78 min, 355 & 357 *m*/*z* (M+H)⁺.

### INTERMEDIATE 24

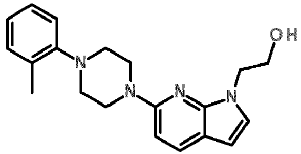

### 2-[6-[4-(o-tolyl)piperazin-1-yl]pyrrolo[2,3-b]pyridin-1-yl]ethanol

Using general procedure 3, intermediate 1 (1.2 g, 6.8 mmol), intermediate 23 (2.7 g, 7.6 mmol), and sodium tert-butoxide (2.5 eq) gave tert-butyl-dimethyl-[2-[6-[4-(o-tolyl)piperazin-1-yl]pyrrolo[2,3-b]pyridin-1-yl]ethoxy]silane, which was then dissolved in THF (20 mL). TBAF (14 mL, 14 mmol) was then added and the reaction mixture stirred at room temperature for 5 days. The reaction mixture was concentrated under reduced pressure and loaded onto an SCX cartridge washing first with methanol then eluting with 2 M (approx.) ammonia in methanol. The ammonia in methanol solution was concentrated under reduced pressure and the residue purified by flash silica column chromatography to give the title compound (1.37 g, 60% yield).
LCMS (Method 2, ES⁺) 1.47 min, 337 *m*/*z* (M+H)⁺.

### INTERMEDIATE 25

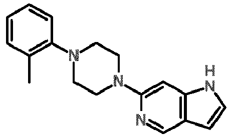

### 6-[4-(o-tolyl)piperazin-1-yl]-1H-pyrrolo[3,2-c]pyridine

Using general procedure 3, intermediate 9 (455 mg, 1.47 mmol), intermediate 1 (236 mg, 1.33 mmol), and sodium tert-butoxide (260 mg, 2.7 mmol) gave triisopropyl-[6-[4-(o-tolyl)piperazin-1-yl]pyrrolo[3,2-c]pyridin-1-yl]silane, which was dissolved in THF (5 mL). TBAF (4 mL, 4 mmol) was then added and the reaction mixture stirred at r.t. overnight. The reaction mixture was treated with an aqueous work up and the organic layer dried (Na₂SO₄) and concentrated under reduced pressure. The resulting residue was passed through an SCX cartridge washing first with methanol then eluting with approx. 2 M ammonia in methanol. The solution was concentrated under reduced pressure to give the title compound (46 mg, 12% yield over two steps).
LCMS (Method 2, ES⁺) 1.31 min, 293 *m*/*z* (M+H)⁺.

### INTERMEDIATE 26

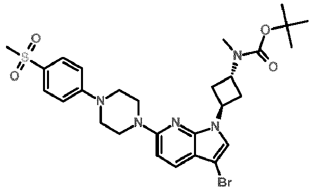

### Trans-tert-butyl N-[3-[3-bromo-6-[4-(4-methylsulfonylphenyl)piperazin-1-yl]pyrrolo[2,3-b]pyridin-1-yl]cyclobutyl]-N-methyl-carbamate

Intermediate 22 (500 mg, 1.15 mmol) and cis-tert-butyl N-(3-hydroxycyclobutyl)-N-methylcarbamate (1.5 eq., 1.7 mmol) were reacted following General Procedure 9 and purified by flash column chromatography eluting from hexane to 70% EtOAc in hexane to give intermediate 26 (410 mg, 78% yield).
LCMS (Method 2, ES⁺) 1.73 min, 618 & 620 *m*/*z* (M+H)⁺.

### INTERMEDIATE 27

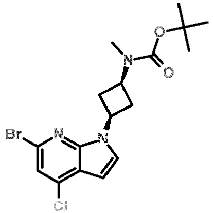

### Cis-tert-butyl N-[3-(6-bromo-4-chloro-pyrrolo[2,3-b]pyridin-1-yl)cyclobutyl]-N-methyl-carbamate

6-Bromo-4-chloro-1H-pyrrolo[2,3-b]pyridine (250 mg, 1.08 mmol, 100 mass%) and trans-tert-butyl N-(3-hydroxycyclobutyl)-N-methyl-carbamate (1.5 eq., 1.62 mmol) were reacted following General Procedure 9 to yield Intermediate 27 (430 mg, 96% yield).
LCMS (Method 2, ES⁺) 1.84 min, 414 & 416 *m*/*z* (M+H)⁺*.* (Br & Cl isotopes check)

### INTERMEDIATE 28

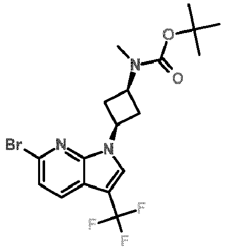

### Cis-tert-butyl N-[3-[6-bromo-3-(trifluoromethyl)pyrrolo[2,3-b]pyridin-1-yl]cyclobutyl]-N-methylcarbamate

6-Bromo-3-(trifluoromethyl)-1H-pyrrolo[2,3-b]pyridine (100 mg, 0.38 mmol) and trans-tert-butyl N-(3-hydroxycyclobutyl)-N-methyl-carbamate (1.5 eq., 0.6 mmol) were reacted following General Procedure 9 to yield intermediate 28 (160 mg, 86% yield).
LCMS (Method 2, ES⁺) 1.84 min, 448 & 450 *m*/*z* (M+H)⁺.

### INTERMEDIATE 29

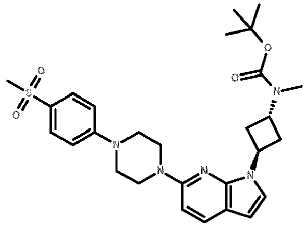

### Trans-tert-butyl N-methyl-N-[3-[6-[4-(4-methylsulfonylphenyl)piperazin-1-yl]pyrrolo[2,3-b]pyridin-1-yl]cyclobutyl]carbamate

6-[4-(4-methylsulfonylphenyl)piperazin-1-yl]-1H-pyrrolo[2,3-b]pyridine (Intermediate 21, 650 mg, 1.8 mmol) and cis-tert-butyl N-(3-hydroxycyclobutyl)-N-methyl-carbamate (1.5 eq., 2.7 mmol) were reacted following the General Procedure 9. The reaction mixture was concentrated and the residue was directly purified by flash chromatography gradient elution from hexane to EtOAc to afford Intermediate 29 (610 mg, 62% yield).
LCMS (Method 2, ES⁺) 1.61 min, 640 *m*/*z* (M+H)⁺.

### INTERMEDIATE 30

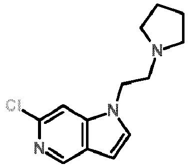

### 6-chloro-1-(2-pyrrolidin-1-ylethyl)pyrrolo[3,2-c]pyridine

6-Chloro-5-azaindole (750 mg, 4.8 mmol) and 1-(2-chloroethyl)pyrrolidine hydrochloride (1 g, 5.76 mmol) were reacted using general procedure 2 to give the title compound (860 mg, 71%).
LCMS (Method 2, ES⁺) 1.06 min, 250 *m*/*z* (M+H)⁺.

### INTERMEDIATE 31

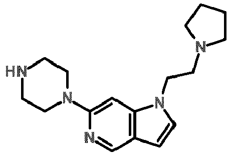

### 6-piperazin-1-yl-1-(2-pyrrolidin-1-ylethyl)pyrrolo[3,2-c]pyridine

Using general procedure 1 and intermediate 30 (250 mg, 1.00 mmol) the title compound was obtained (250 mg, 83%).
LCMS (Method 2, ES⁺) 0.94 min, 300 *m*/*z* (M+H)⁺.

### INTERMEDIATE 32-35

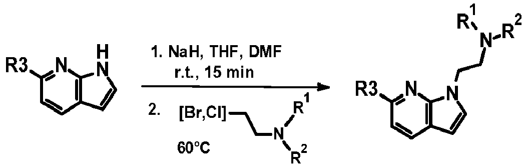

Intermediates 32-35 can be synthesized from commercially available 6-bromo-1H-pyrrolo[2,3-b]pyridine and the appropriate alkyl halide using General Procedure 2.

| Interm. Number | Structure | Alkyl halide | Compound Name | LCMS (Method 2) RT (min) | *m*/*z* (M+H)⁺ |
|---|---|---|---|---|---|
| 32 | 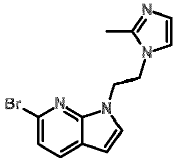 | 1-(2-Chloroethyl)-2-methyl-1H-imidazole hydrochloride | 6-bromo-1-[2-(2-methylimidazol-1-yl)ethyl]pyrrolo[2, 3-b]pyridine | 1.07 | 305 & 307 |
| 33 | 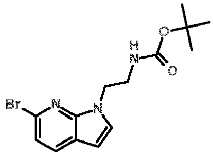 | N-Boc-2-chloethylamine | tert-butyl N-[2-(6-bromopyrrolo[2,3-b]pyridin-1-yl)ethyl]carbamat e | 1.40 | 340 & 242 |
| 34 | 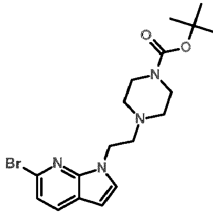 | tert-butyl 4-(2-bromoethyl)pip erazine-1-carboxylate | tert-butyl 4-[2-(6-bromopyrrolo[2,3-b]pyridin-1-yl)ethyl]piperazin e-1-carboxylate | 1.50 | 409 & 411 |
| 35 | 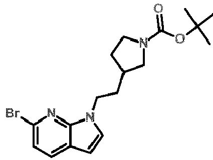 | tert-butyl 3-(2-bromoethyl)pyr rolidine-1-carboxylate | tert-butyl 3-[2-(6-bromopyrrolo[2,3-b]pyridin-1-yl)ethyl]pyrrolidin e-1-carboxylate | 1.54 | 338 & 340 [M-tBu+H] |

### INTERMEDIATE 36

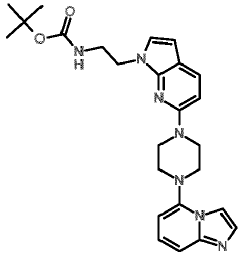

### tert-butyl N-[2-[6-(4-imidazo[1,2-a]pyridin-5-ylpiperazin-1-yl)pyrrolo[2,3-b]pyridin-1-yl]ethyl]carbamate

General Procedure 3, using intermediate 33 (384 mg, 1.13 mmol) and intermediate 5 (240 mg, 1.18 mmol) gave the title compound (252 mg, 48%).
LCMS (Method 2, ES⁺) 1.41 min, 462 *m*/*z* (M+H)⁺.

### INTERMEDIATE 37-45

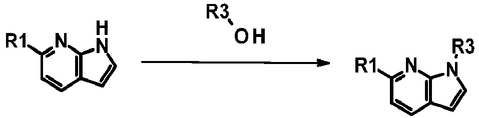

Intermediates 37-45 were synthesized according to General Procedure 9 from an appropriate heterocycle and alcohol tabulated below.

Intermediate 39 was purified by filtration through an SCX cartridge washing first with methanol then eluting with approx. 2 M ammonia in methanol. The ammonia in methanol solution was concentrated under reduced pressure to give the desired product.

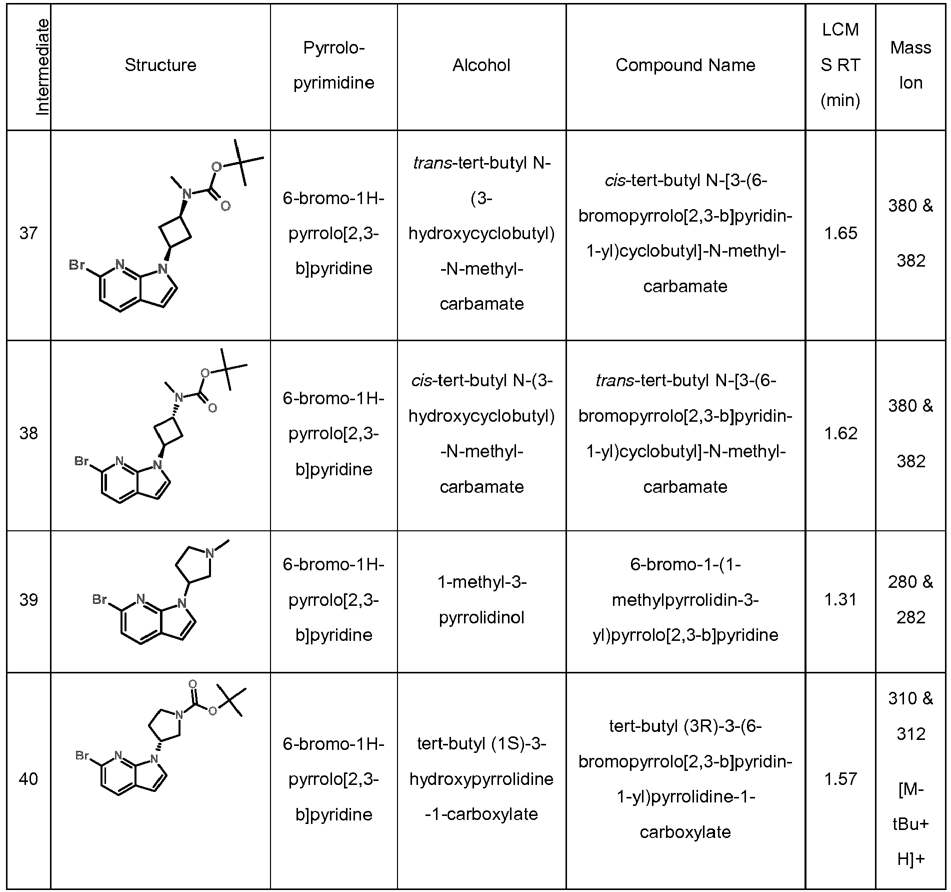

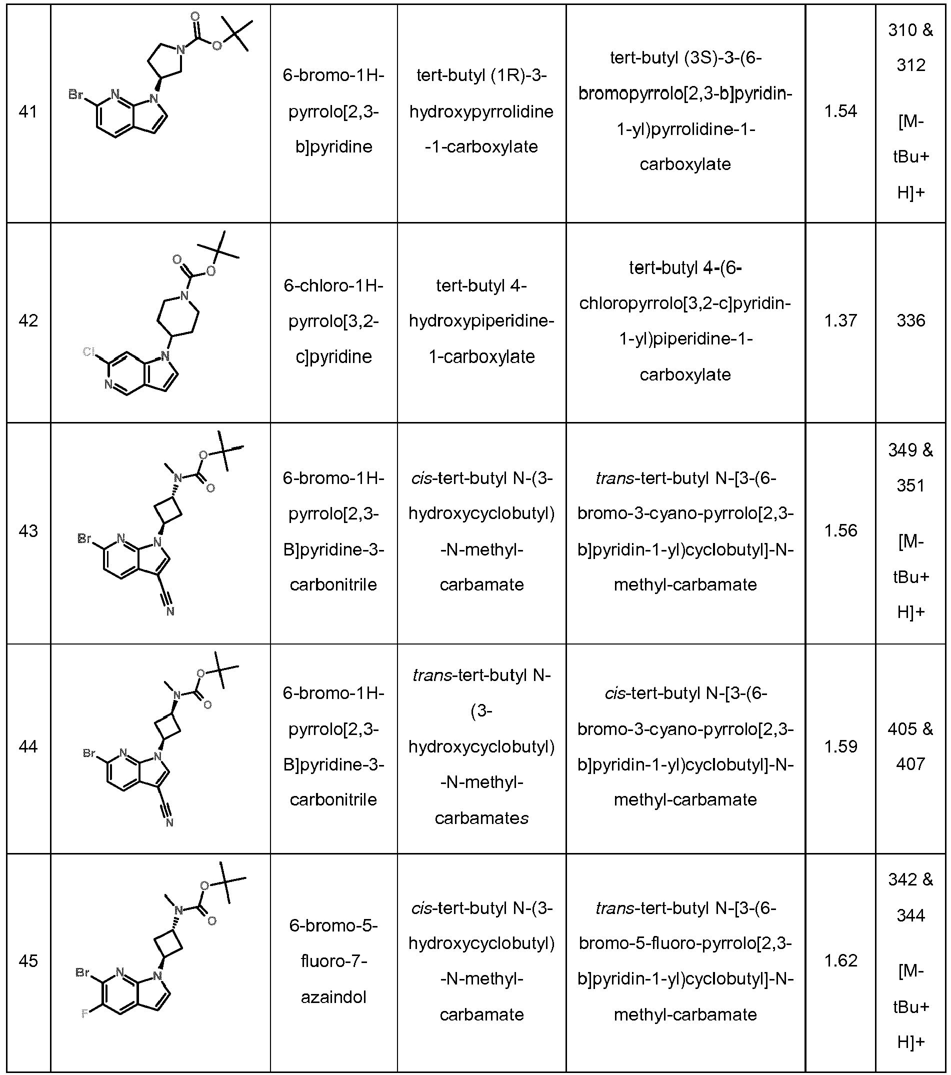

### INTERMEDIATE 46

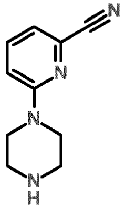

### 6-piperazin-1-ylpyridine-2-carbonitrile

Intermediate 46 was prepared using General Procedure 3, from 6-bromo-2-pyridinecarbonitrile (500 mg, 2.7 mmol) and tert-butyl piperazine-1-carboxylate (610 mg, 3.2 mmol), followed by General Procedure 13 to give the title compound after reverse phase chromatography (120 mg, 23%)
LCMS (Method 2, ES+) 0.51 min, 189 m/z (M+H)⁺.

### INTERMEDIATE 47

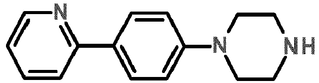

### 1-[4-(2-pyridyl)phenyl]piperazine

Intermediate 47 was prepared according to General Procedure 1 from 2-(4-bromophenyl)pyridine (529 mg, 2.15 mmol) and tert-butyl piperazine-1-carboxylate (400 mg, 2.15 mmol). The title compound was obtained in quantitative yield (139 mg).
LCMS (Method 2, ES+) 0.85 min, 240 m/z (M+H)+.

### INTERMEDIATE 48

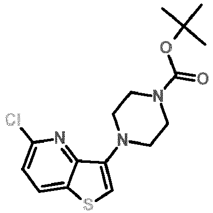

### tert-butyl 4-(5-chlorothieno[3,2-b]pyridin-3-y)piperazine-1-carboxylate

General Procedure 14, using 3-bromo-5-chlorothieno[3,2-B]pyridine (200 mg, 0.79 mmol) and 1-boc-piperazine (180 mg, 0.95 mmol) at 80 °C gave the title compound (24 mg, 9%).
LCMS (Method 2, ES+) 1.53 min, 354 & 356 m/z (M+H)+.

### INTERMEDIATE 49

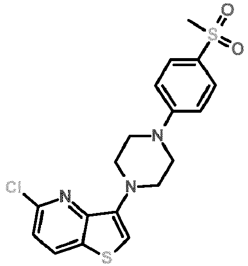

### 5-chloro-3-[4-(4-methylsulfonylphenyl)piperazin-1-yl]thieno[3,2-b]pyridine

General Procedure X, using 3-bromo-5-chlorothieno[3,2-B]pyridine (200 mg, 0.79 mmol) and 1-[4-(methylsulphonyl)phenyl]piperazine (208 mg, 0.87 mmol) at 80 °C gave the title compound (20 mg, 6%).
LCMS (Method 2, ES+) 1.36 min, 408 & 410 m/z (M+H)+.

### INTERMEDIATE 50

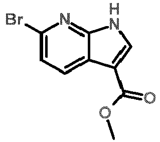

### methyl 6-bromo-1H-pyrrolo[2,3-b]pyridine-3-carboxylate

6-bromo-1H-pyrrolo[2,3-b]pyridine (1 g, 5.1 mmol) was dissolved in DCM (40 mL) and AlCl₃ (3.5 equiv., 17.8 mmol) was added at r.t. portion-wise. The mixture was stirred for 30 minutes before trichloroacethyl chloride (1 equiv., 5.1 mmol) was added drop-wise. The mixture was stirred at r.t. for 2 hours. H₂O and DCM were added and the layers were separated. The solvent was removed under vacuum. The solid was dissolved in MeOH (20 mL) and KOH (200 mg, 3.6 mmol) was added. The mixture was stirred at 60°C for 3 hours until complete consumption of the starting material. Then the reaction was cooled to r.t. and EtOAc and HCl (2M) aq. solution was added. The layers were separated and the organic layer was dried over Na₂SO₄. The volatiles were removed under vacuum and the residue purified by flash chromatography with gradient elution from hexane to EtOAc, and then 10% MeOH in EtOAc to yield Intermediate 50 (420 mg, 24% yield).
LCMS (Method 2, ES+) 1.01 min, 255 & 257 m/z.

### INTERMEDIATE 51

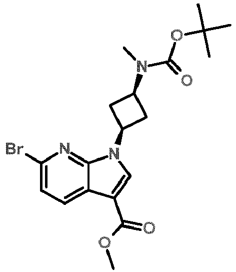

### cis-methyl 6-bromo-1-[3-[tert-butoxycarbonyl(methyl)amino]cyclobytyl]pyrrolo[2,3-b]pyridine-3-carboxylate

Intermediate 50 (400 mg, 1.6 mmol) was reacted with tert-butyl-N-(trans-3-hydroxycyclobutyl)-N-methylcarbamate (1.5 equiv., 2.3 mmol) following the General Procedure 9. The product was purified by flash column chromatography gradient elution from hexane to 70% EtOAc in hexane to yield Intermediate 51 (550 mg, 80% yield).
LCMS (Method 2, ES+) 1.66 min, 438 & 440 m/z (M+H)+.

### INTERMEDIATE 52

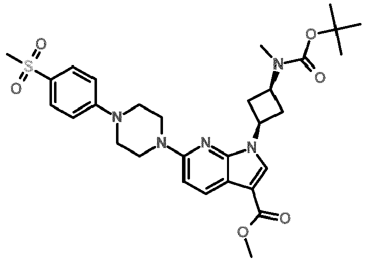

### Methyl 1-[3-[tert-butoxycarbonyl)methyl)amino]cyclobutyl]-6-[4-methylsulfonylphenyl) piperazin-1-yl]pyrrolo[2,3-b]pyridine-3-carboxylate

Intermediate 51 (315 mg, 0.7 mmol)and 1-[4-methylsulfonyl)phenyl]piperazine were reacted following the General Procedure 3 at 60 °C. The mixture was cooled to r.t. and H₂O and EtOAc added. The layers were separated and the aq. phase was extracted with EtOAc. The combined organic layers were dried over Na₂SO₄ and the volatiles were removed under vacuum. The residue was purified by flash column chromatography using gradient elution from hexane to EtOAc and then EtOAc to 30% of MeOH in EtOAc to afford Intermediate 52 (140 mg, 19% yield) and Intermediate 53.
LCMS (Method 2, ES⁺) 1.54 min, 598 *m*/*z* (M+H)⁺.

### Intermediate 53

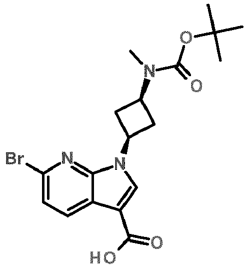

### 6-bromo-1-[3-[tert-butoxycarbonyl(methyl)amino]cyclobutyl]pyrrolo[2,3-b]pyridine-3-carboxylic acid

Intermediate 53 was obtained (180 mg, 34% yield) as a by-product from the synthesis of Intermediate 52.
LCMS (Method 2, ES+) 1.14 min, 424 & 426 m/z

### Examples 1-22

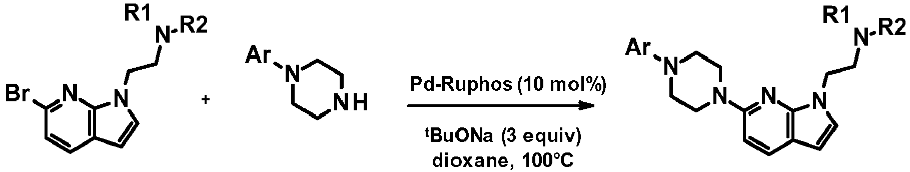

The following compounds were synthesised using General Procedure 3 from heteroaryl halide and the appropriate amine (commercially available or synthesised intermediate) as noted in the table below.

### Examples 1-9, Example 11 and Example 17-19: LCMS Method 1

### Example 10: LCMS Method 2

### Examples 12-16: LCMS Method 4

### Examples 20-22: LCMS Method 5

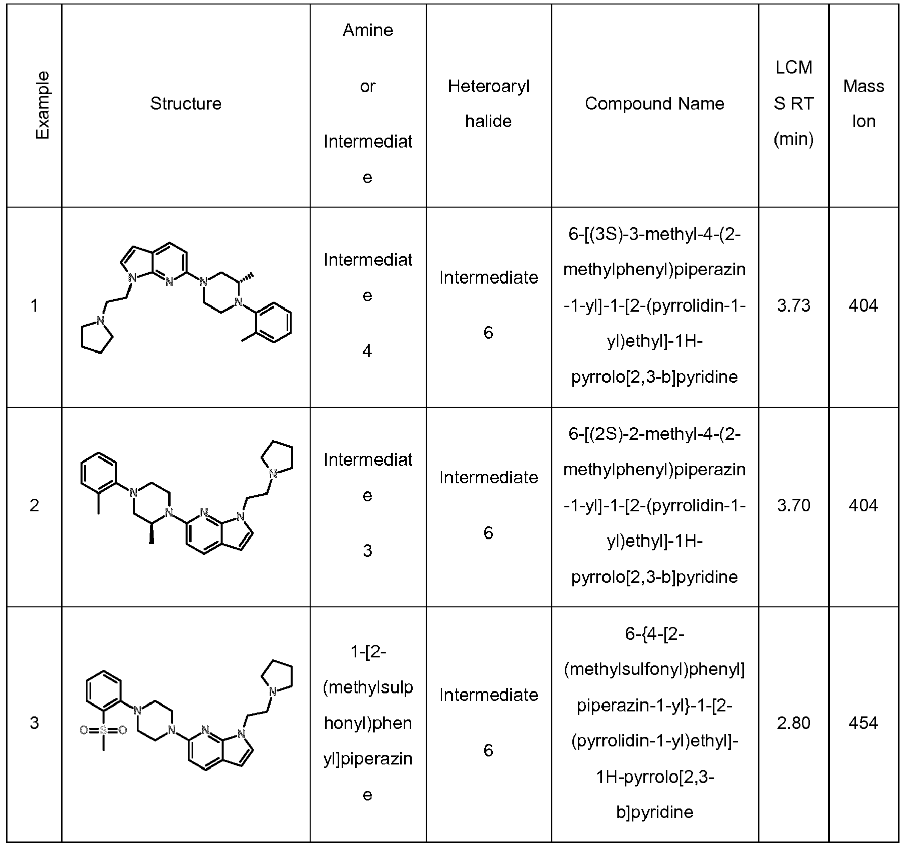

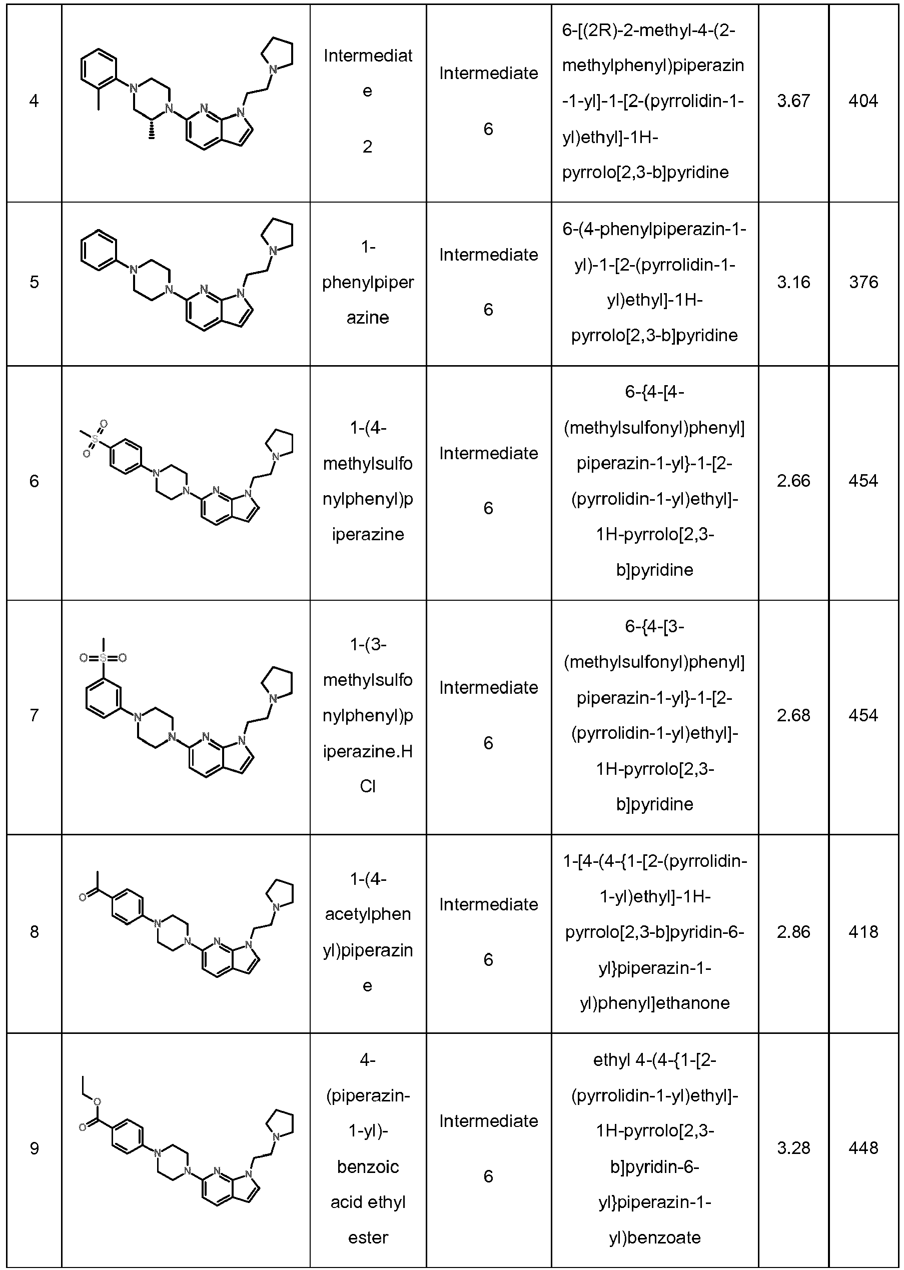

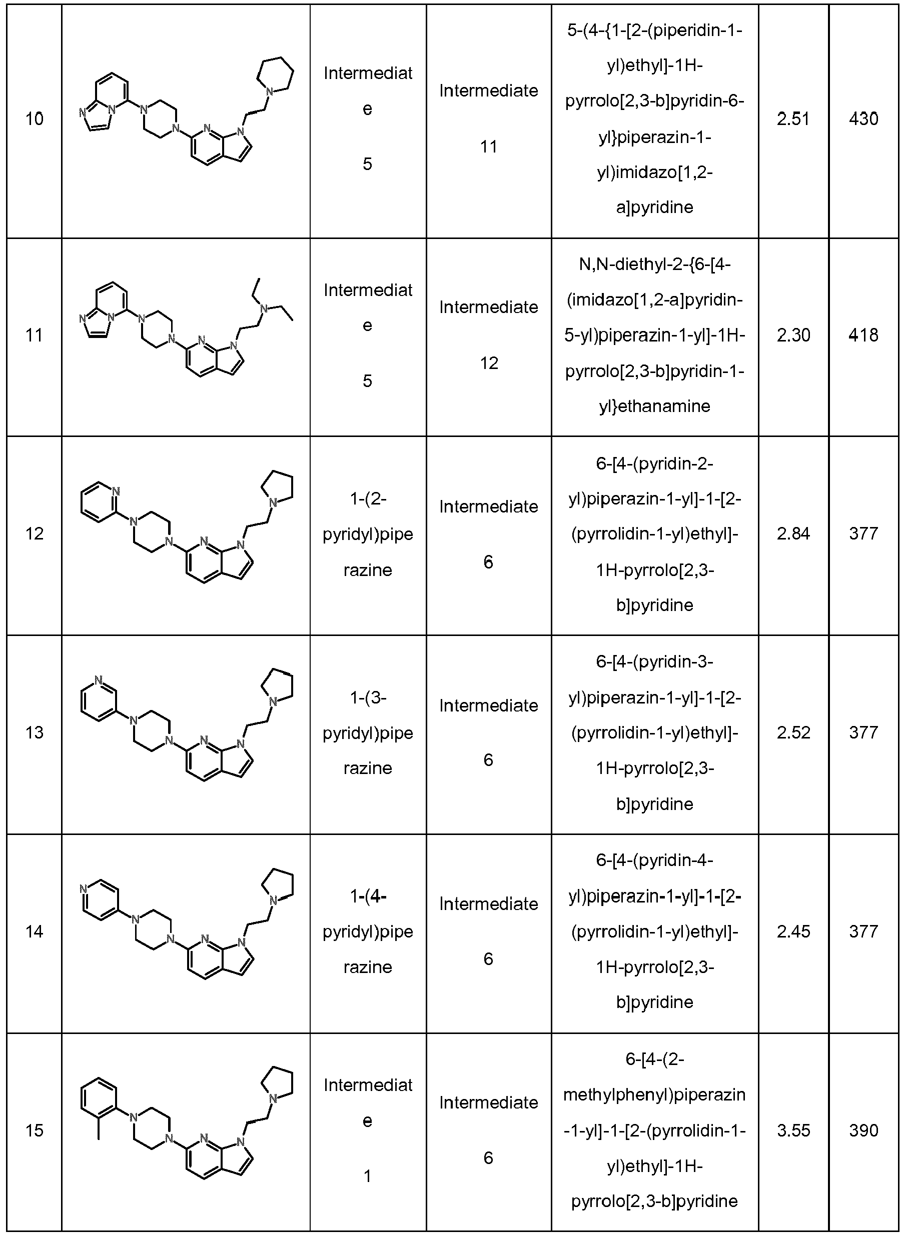

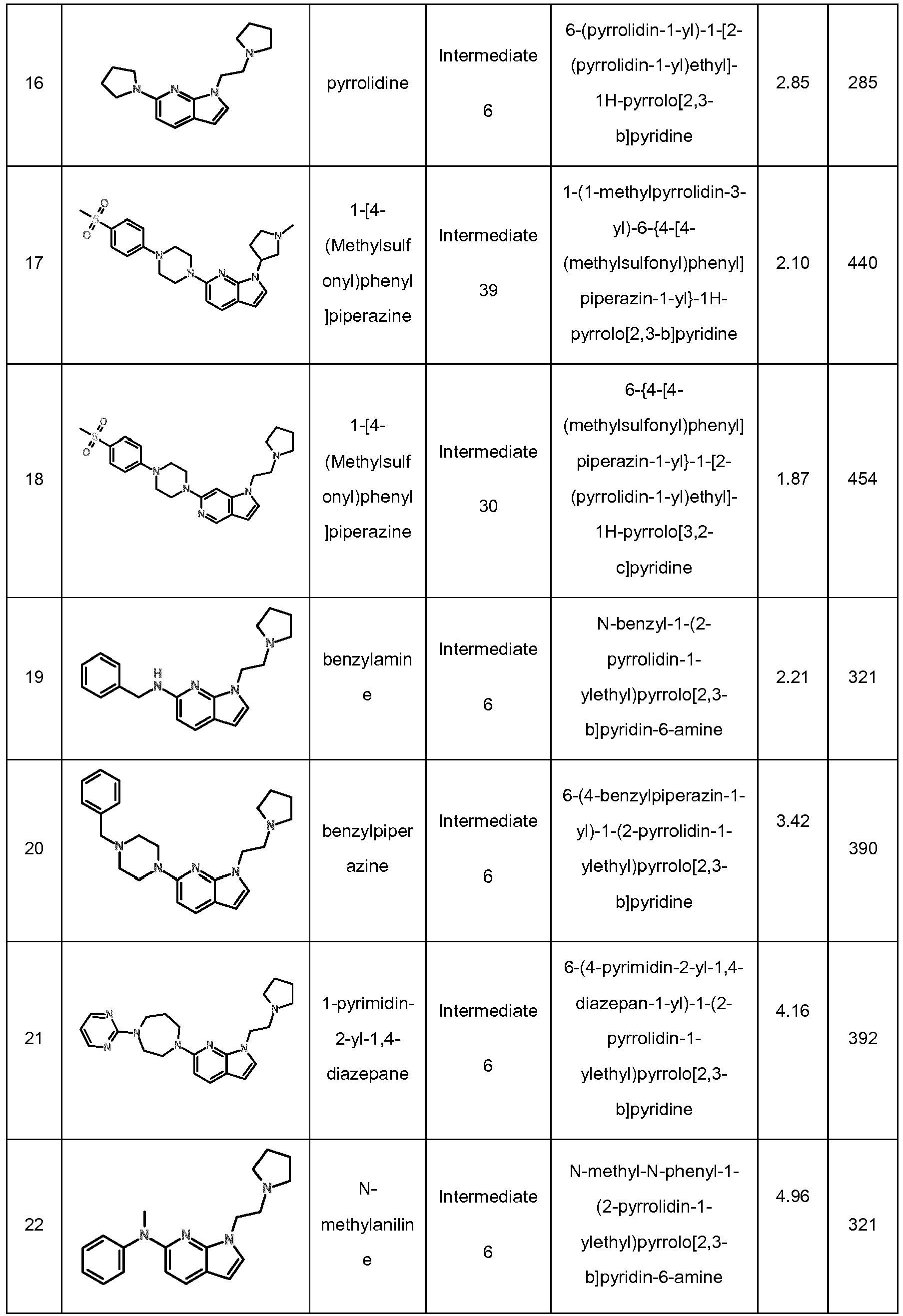

### Examples 23-35

Examples 23-35 were prepared in two steps according to General Procedure 3 followed by General Procedure 13 and analysed by LCMS Method 1.

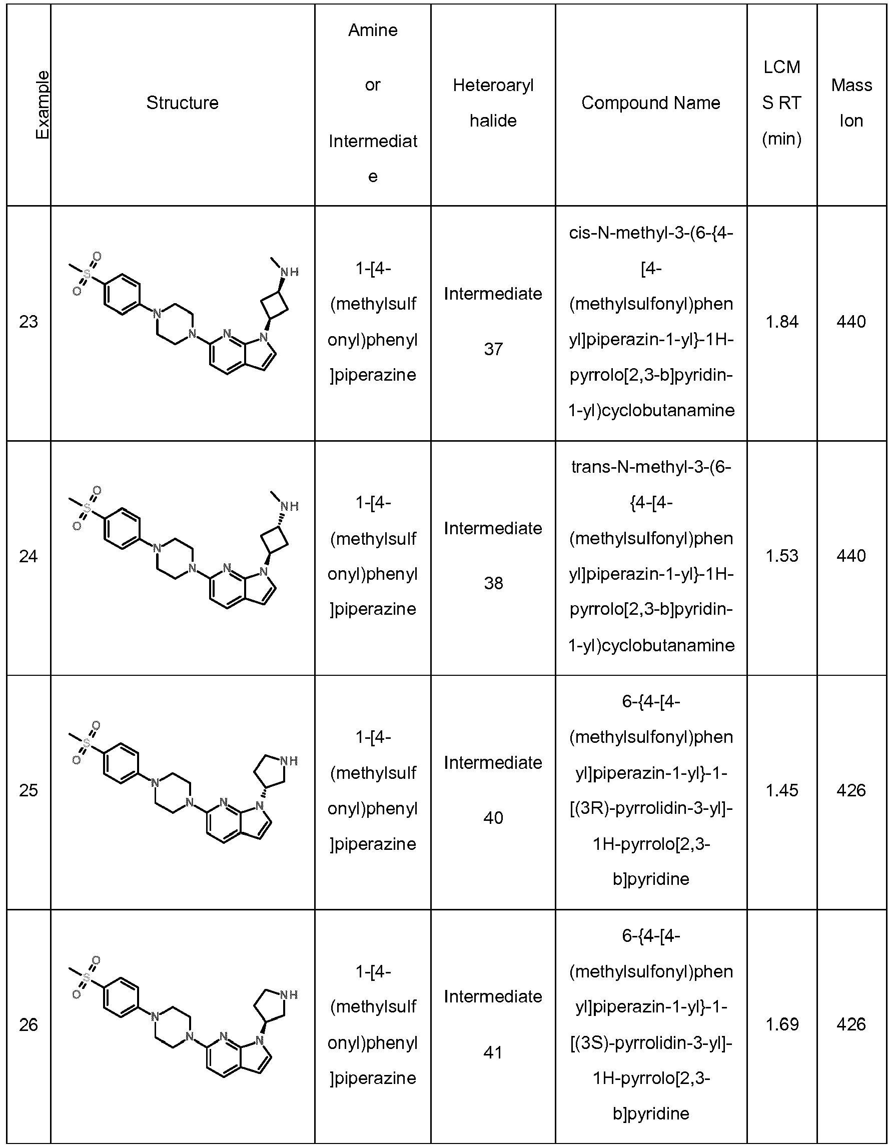

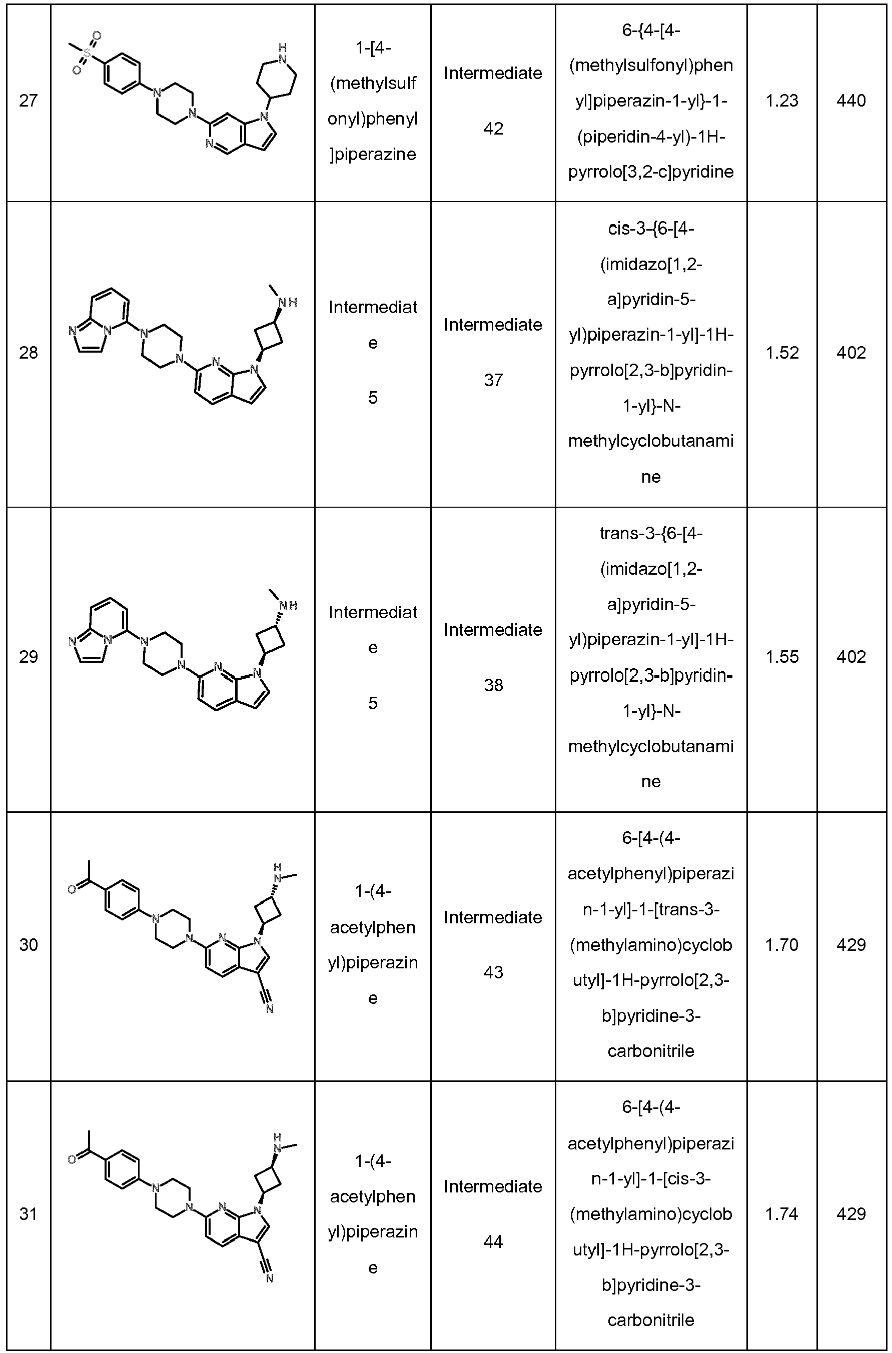

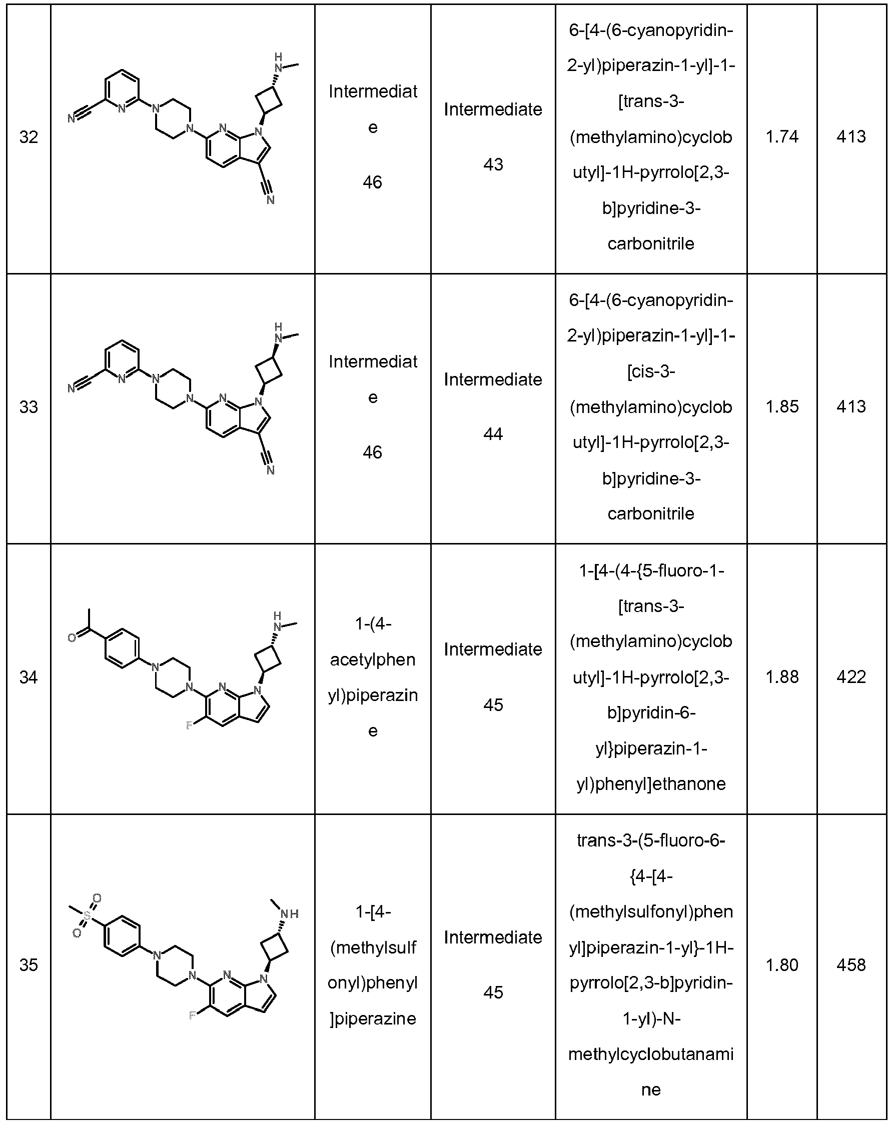

### Examples 36-39

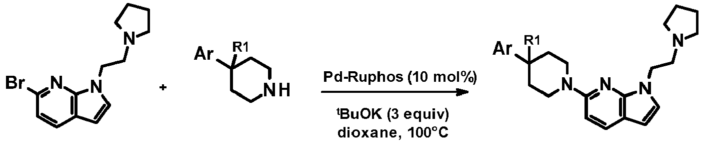

The following compounds were synthesized from Intermediate 6 and the appropriate piperidine in accordance with General Procedure 4.

Examples were analysed by LCMS Method 1.

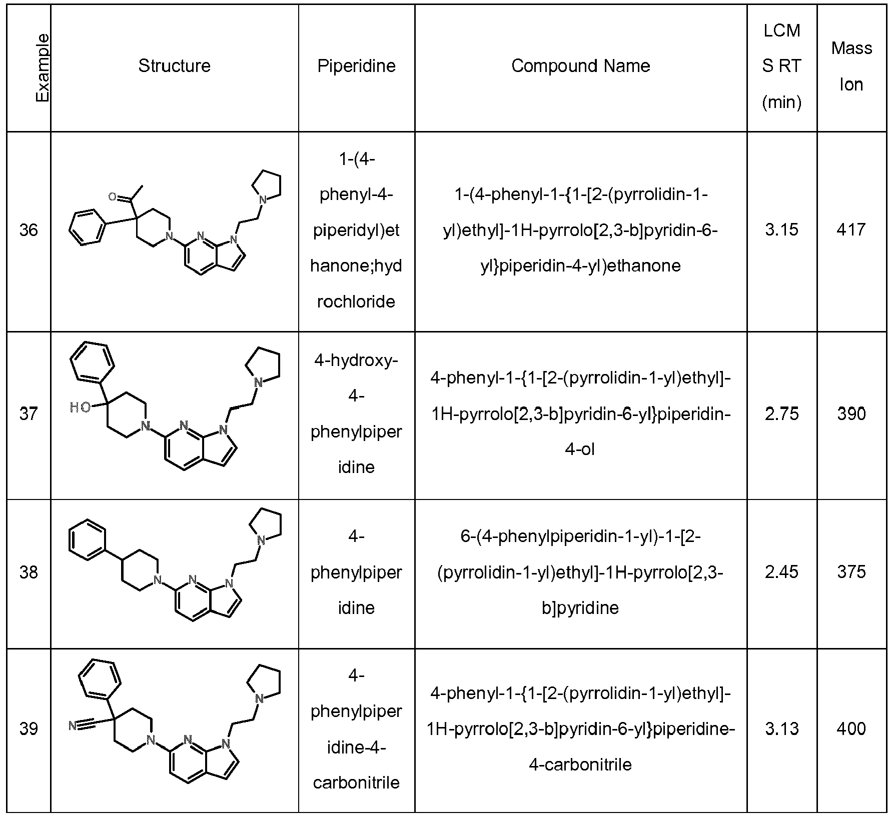

### Examples 40-58

The following compounds were synthesized from Intermediate 7 and the appropriate aryl bromide in accordance with General Procedure 5.

Examples 40-51 were analysed by LCMS Method 3.

Examples 52-58 were analysed by LCMS Method 1.

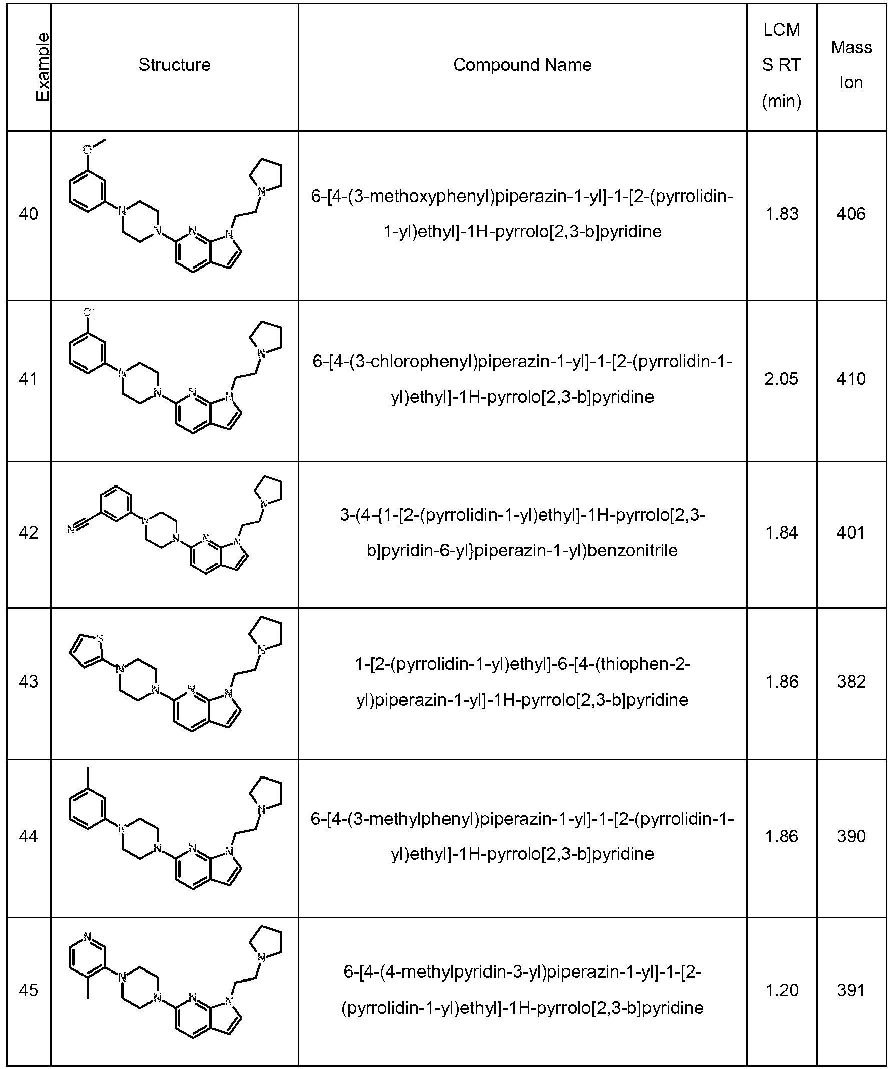

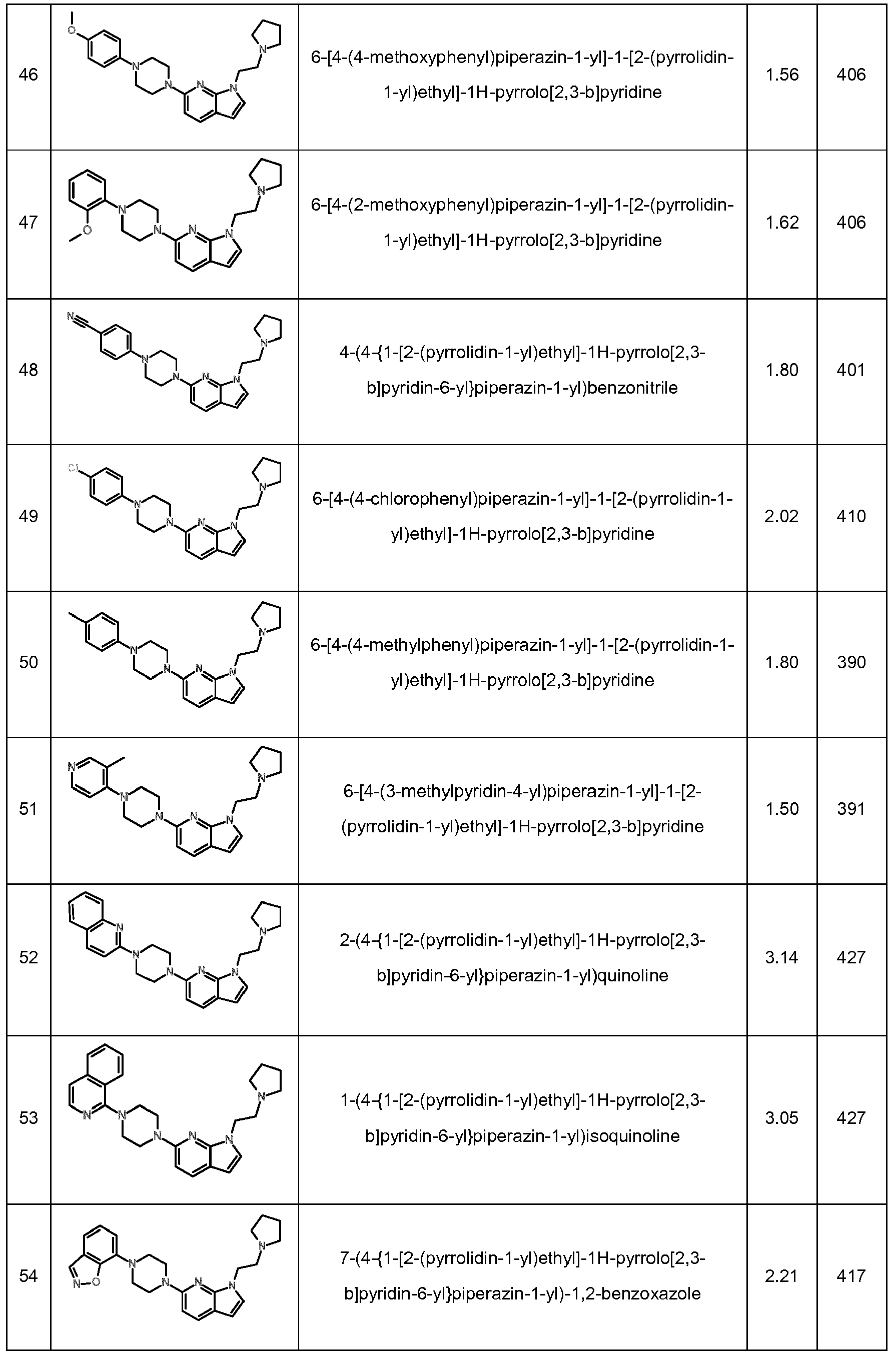

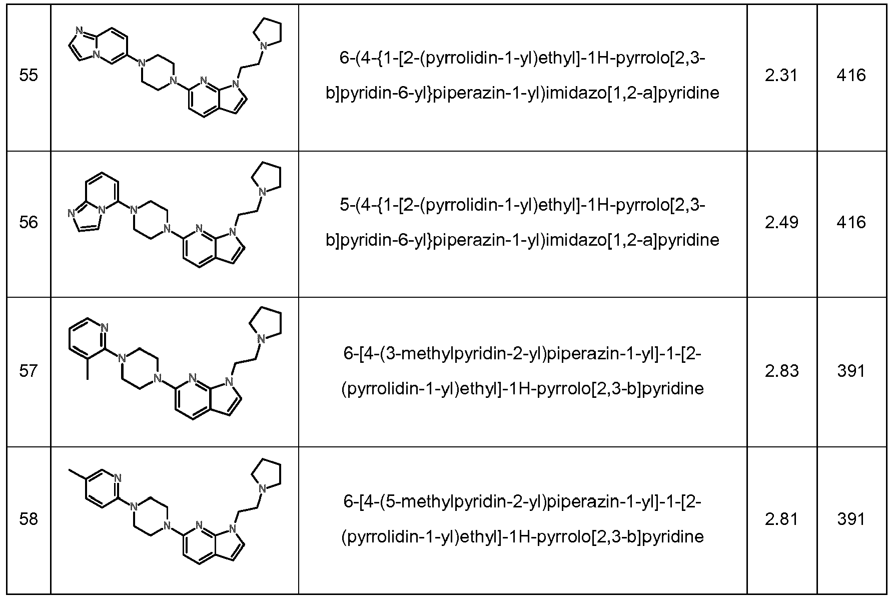

### Examples 59-61

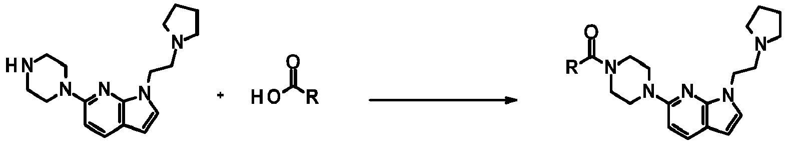

Examples 59-61 were synthesized from Intermediate 7 and the appropriate carboxylic acid in accordance with General Procedure 6 and analysed by LCMS Method 3.

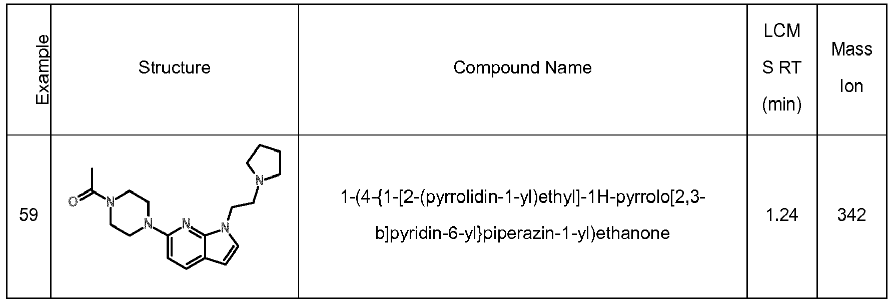

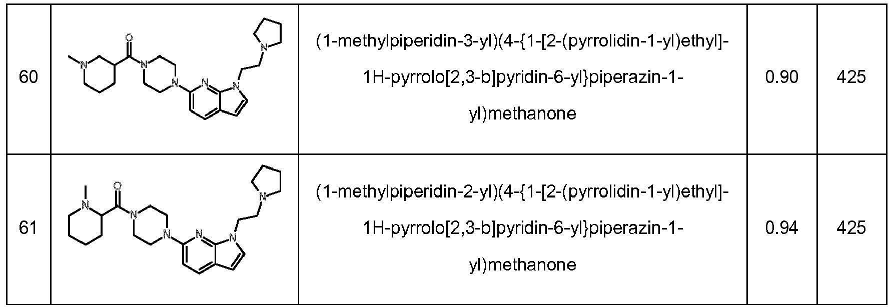

### Examples 62-77

The following compounds were synthesised from Intermediate 20 and the appropriate amine in accordance with General Procedure 7.

### Examples 62-75: LCMS Method 3

### Example 76-77: LCMS Method 1

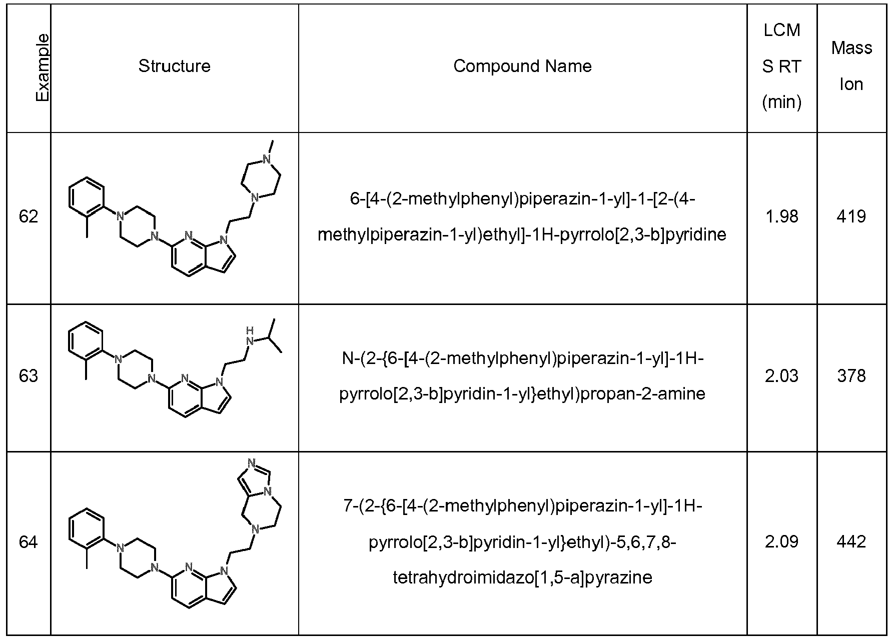

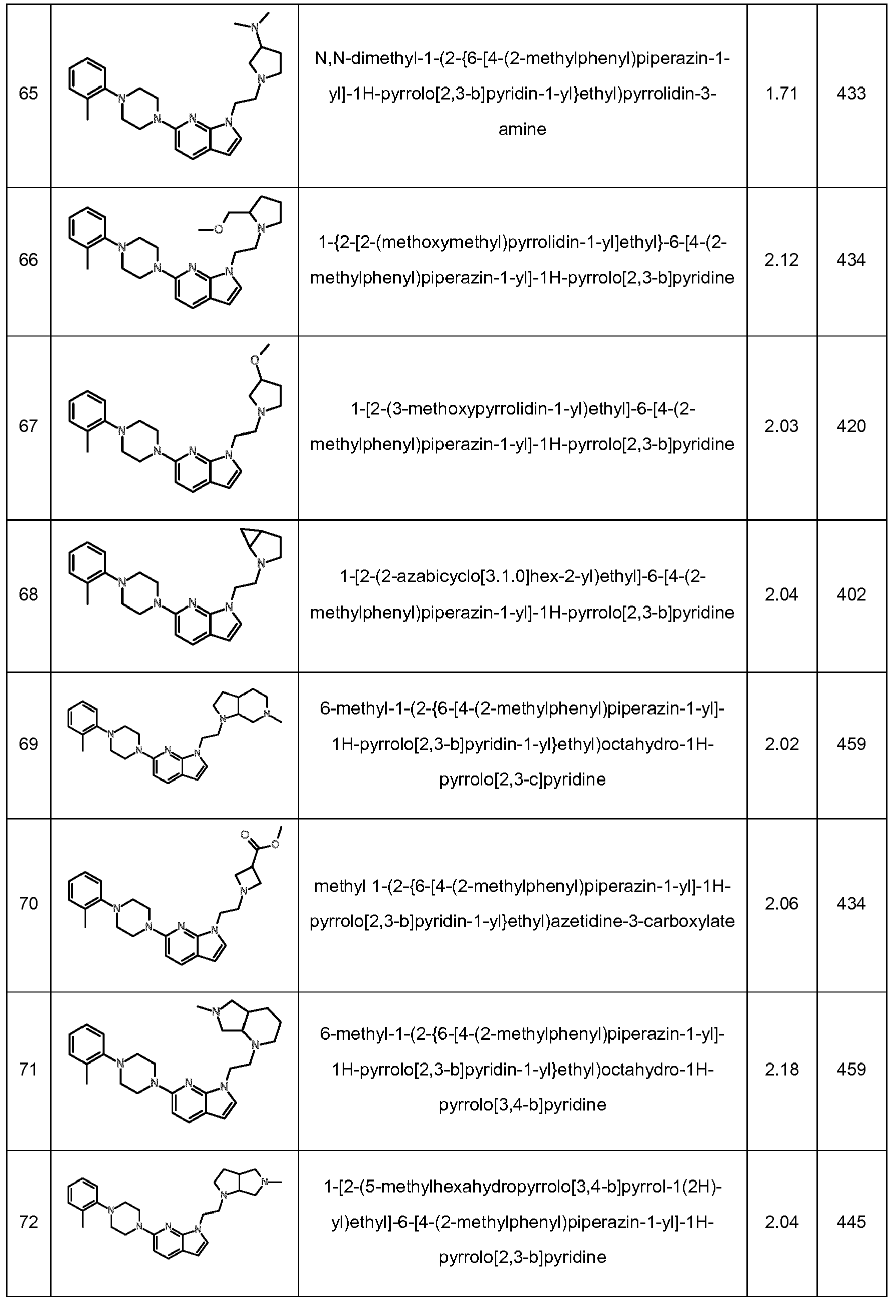

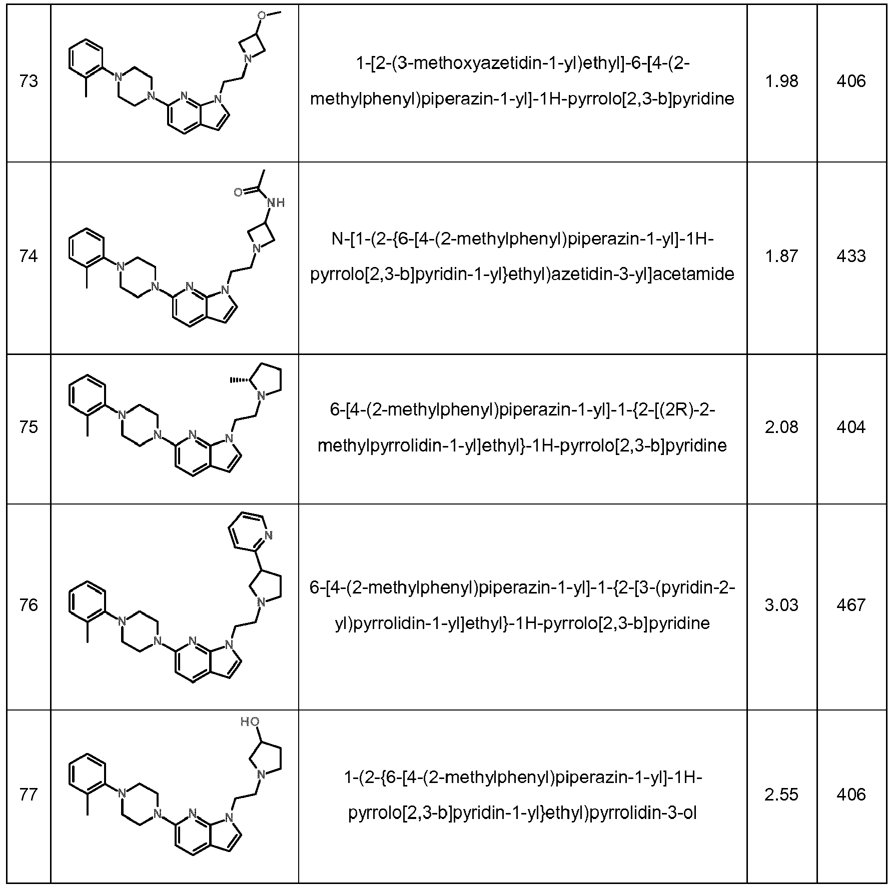

### Examples 78-94

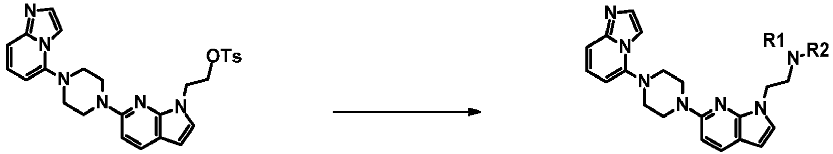

The following compounds were synthesised from Intermediate 18 and the appropriate amine in accordance with General Procedure 8.

Examples 78-94 were analysed by LCMS Method 3.

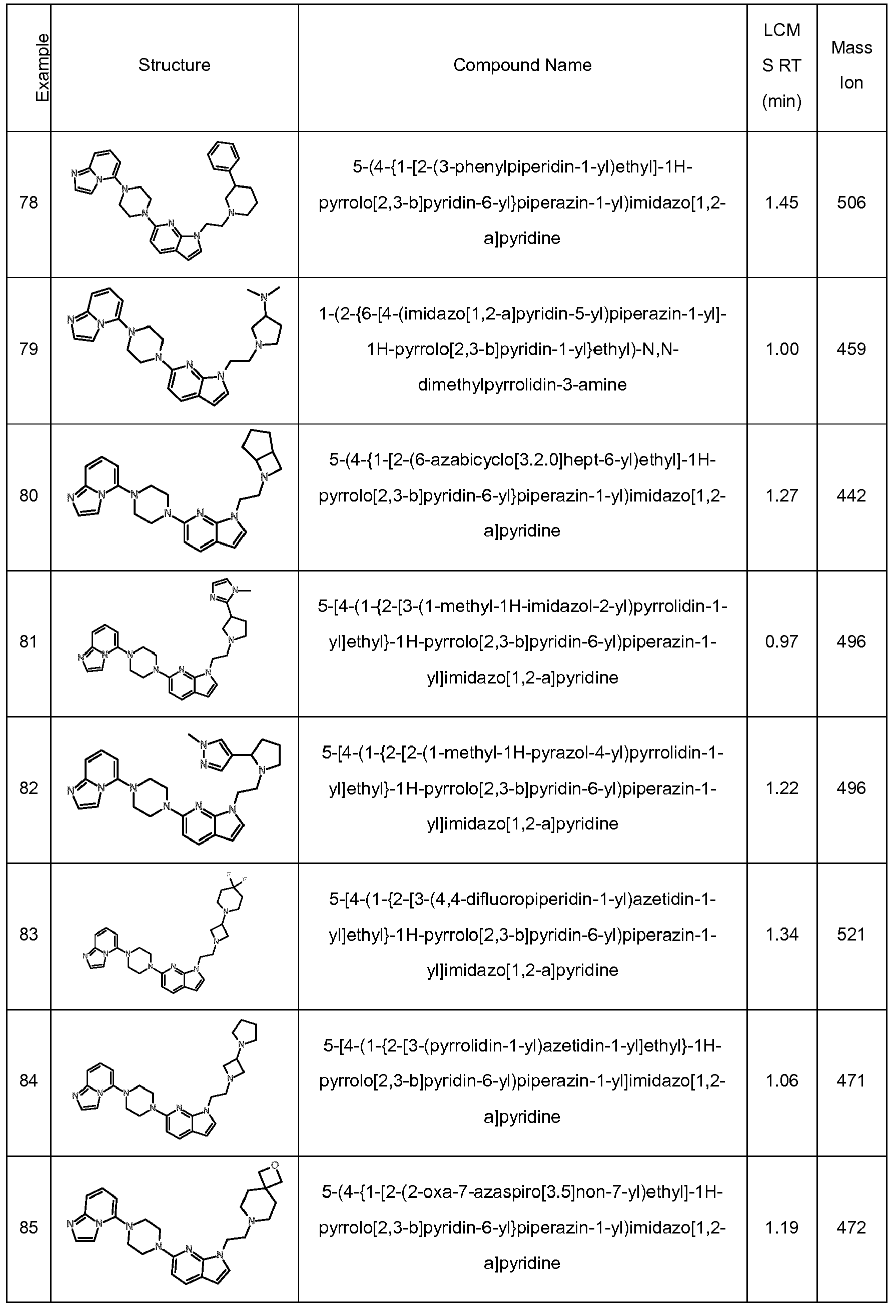

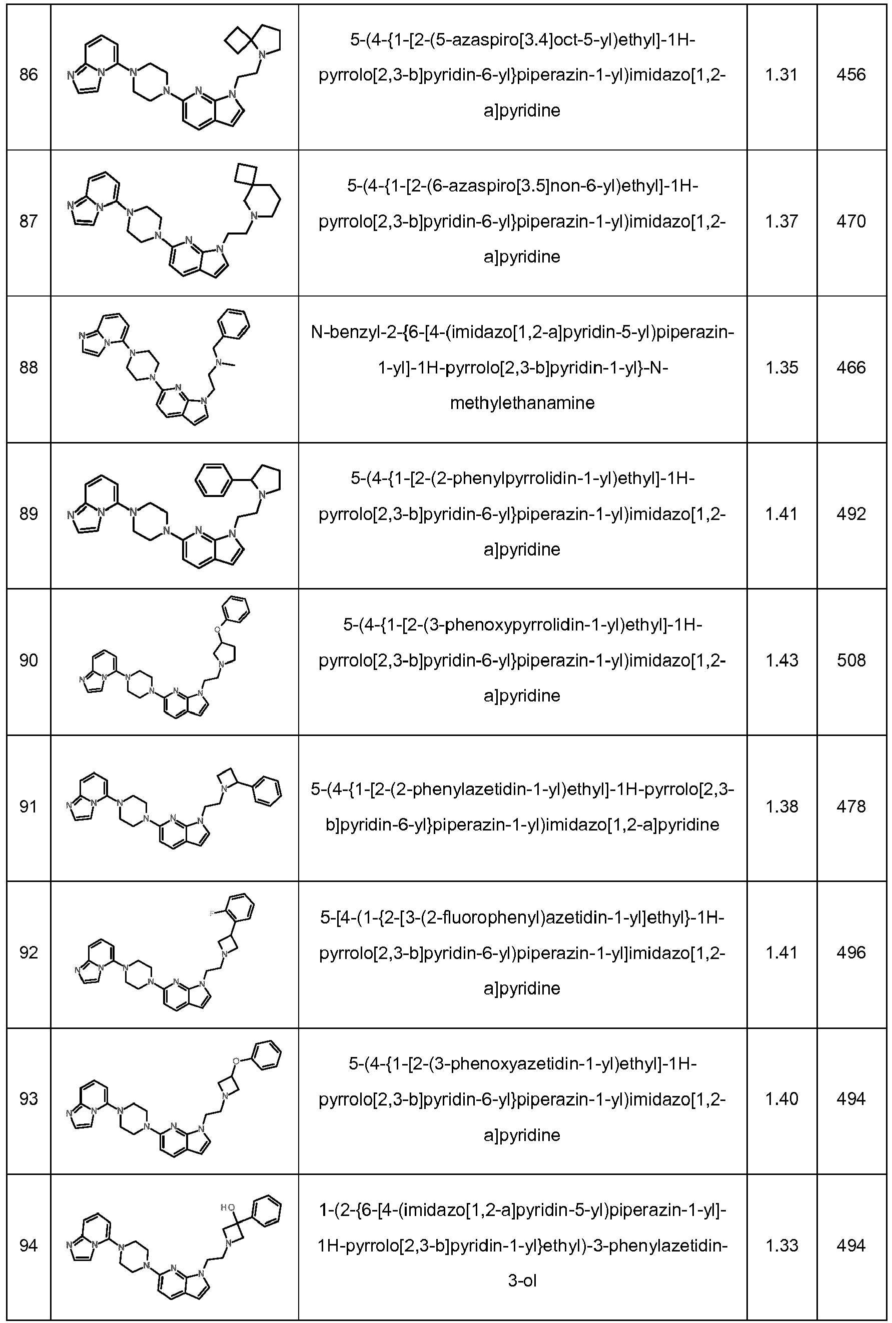

### Examples 95-98

Intermediate 26 (70 mg, 0.11 mmol), boronic ester (2 eq., 0.23 mmol), K₂CO₃ (2 eq., 0.23 mmol) and PdCl2(dppf)-DCM complex (0.1 equiv., 0.01 mmol) were dissolved in 1,4-dioxane (0.1 M, 1 mL). H₂O (1 M, 0.1 mL) was added and the mixture degassed for 1 min. The mixture was stirred overnight at 100 °C. The mixture was cooled to ambient temperature and DCM (3 mL) and H₂O (2 mL) were added and the layers separated. TFA (1 mL) was added to the DCM solution and the reaction mixture was stirred at r.t. for 2 hours. The residue was filtered through an SCX column, washing with MeOH and DCM, and then eluting the product eluted with NH₃ (7N) in MeOH. The volatiles were removed under vacuum and the residue purified by reverse phase column chromatography.

Examples 95-98 were synthesised from Intermediate 26 and the appropriate commercially available boronic ester in accordance with the foregoing procedure.

Examples were analysed by LCMS Method 1.

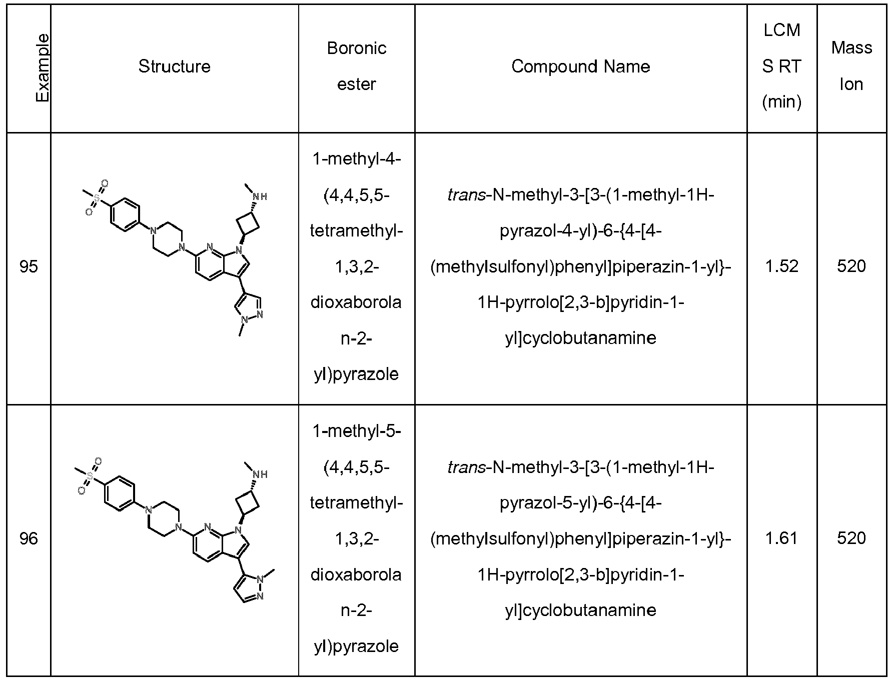

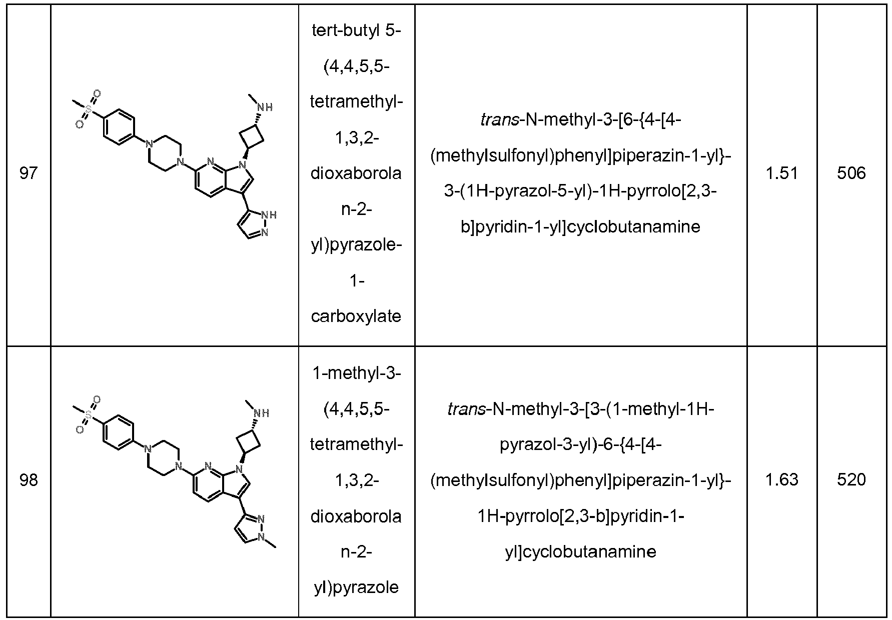

### Examples 99-110

Examples 99-110 were synthesised from the appropriate amine and aldehyde in accordance with General Procedure 12 followed by General Procedure 13.

Examples 99-110 were analysed by LCMS Method 1.

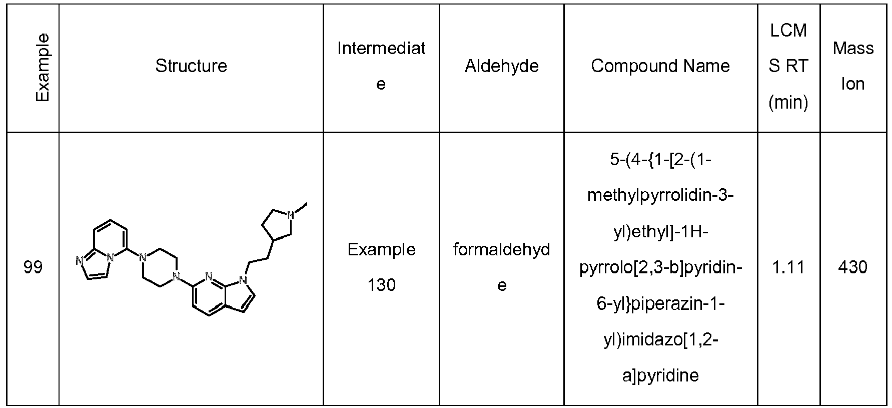

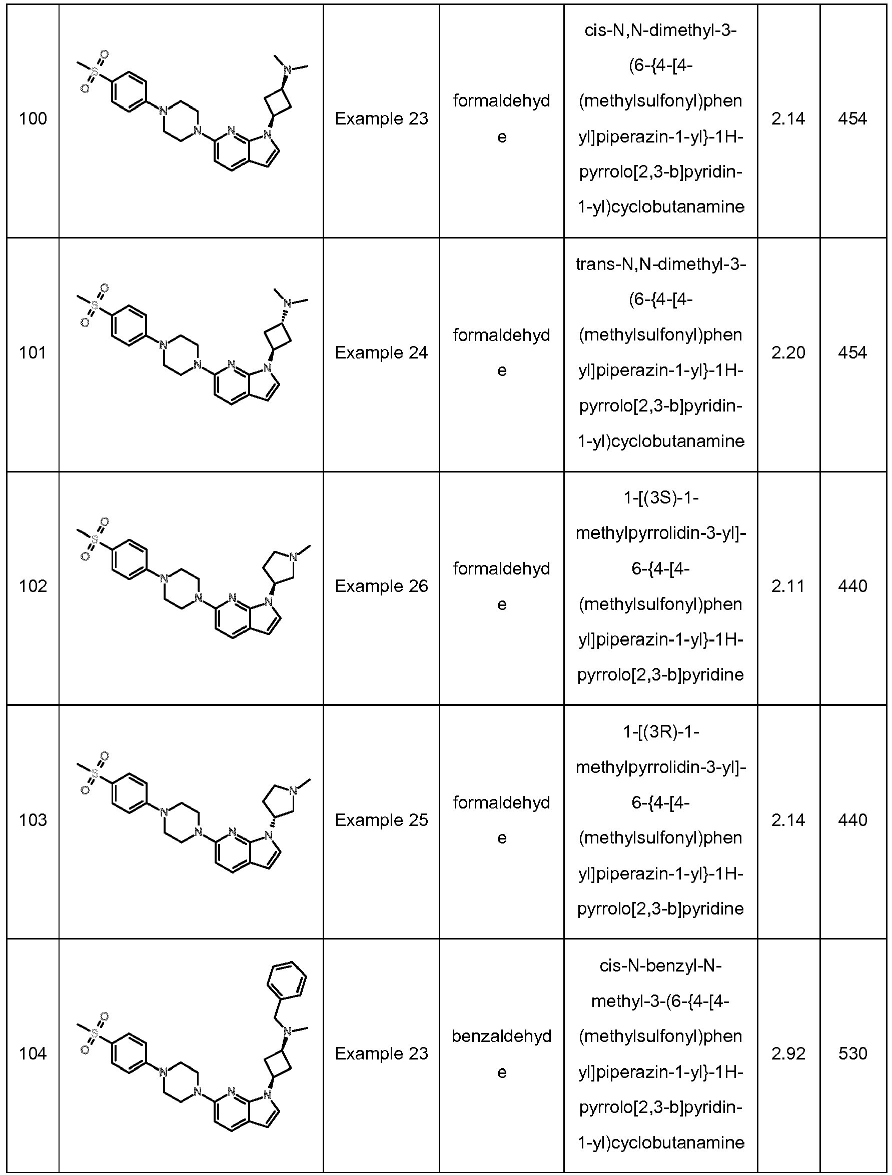

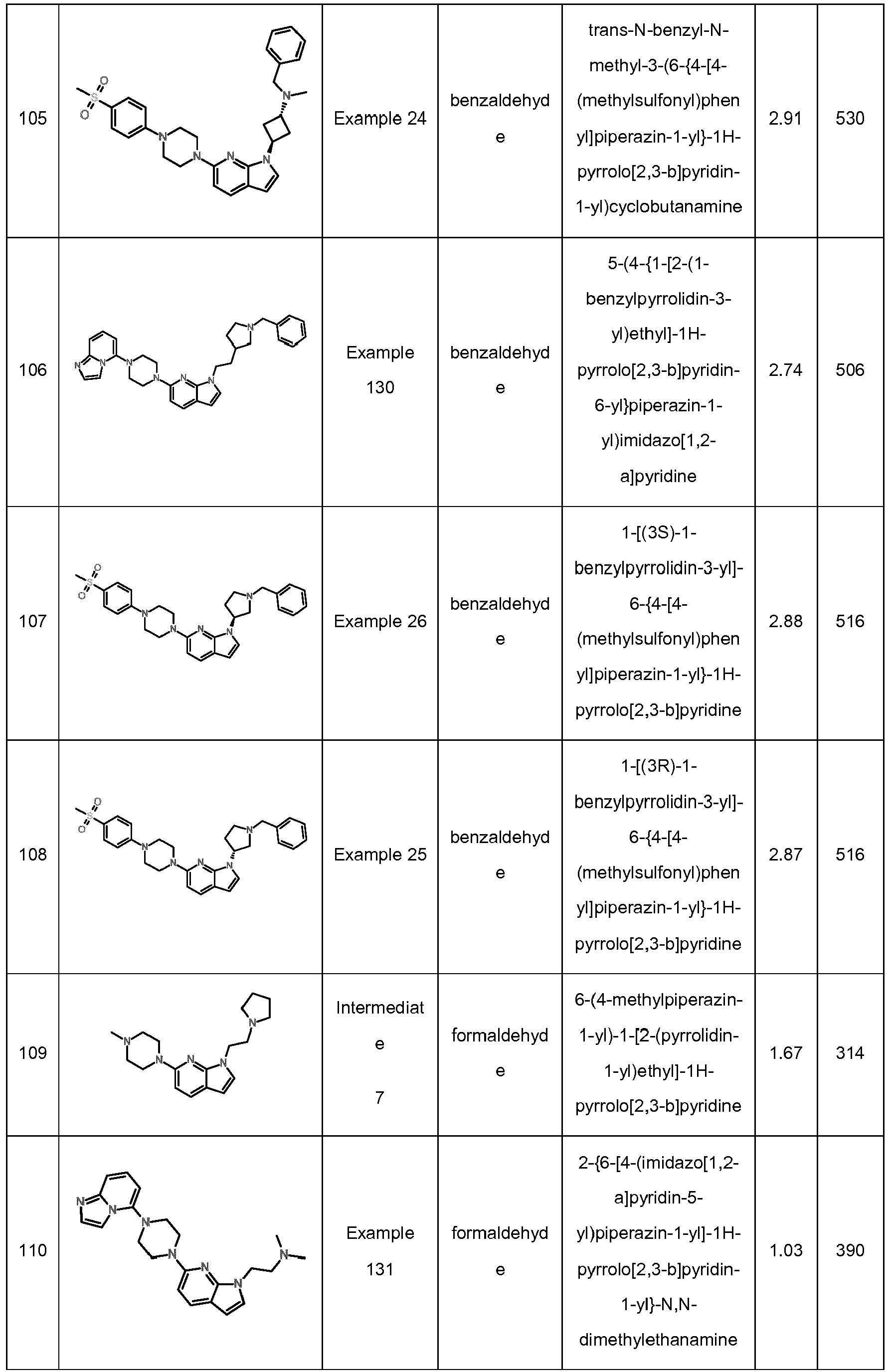

### Example 111

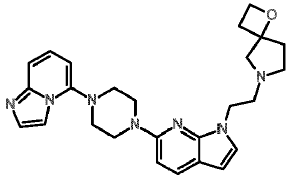

### 5-(4-{1-[2-(1-oxa-6-azaspiro[3.4]oct-6-yl)ethyl]-1H-pyrrolo[2,3-b]pyridin-6-yl}piperazin-1-yl)imidazo[1,2-a]pyridine

Example 111 was prepared following General Procedure 8 using Intermediate 18 (50 mg, 0.09 mmol) and 1-oxa-7-azaspiro[3.4]octane (17 mg, 1.5 eq.). The reaction mixture was then heated at 80 °C overnight in a sealed vial. The reaction was cooled down to ambient temperature and H₂O and DCM added. The layers were separated and the aq. phase was extracted with DCM. The solvent was removed under vacuum and the residue was purified by chromatography gradient elution from DCM to 30% MeOH in DCM to yield Example 111 (5 mg, 12% yield).
LCMS (Method 1, ES+) 2.01 min, 458 m/z (M+H)⁺.

### Example 112

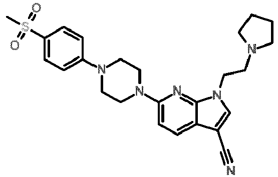

### 6-{4-[4-(methylsulfonyl)phenyl]piperazin-1-yl}-1-[2-(pyrrolidin-1-yl)ethyl]-1H-pyrrolo[2,3-b]pyridine-3-carbonitrile

6-bromo-1-(2-pyrrolidin-1-ylethyl)pyrrolo[2,3-b]pyridine-3-carbonitrile (300 mg, 0.1 mmol) was dissolved in a mixture of DMF:THF (10 mL, 1:1) The pink solution was cooled to 0 °C and sodium hydride (79 mg, 0.11 mmol, 2.2 eq., 60 mass%) added slowly portion-wise over a period of 30 minutes. 1-(2-chloroethyl)pyrrolidine hydrochloride (1.5 eq., 1.4 mmol) was then added carefully portion-wise and the reaction mixture stirred at 80°C overnight until the starting material was consumed. The crude was dissolved in DCM and H₂O added. The layers were separated and the aq. phase was extracted with DCM. The solvent was removed under vacuum and the resulting residue used in General Procedure 3 with 1-[4-(methylsulfonyl)phenyl]piperazine (1.5 eq., 0.94 mmol). The residue was purified by column chromatography gradient elution from DCM to 30% MeOH in DCM and then re-purified by reverse phase chromatography to yield Example 112 (4 mg, 1% yield).
LCMS (Method 1, ES+) 2.62 min, 479 m/z (M+H)⁺.

### Example 113

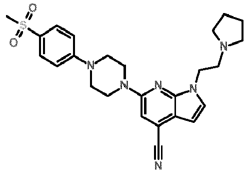

### 6-{4-[4-(methylsulfonyl)phenyl]piperazin-1-yl}-1-[2-(pyrrolidin-1-yl)ethyl]-1H-pyrrolo[2,3-b]pyridine-4-carbonitrile

4-Cyano-6-bromo-7-azaindole (200 mg, 0.9 mmol) reacted with 1-(2-chloroethyl)pyrrolidine hydrochloride (1.5 eq., 1.35 mmol) following General Procedure 2. H₂O and EtOAc were added and the layers separated and the aq. phase was extracted with EtOAc. The solvent was removed under vacuum and the residue used following General Procedure 3 without any further purification using 1-[4-(methylsulfonyl)phenyl]piperazine (1.5 equiv., 1.97 mmol). The residue was purified by column chromatography gradient elution from DCM to 30% MeOH in DCM, and then re-purified by reverse phase column chromatography give Example 113 (5 mg, 7% yield).
LCMS (Method 1, ES+) 2.26 min, 479 m/z (M+H)⁺.

### Example 114

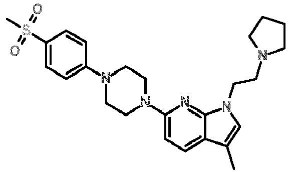

### 3-methyl-6-{4-[4-(methylsulfonyl)phenyl]piperazin-1-yl}-1-[2-(pyrrolidin-1-yl)ethyl]-1H-pyrrolo[2,3-b]pyridine

Intermediate 21 (100 mg, 0.28 mmol), cobalt tetrafluoroborate hexahydrate (0.1 mmol), tris[2-(diphenylphosphino)ethyl]phosphine (0.1 mmol) and K₂CO₃ (1 mmol, 1 mmol) were dissolved in methanol (1 mL). The mixture was heated to 100°C in a sealed vial overnight. The crude reaction mixture was filtered over celite and the volatiles removed under vacuum. The residue was submitted to alkylation following General Procedure 2 using 1-(2-chloroethyl)pyrrolidine hydrochloride (1.5 equiv.). The crude reaction mixture was diluted with EtOAc and H₂O added. The layers were separated and the aq. phase was extracted with EtOAc. Combined organic layers were dried over Na₂SO₄ and the solvent was removed under vacuum. The residue was filtered over celite and rinsed with DCM. After removing the solvent, the residue was purified by reverse phase column chromatography to give Example 114 (2 mg, 2% yield).
LCMS (Method 1, ES+) 2.83 min, 468 m/z (M+H)⁺.

### Example 115

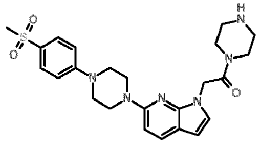

### 2-(6-{4-[4-(methylsulfonyl)phenyl)piperazin-1-yl}-1H-pyrrolo[2,3-b]pyridin-1-yl)-1-(piperazin-1-yl)ethanone

6-bromo-1H-pyrrolo[2,3-b]pyridine (100 mg, 0.5 mmol) and 1-BOC-4-chloroacetyl-piperazine (1 eq., 0.5 mmol) were reacted following General Procedure 2. H₂O was carefully added and then DCM was added. The crude product was reacted with 1-[4-(methylsulfonyl)phenyl]piperazine following General Procedure 3 and quenched with H₂O and then DCM was added. The layers were separated and solvent removed under vacuum. The crude residue was dissolved in DCM (0.5 mmol/mL) and TFA (1 mL) added, and stirred at ambient temperature until the reaction completed. The volatiles were removed under vacuum and the residue was filtered through celite. The crude product was purified by reverse phase column chromatography yield Example 115 (14 mg, 17% yield over two steps).
LCMS (Method 1, ES+) 1.57 min, 483 m/z (M+H)⁺.

### Example 116

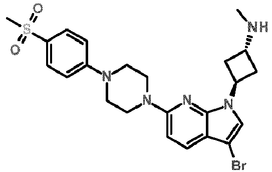

### trans-3-(3-bromo-6-{4-[4-(methylsulfonyl)phenyl]piperazin-1-yl}-1H-pyrrolo[2,3-b]pyridin-1-yl)-N-methylcyclobutanamine

Intermediate 29 (550 mg, 1.02 mmol) was dissolved in DCM (30 mL) and cooled to 0 °C. NBS (165 mg, 0.92 mmol) was added portion-wise. The reaction mixture was stirred in a cooling bath for 10 min then quenched with saturated NaHCO₃ aq. solution (20 mL) and diluted with DCM (20 mL). The aqueous layer was extracted with DCM (2 x 10 mL), the combined organics were dried over Na₂SO₄ and concentrated under reduced pressure. The crude was purified by flash chromatography gradient elution from 20% EtOAc to EtOAc and then to DCM in MeOH giving a mixture of brominated products. The white solid was dissolved in DCM (0.25 M) and TFA (5 mL) and stirred at r.t. until completion of the reaction. The volatiles were removed under vacuum and the residue was dissolved in MeOH and filtered through an SCX column, washing with MeOH and DCM and then eluting with NH₃ (4M) in MeOH. The solvent was removed under vacuum and the residue was purified by preparative HPLC affording Example 116 (7 mg, 1 % yield).
LCMS (Method 1, ES+) 1.89 min, 518 & 520 m/z (M+H)⁺.

### Example 117

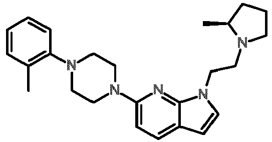

### 6-[4-(2-methylphenyl)piperazin-1-yl]-1-{2-(2S)-2-methylpyrrolidin-1-yl]ethyl}-1H-pyrrolo[2,3-b]pyridine

In a sealed vial Intermediate 20 (45 mg, 0.09 mmol) was dissolved in acetonitrile (2 mL) and triethylamine (0.03 mL, 0.2 mmol) added followed by (S)-2-methylpyrrolidine hydrochloride (15 mg, 0.12 mmol). The reaction mixture was heated at 80 °C then cooled to room temperature and passed through an SCX cartridge washing with MeOH then eluting 2M NH₃ in MeOH. The ammonia in methanol solution was concentrated under reduced pressure and the crude residue purified by reverse phase column chromatography to give the title compound (16 mg, 43% yield)
LCMS (Method 1, ES+) 3.28 min, 404 m/z (M+H)⁺.

### Example 118

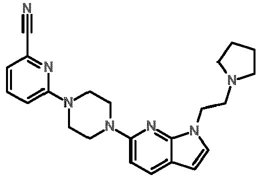

### 6-(4-{1-[2-(pyrrolidin-1-yl)ethyll-1H-pyrrolo[2,3-b]pyridin-6-yl}piperazin-1-yl)pyridine-2-carbonitrile

A mixture of Intermediate 7 (107 mg, 0.35 mmol), 2-cyano-6-fluoropyridine (44 mg, 0.36 mmol), and potassium carbonate (100 mg, 0.71 mmol) was dissolved in dimethylsulfoxide (2 mL) and heated at 100 °C overnight. The cooled reaction mixture was then treated with an aqueous work up, the organic solvent dried (Na₂SO₄) and concentrated under reduced pressure. A third of the crude residue was purified by reverse phase column chromatography to give the title compound (19.5 mg, 14% yield).
LCMS (Method 1, ES+) 2.53 min, 402 m/z (M+H)⁺.

### Example 119

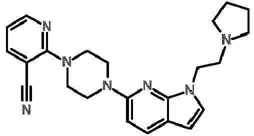

### 2-(4-{1-[2-(pyrrolidin-1-yl)ethyl]-1H-pyrrolo[2,3-b]pyridin-6-yl]piperazin-1-yl)pyridine-3-carbonitrile

A mixture of intermediate 7 (90 mg, 0.30 mmol) and 3-cyano-2-fluoropyridine (45 mg, 0.36 mmol) was dissolved in tetrahydrofuran (2 mL) and triethylamine (0.1 mL, 0.7 mmol) added. The resulting yellow solution was heated at 100 °C overnight. The cooled reaction mixture was then treated with an aqueous work up and the organic layer concentrated under reduced pressure. Half of the crude residue was purified by reverse phase column chromatography to give the title compound (31.2 mg, 25% yield).
LCMS (Method 1, ES+) 2.39 min, 402 m/z (M+H)⁺.

### Example 120

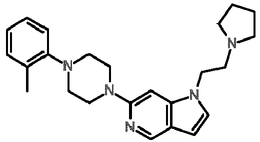

### 6-{4-(2-methylphenyl)piperazin-1-yl]-1-[2-(pyrrolidin-1-yl)ethyl]-1H-pyrrolo[3,2-c]pyridine

Example 120 was prepared according to General Procedure 2 with Intermediate 25 (46 mg, 0.16 mmol) and 1-(2-chloroethyl)pyrrolidine hydrochloride (33 mg, 0.19 mmol). Reverse phase column chromatography for the final purification gave the title compound (16 mg, 26% yield).
LCMS (Method 1, ES+) 2.61 min, 390 m/z (M+H)⁺.

### Example 121

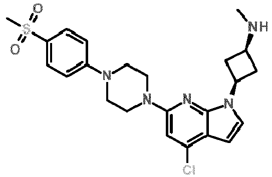

### cis-3-(4-chloro-6-{4-[4-(methylsulfonyl)phenyl]piperazin-1-yl)-1H-pyrrolo[2,3-b]pyridin-1-yl)-N-methylcyclobutanamine

Intermediate 27 (330 mg, 1 eq.) and 1-[4-(methylsulfonyl)phenyl]piperazine (1.1 eq., 0.88 mmol) were reacted following General Procedure 3. The residue was purified by flash column chromatography using gradient elution from hexane to EtOAc. The obtained product was dissolved in DCM (5 mL) and TFA (1 mL) added. The mixture was stirred for 1 hour at ambient temperature. Then the mixture was filtered through an SCX-column and rinsed with DCM and MeOH. The product was eluted with NH₃ (4N) in MeOH. The volatiles were removed under vacuum and then the residue was purified by reverse phase column chromatography to yield the title compound as a white solid (19 mg, 5% yield).
LCMS (Method 1, ES+) 1.81 min, 474 m/z (M+H)⁺.

### Example 122

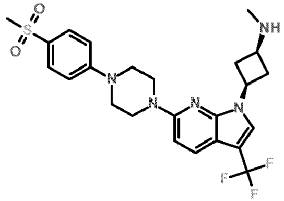

### cis-N-methyl-3-[6-{4-[4-(methylsulfonyl)phenyl]piperazin-1-yl)-3-(trifluoromethyl)-1H-pyrrolo[2,3-b]pyridin-1-yl]cyclobutanamine

Intermediate 28 (160 mg, 0.36 mmol) and 1-[4-(methylsulfonyl)phenyl]piperazine (1.1 eq., 0.4 mmol) were reacted following General Procedure 3. The residue was purified by flash column chromatography using gradient elution from hexane to EtOAc. The resulting residue was dissolved in DCM (5 mL) and TFA (2 mL) was added. The mixture was stirred at ambient temperature for 2 hours and the volatiles removed under vacuum. The residue was purified by flash column chromatography using gradient elution from hexane to EtOAc, and then 20% MeOH in EtOAc to give Example 122 (2 mg, 8% yield).
LCMS (Method 1, ES+) 1.92 min, 408 m/z (M+H)⁺.

### Example 123

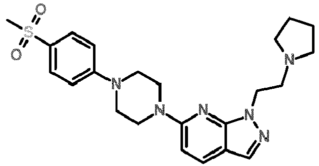

### 6-{4-[4-(methylsulfonyl)phenyl]piperazin-1-yl}-1-[2-(pyrrolidin-1-yl)ethyl]-1H-pyrazolo[3,4-b]pyridine

6-bromo-1H-pyrrolo[3,4-b]pyridine (100 mg, 0.51 mmol) and 1-(2-chloroethyl)pyrrolidine hydrochloride (1.2 eq., 0.61 mmol) were reacted following General Procedure 2. After work-up, the mixture was reacted with 1-[4-(methylsulfonyl)phenyl]piperazine (1.5 eq., 0.76 mmol) following General Procedure 3. The residue was purified by reverse phase column chromatography to yield the title compound as a white solid (8 mg, 3% yield).
LCMS (Method 1, ES+) 1.59 min, 455 *mlz* (M+H)⁺.

### Example 124

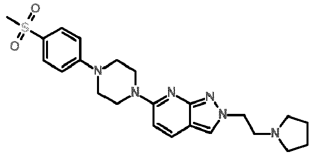

### 6-{4-[4-(methylsulfonyl)phenyl]piperazin-1-yl}-2-[2-(pyrrolidin-1-yl)ethyl]-2H-pyrazolo[3,4-b]pyridine

Example 124 was prepared according to the procedure described for Example 123 and isolated as an alternative regioisomer during purification.
LCMS (Method 1, ES⁺) 1.82 min, 455 *m*/*z* (M+H)⁺.

### Example 125

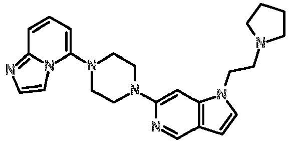

### 5-(4-{1-[2-(pyrrolidin-1-yl)ethtl-1H-pyrrolo[3,2-c]pyridin-6-yl}piperazin-1-yl)imidazo[1,2-a]pyridine

Intermediate 30 (75 mg, 0.30 mmol) and Intermediate 5 (75 mg, 0.37 mmol) were reacted using General Procedure 3 to give the title compound (63 mg, 50% yield).
LCMS (Method 1, ES⁺) 1.79 min, 416 *m*/*z* (M+H)⁺.

### Example 126

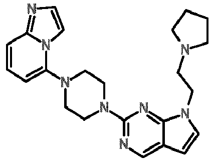

### 2-[4-(imidazo[1,2-a]pyridin-5-yl]piperazin-1-yl]-7-[2-pyrrolidin-1-yl)ethyl]-7H-pyrrolo[2,3-d]pyrimidine

A mixture of 2-chloro-7H-pyrrolo[2,3-D]pyrimidine (120 mg, 0.76 mmol) and Intermediate 5 (117 mg, 0.58 mmol) were suspended in 1-butanol (3 mL) and trifluoroacetic acid (0.07 mL, 0.9 mmol) added. The reaction mixture was then heated at 120 °C overnight. The reaction mixture was cooled to room temperature and filtered through a SCX cartridge washing first with methanol then eluting with 2 M ammonia in methanol. The ammonia in methanol solution was concentrated under reduced pressure and the residue partially purified using silica column chromatography. The crude mixture was then reacted with 1-(2-chloroethyl)pyrrolidine hydrochloride (102 mg, 0.59 mmol) according to General Procedure 2 to give the title compound (38 mg, 15% yield).
LCMS (Method 1, ES⁺) 1.92 min, 417 *m*/*z* (M+H)⁺.

### Example 127

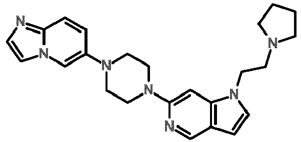

### 6-(4-{1-[2-(pyrrolidin-1-yl)ethyl]-1H-pyrrolo[3,2-c]pyridin-6-yl}piperazin-1-yl)imidazo[1,2-a]pyridine

The title compound was prepared according to General Procedure 5 using intermediate 31 (48 mg, 0.16 mmol) instead of intermediate 7. An aqueous work up was not preformed and the crude reaction mixture was directly filtered through celite followed by SCX filtration. The SCX cartridge was washed with methanol the crude product eluted with 2 M ammonia in methanol. Final purification of the residue by reverse phase column chromatography gave the title compound (3 mg, 4% yield).
LCMS (Method 1, ES⁺) 1.58 min, 416 *m*/*z* (M+H)⁺.

### Example 128

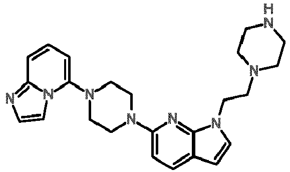

### 5-(4-{1-[2-(piperazin-1-yl)ethyl]-1H-pyrrolo[2,3-b]pyridin-6-yl}piperazin-1-yl)imidazo[1,2-a]pyridine

Using General Procedure 3, Intermediate 34 (175 mg, 0.43 mmol) and Intermediate 5 (90 mg, 0.44 mmol) gave tert-butyl 4-[2-[6-(4-imidazo[1,2-a]pyridin-6-ylpiperazin-1-yl)pyrrolo[2,3-b]pyridin-1-yl]ethyl]piperazine-1-carboxylate. This was then dissolved in dichloromethane (3 mL) and treated with trifluoracetic acid (1 mL). The reaction mixture was stirred at room temperature for 36 h then passed through an SCX cartridge, washing first with methanol then eluting with 2 M ammonia in methanol. The ammonia in methanol solution was concentrated under reduced pressure and the residue purified by reverse phase chromatography to give the title compound (10 mg, 8% yield).
LCMS (Method 1, ES⁺) 1.69 min, 431 *m*/*z* (M+H)⁺.

### Example 129

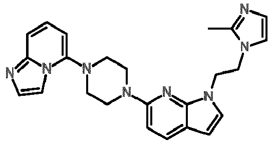

### 5-(4-{1-[2-(2-methyl-1H-imidazol-1-yl)ethyl]-1H-pyrrolo[2,3-b]pyridin-6-yl}piperazin-1-yl)imidazo[1,2-a]pyridine

Intermediate 32 (100 mg, 0.28 mmol) and intermediate 5 (60 mg, 0.29 mmol) were treated according to General Procedure 3 to give the title compound (17 mg, 13% yield).
LCMS (Method 1, ES⁺) 1.82 min, 427 *m*/*z* (M+H)⁺.

### Example 130

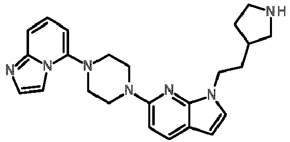

### 5-(4-{1-[2-(pyrrolidin-3-yl)ethyl]-1H-pyrrolo[2,3-b]puridin-6-yl}piperazin-1-yl)imidazo[1,2-a]pyridine

Intermediate 35 (130 mg, 0.28 mmol) and Intermediate 5 (60 mg, 0.29 mmol) were treated according to General Procedure 3 to give tert-butyl 3-[2-[6-(4-imidazo[1,2-a]pyridin-5-ylpiperazin-1-yl)pyrrolo[2,3-b]pyridin-1-yl]ethyl]pyrrolidine-1-carboxylate, which was dissolved in DCM (4 mL) and treated with TFA (1 mL). The reaction mixture was stirred at room temperature for 3 hours then passed through an SCX cartridge, washing first with methanol then eluting with 2 M ammonia in methanol. The ammonia in methanol solution was concentrated under reduced pressure and a fraction of the residue (50 mg) purified by reverse phase chromatography to give the title compound (11 mg).
LCMS (Method 1, ES⁺) 1.62 min, 416 *m*/*z* (M+H)⁺.

### Example 131

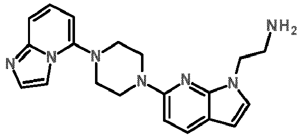

### 2-{6-[4-(imidazo[1,2-a]pyridin-5-yl)piperazin-1-yl]-1H-pyrrolo[2,3-b]pyridin-1-yl}ethanamine

Intermediate 36 (110 mg, 0.23 mmol) was dissolved in dichloromethane (3 mL) and trifluoroacetic acid (0.5 mL) added. The reaction mixture was stirred at room temperature for 3 h then passed through an SCX cartridge eluting with methanol then with 2 M ammonia in methanol. 20% of the resulting residue was purified by reverse phase column chromatography to afford the title compound (6 mg, 7% yield)
LCMS (Method 1, ES⁺) 1.60 min, 362 *m*/*z* (M+H)⁺.

### Examples 132-134

The following compounds were synthesized from Intermediate 7 and the appropriate aryl bromide in accordance with General Procedure 5.

Examples were analysed by LCMS Method 3.

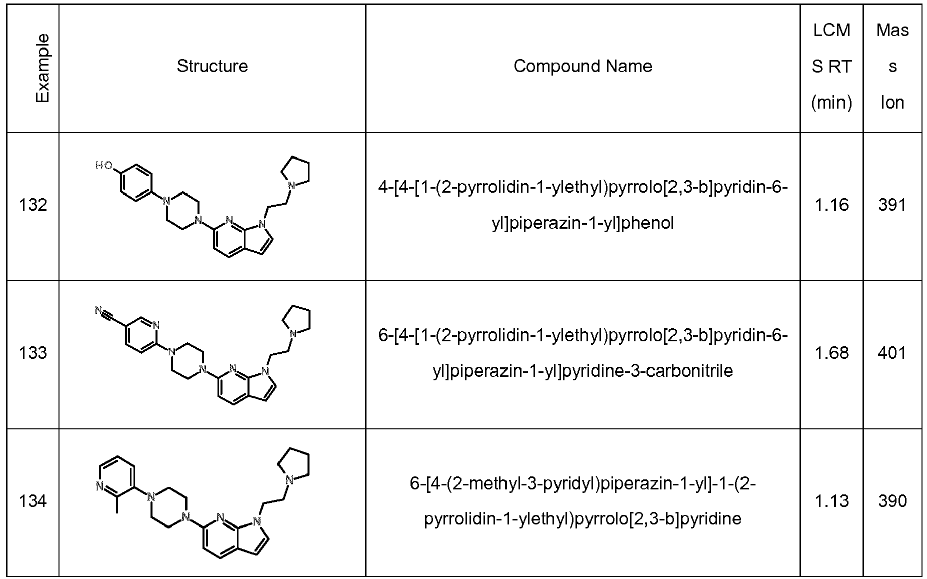

### Examples 135-139

Examples 135-139 were prepared according to General Procedure procedure 6 from Intermediate 7 and the appropriate carboxylic acid and analysed by LCMS Method 3.

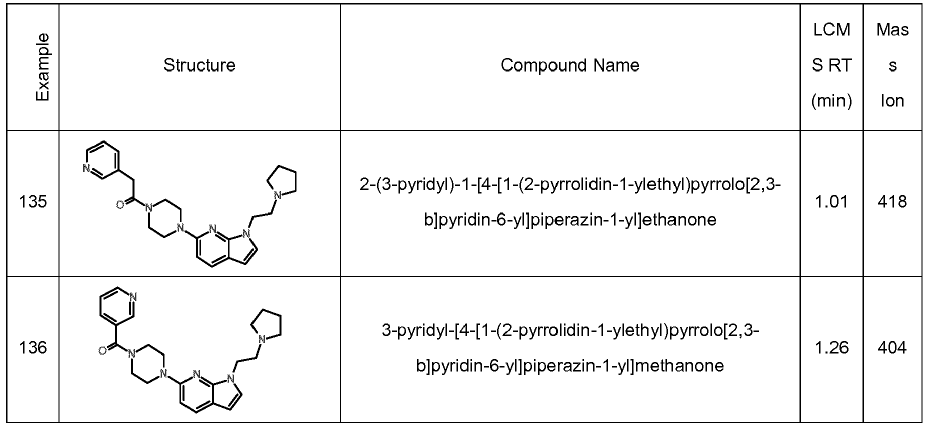

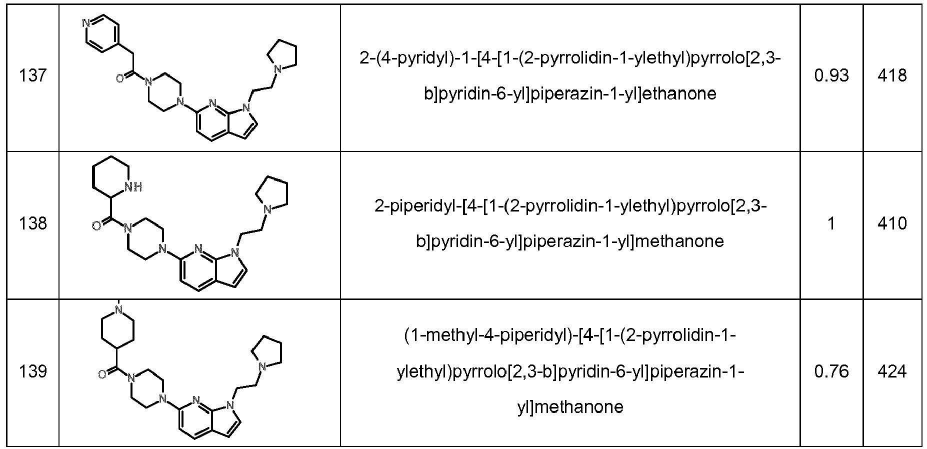

### Examples 140-143

The following compounds were synthesised from Intermediate 20 and the appropriate amine in accordance with General Procedure 7.

Example were analysed by LCMS Method 1

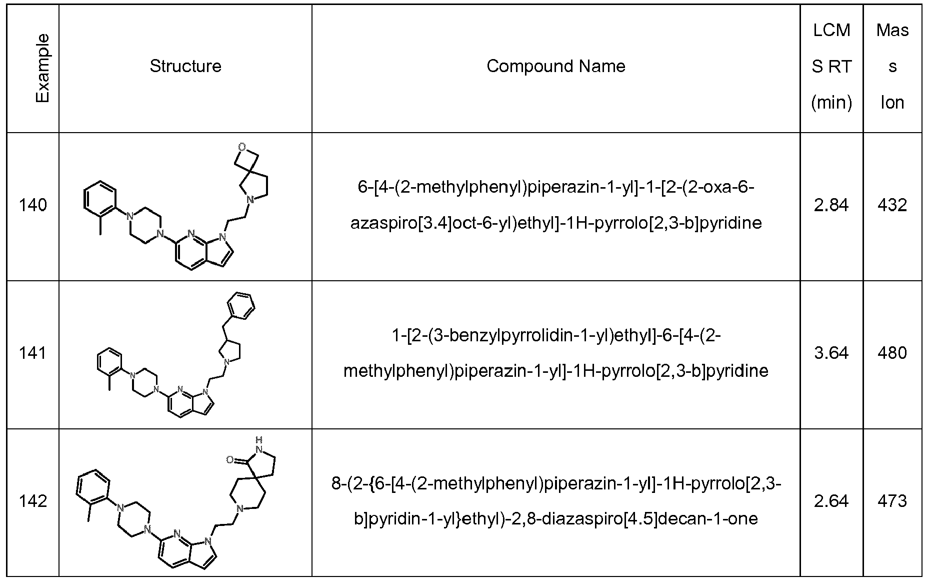

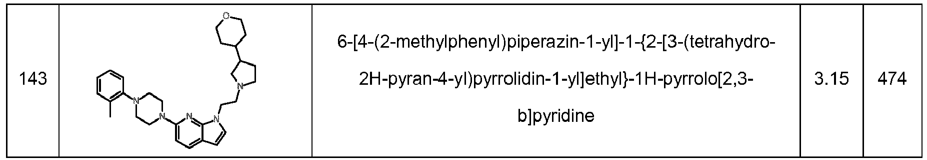

### Example 144

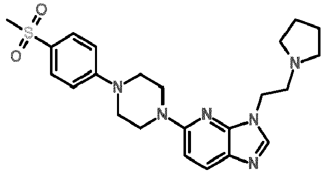

### 5-{4-[4-(methylsulfonyl)phenyl]piperazin-1-yl}-3-[2-pyrrolidin-1-yl)ethyl]-3H-imidazo[4,5-b]pyridine

5-Bromo-1H-imidazo[4,5,b]pyridine (150 mg, 0.75 mmol) and 1-(2-chloroethyl)pyrrolidine hydrochloride (1.2 eq., 0.8 mmol) were reacted following General Procedure 2. After an aq. work-up, the crude residue was reacted with 1-[4-(methylsulfonyl)phenyl]piperazine (1.5 eq.) following General Procedure 3. The residue was purified by reverse phase column chromatography to yield Example 144 (2 mg, 1% yield).
LCMS (Method 1, ES+) 1.52 min, 455 m/z (M+H)⁺.

### Example 145

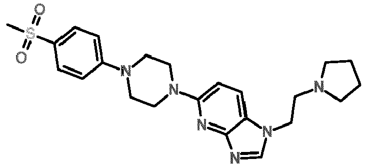

### 5-{4-[4-(methylsulfonyl)phenyl]piperazin-1-yl}-1-[2-(pyrrolidin-1-yl)ethyl]-1H-imidazo[4,5-b]pyridine

Example 145 was prepared according to the procedure described for Example 144 and isolated as an alternative regioisomer during purification.
LCMS (Method 1, ES⁺) 1.61 min, 455 m/z (M+H)⁺.

### Examples 146-148

The following Examples were synthesised using General Procedure 3 using Intermediate 6 and an appropriate amine.

Examples were analysed using LCMS Method 1.

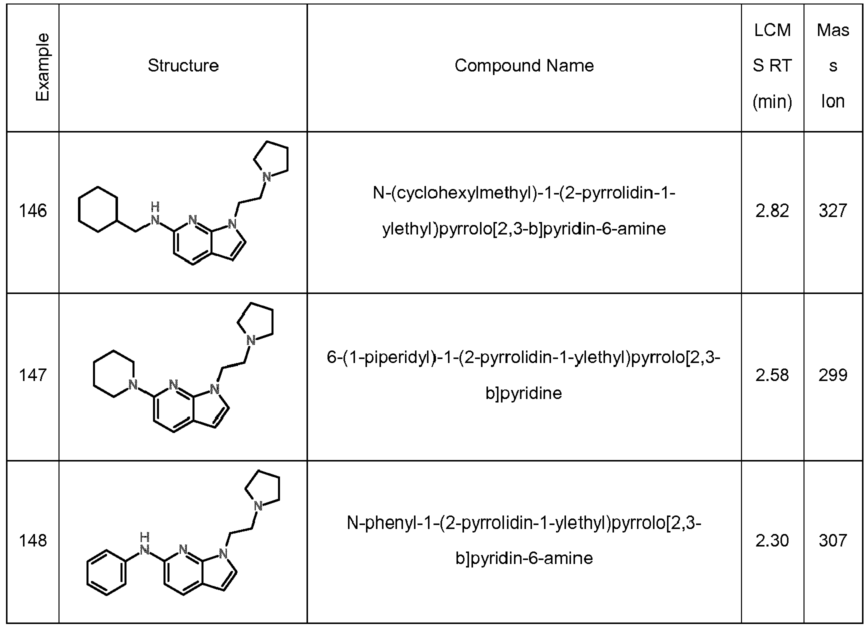

### Example 149

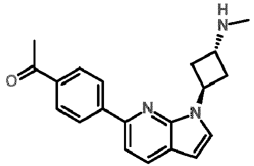

### 1-(4-{1-[trans-3-(methylamino)cyclobutyl]-1H-pyrrolo[2,3-b]pyridin-6-yl}phenyl)ethanone

Intermediate 38 (100 mg, 0.3 mmol) and 4-acetylphenylboronic acid (1.5 eq., 0.4 mmol), were reacted following General Procedure 10. The residue was dissolved in DCM (0.05 M) and TFA (1 mL) was added at 0°C, then the reaction was stirred at ambient for an additional 2 hours. The residue was filtered over a SCX column and washed with DCM and MeOH, then eluted with methanolic NH₃ (4N). The product was purified by reverse phase chromatography to yield Example 149 (35 mg, 42% yield).
LCMS (Method 1, ES+) 1.71 min, 320 m/z (M+H)⁺.

### Example 150

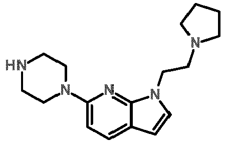

### 6-piperazin-1-yl-1-(2-pyrrolidin-1-ylethyl)pyrrolo[2,3-b]pyridine

Example 150 is also Intermediate 7. Synthesis and characterisation are described above.

### Example 151

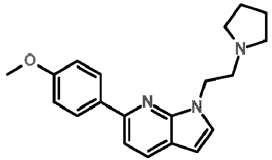

### 6-(4-methoxphenyl)-1-(2-pyrrolidin-1-ylethyl)pyrrolo[2,3-b]pyridine

Example 151 was prepared according to a procedure similar to that described for Example 149 using Intermediate 6 in place of Intermediate 38 and 4-methoxyphenylboronic acid in place of 4-acetylphenylboronic acid.
LCMS (Method 1, ES+) 2.39 min, 322 m/z (M+H)⁺.

### Examples 152-154

Examples 152-154 were prepared in two steps according to General Procedure 3 followed by general procedure 13 and analysed by LCMS Method 1.

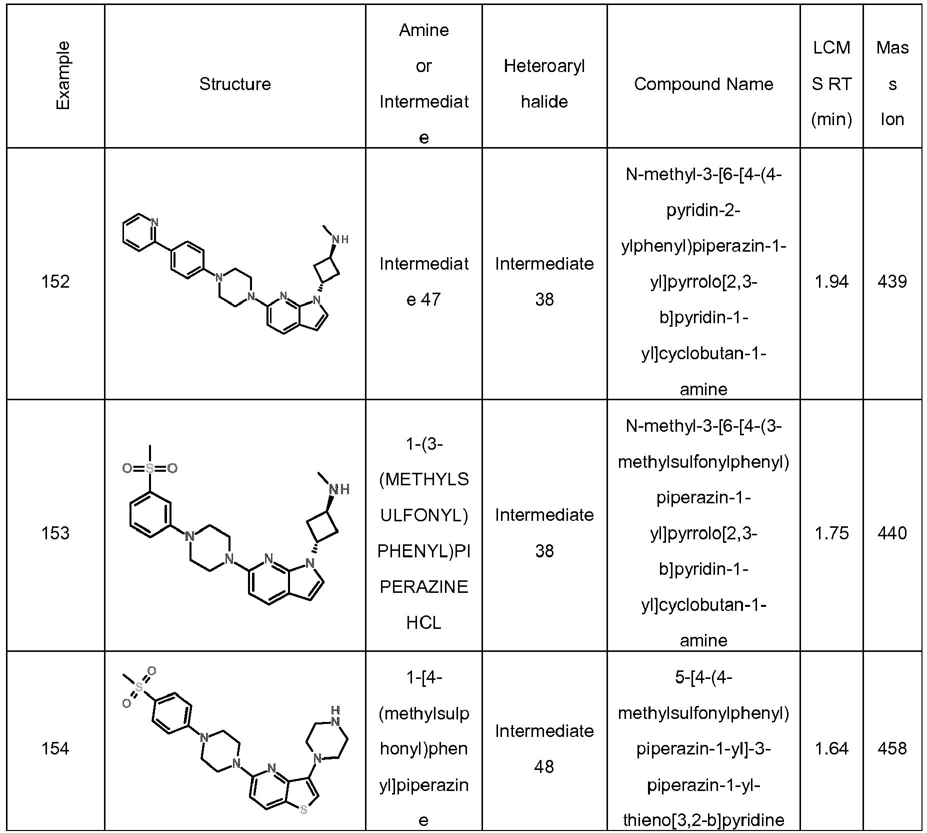

### Example 155

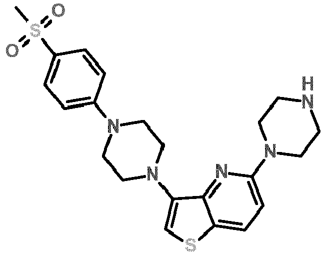

### 3-[4-(4-methylsulfonylphenyl)piperazin-1-yl]-5-piperazin-1-yl-thieno[3,2-b]pyridine

Examples 155 was prepared in two steps according to General Procedure 14 followed by general procedure 13 and analysed by LCMS Method 1.
LCMS (Method 1, ES+) 1.69 min, 458 m/z (M+H)⁺.

### Example 156

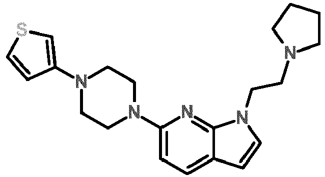

### 1-(2-pyrrolidin-1-ylethyl)-6-[4-(3-thienyl)piperazin-1-yl]pyrrolo[2,3-b]pyridine

Example 156 was prepared from Intermediate 7 and 3-bromothiophene in accordance with General Procedure 5 and analysed by LCMS Method 3.
LCMS (Method 3, ES+) 1.73 min, 382 m/z (M+H)⁺.

### Example 157

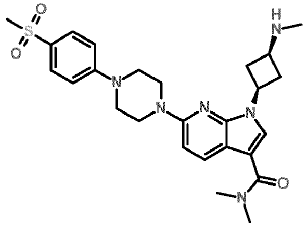

### cis-N,N-dimethyl-1-[3-(methylamino)cyclobutyl]-6-[4-(4-methylsulfonylphenyl)piperazin-1-yl]pyrrolo[2,3-b]pyridine-3-carboxamide

Intermediate 53 (20 mg, 0.05 mmol), dimethylamine (1.2 equiv., 0.06 mmol, 2 mmol/mL) and HATU (25 mg, 0.07 mmol, 1.4 equiv.) were dissolved in DMF (0.25 mL, 0.25 M) and DIPEA (0.04 mL, 0.2 mmol, 4 equiv) was added. The mixture was then stirred at room temperature under N₂ until the reaction was complete (1h). Then, DCM and NH₄Cl aq. sat solution were added to the reaction mixture and the layers were separated. The aq. phase was extracted with DCM, the layers were separated and the volatiles were removed under vacuum. The resulting residue was dissolved in 1,4-dioxane (0.05 M) and was reacted with 1-[4-(methylsulfonyl)phenyl]piperazine (1.5 equiv., 0.07 mmol) following General Procedure 3. The reaction mixture was cooled to r.t. and DCM and H₂O were added. The layers were separated and the aqueous phase was extracted with EtOAc. The combined organic layers were dried over Na₂SO₄ and the solvents were removed under vacuum.

The residue was dissolved in DCM (5 mL) and TFA (1 mL) was added. The mixture was stirred at ambient temperature for 2 hours. The reaction crude was filtered through an SCX column and washed with MeOH and DCM. The product was eluted with NH₃ (4N) in MeOH. Volatiles were removed under vacuum and the residue purified by preparative chromatography to give Example 157 (8 mg, 34% yield).
LCMS (Method 1, ES+) 1.44 min, 511 m/z (M+H)+.

### Example 158

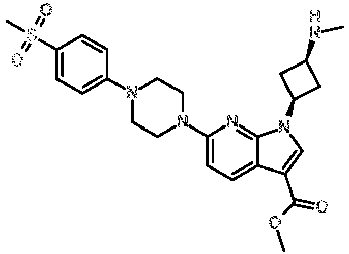

### cis-methyl 1-[3-(methylamino)cyclobutyl]-6-[4-(4-methylsulfonylphenyl)piperazin-1-yl]pyrrolo[2,3-b]pyridine-3-carboxylate

Intermediate 52 (100 mg, 0.17 mmol) was dissolved in DCM (8 mL, 0.02 M) and cooled to 0°C. TFA (2 mL) was added and the mixture stirred at r.t. for 2 h. The mixture was filtered through an SCX column and the column washed with MeOH and then eluted with 4N NH₃ in MeOH. After removing the volatiles, the residue was purified by preparative column chromatography to afford Example 158 (11 mg, 13% yield).
LCMS (Method 1, ES+) 1.67 min, 498 m/z (M+H)+.

### Example 159 - Biological assay

The ability of these compounds encompassed by the present invention to modulate 2'3'-cGAMP stimulated STING signaling as investigated in cell assays. HEK Blue ISG permeabilised cells were pre-incubated with compound for 60 mins at 37 °C, 5% CO₂. The cells were then stimulated with 2'3'-cGAMP. The effect of compound on the 2'3'-cGAMP induced production of Type I interferons is indirectly determined by an ISG54 reporter assay. Type 1 interferons produced by the cells were measured using HEK Blue detection reagent. The ability of compound to inhibit 2'3'-cGAMP stimulated production of Type I interferons from HEK Blue ISG cells was measured as pIC₅₀ WT (wild-type).

Compound activity was assayed for STING dependence using HEK Blue ISG KO STING cells (counter-screen). 2'3'-cGAMP does not induce production of Type I interferons from cells that lack the receptor STING.

In the counter-screen, compounds were tested for their ability to modulate the production of Type I interferons by pre-treating permeabilised HEK Blue ISG KO STING cells with compound for 60 mins at 37 °C, 5% CO₂ and then stimulating with interferon. The effect of compound on the interferon induced production of Type I interferons is indirectly determined by an ISG54 reporter assay. Type 1 interferons produced by the cells were measured using HEK Blue detection reagent. The ability of compound to inhibit interferon stimulated production of Type I interferons from HEK Blue ISG KO STING cells was measured as pIC₅₀ KO.
All the pharmaceutically active compounds described herein have pIC₅₀ superior to 4.5 in HEK Blue ISG cells and poorer pIC₅₀ in the HEK Blue ISG KO STING assay.

## Claims

1. A compound of general formula I, or a pharmaceutically acceptable acid addition salt, a racemic mixture or its corresponding enantiomer and/or optical isomers thereof, 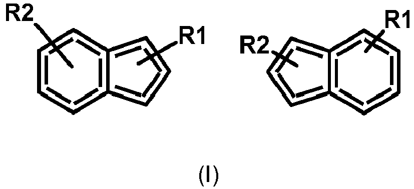 wherein
- the central core A which is the 6,5 heterobicyclic rings contains at least one heteroatom from O,N,S and C atoms can be optionally substituted by halogen, cyano, C1-6 alkyl, trifluoromethyl, difluromethyl, (C2-6) alkenyl, hydroxy, (C1-6) alkoxy, difluoromethoxy, trifluoromethoxy, trifluoroethoxy, (C1-6) alkylthio, (C1-6) alkylsulphonyl, amino, (C1-6) alkylamino, di(C1-6)alkylamino, (C1-6)alkoxy(C1-6)alkyl-amino, N-[(C1-6)alkyl]-N-[hydroxy(C1-6)alkyl]amino, (C2-6) alkylcarbonylamino, (C2-6) alkoxycarbonylamino, (C1-6) alkylsulphonylamino, formyl, (C2-6) alkylcarbonyl, carboxy, (C2-6) alkoxycarbonyl, aminocarbonyl, (C1-6) alkylaminocarbonyl, di(C1-6)alkylaminocarbonyl, aminosulphonyl, (C1-6) alkylaminosulphonyl or di(C1-6)alkylaminosulphonyl; (C3-7)heterocycloalkyl or (C3-7)spiroheterocycloalkyl, either of which groups may be optionally substituted by one or more substituents;
- R1 represents alkyl or cycloalkyl amines optionally substituted including (C1-3) aminoalkyl, (C3-7) aminocycloalkyl, (C1-3)alkylimidazole, (C1-3)alkyl isoindoline, (C1-3)alkylpiperazine, (C1-3)alkylpiperidine, (C1-3)alkyl imidazopiperazine, (C1-3)alky(C4-7)aminocycloalkyl, (C1-3)alky(C4-7)aminodicycloalkyl; and
- R2 represents aryl, heteroaryl, heterobicyclic, (C4-7) aminocycloalkyl, cycloalkyl, heterocycloalkyl, (C6-8) diaminocycloalkyl, morpholino, (C4-7)cylcoalkylmethyl, piperzinyl, piperdinyl. R2 is optionally substituted with groups including hydroxyl, (C1-6)alkyl, acetyl, halogen, cyano, C1-6 alkyl, trifluoromethyl, difluromethyl, (C2-6) alkenyl, hydroxy, (C1-6) alkoxy, difluoromethoxy, trifluoromethoxy, trifluoroethoxy, (C1-6) alkylthio, (C1-6) alkylsulphonyl, amino, (C1-6) alkylamino, di(C1-6)alkylamino, (C1-6)alkoxy(C1-6)alkyl-amino, N-[(C1-6)alkyl]-N-[hydroxy(C1-6)alkyl]amino, (C2-6) alkylcarbonylamino, (C2-6) alkoxycarbonylamino, (C1-6) alkylsulphonylamino, formyl, (C2-6) alkylcarbonyl, carboxy, (C2-6) alkoxycarbonyl, aminocarbonyl, (C1-6) alkylaminocarbonyl, di(C1-6)alkylaminocarbonyl, aminosulphonyl, (C1-6) alkylaminosulphonyl or di(C1-6)alkylaminosulphonyl; (C3-7)heterocycloalkyl or (C3-7)spiroheterocycloalkyl, either of which groups may be optionally substituted by one or more substituents.

2. A compound according to claim 1, wherein the central core A is selected from the group consisting of

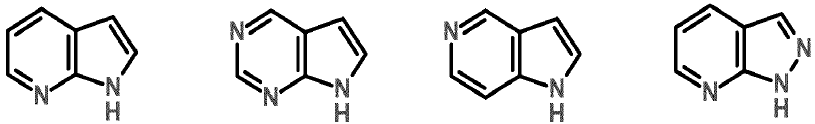

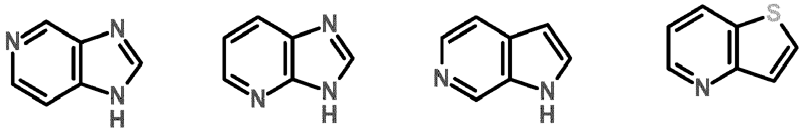

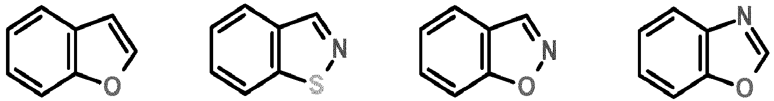

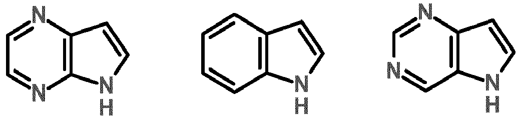

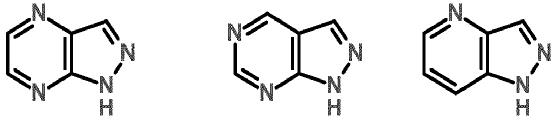

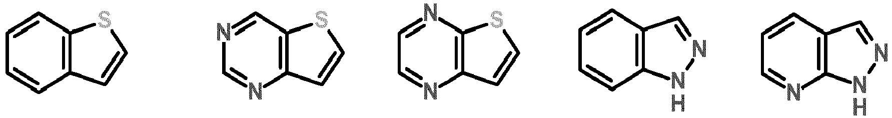

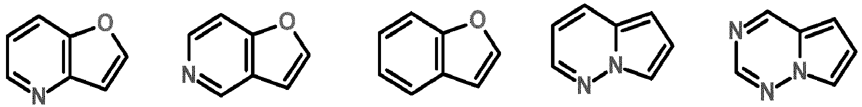

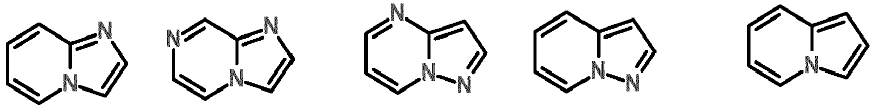

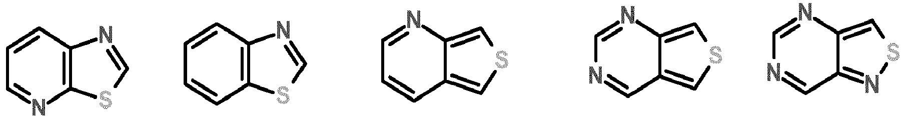

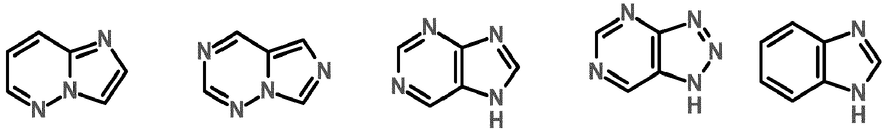

3. A compound according to claim 2, wherein the central core A is selected from the group consisting of

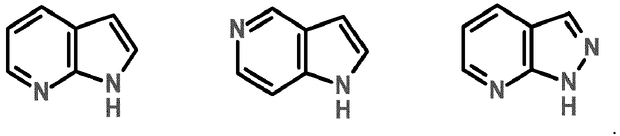

4. A compound according to claim 3, wherein the central core A is

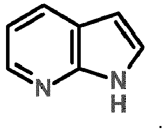

5. A compound according to to any of the preceding claims, wherein R1 is ethyl-azabicyclo[3.2.0]heptane ; or ethyl-2-methyl-pyrrolidine or (C4-7) cycloalkylamines including 3-substituted-N-methyl-cyclobutanamine and piperazine.

6. A compound according to claim 5, wherein R1 is 3-substituted-N-methyl-cyclobutanamine.

7. A compound according to to any of the preceding claims, wherein R2 is 1-substituted-4-aryl-piperazine or 1-substituted-4-heteroaryl-piperazine or 1-(4-substituted-aryl)piperazine or 1-(4-substituted-heteroaryl)piperazine or 1-substituted-4-aryl-piperidine or 1-substituted4-heteroaryl-piperidine or 1-(4-substituted-aryl)piperidine or 1-(4-substituted-heteroaryl)piperidine with optional substitution on aryl, heteroaryl, piperazine or piperidine groups.

8. A compound according to claim 7, wherein R2 is 1-substituted-4-aryl-piperazine or 1-substituted-4-heteroaryl-piperazine, 1-substituted-4-aryl-piperidine or 1-substituted-4-heteroaryl-piperidine.

9. A compound according to claim 1, wherein
- the central core A is 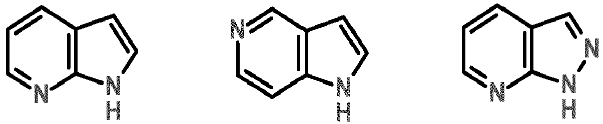
- R1 is ethyl-azabicyclo[3.2.0]heptane ; or ethyl-2-methyl-pyrrolidine or (C4-7) cycloalkylamines including 3-substituted-N-methyl-cyclobutanamine and piperazine;
- R2 is 1-substituted-4-aryl-piperazine or 1-substituted-4-heteroaryl-piperazine or 1-(4-substituted-aryl)piperazine or 1-(4-substituted-heteroaryl)piperazine or 1-substituted-4-aryl-piperidine or 1-substituted4-heteroaryl-piperidine or 1-(4-substituted-aryl)piperidine or 1-(4-substituted-heteroaryl)piperidine with optional substitution on aryl, heteroaryl, piperazine or piperidine groups.

10. A compound according to claim 9, wherein
- the central core A is 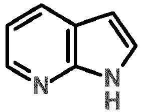
- R1 is 3-substituted-N-methyl-cyclobutanamine;
- R2 is 1-substituted-4-aryl-piperazine or 1-substituted-4-heteroaryl-piperazine, 1-substituted-4-aryl-piperidine or 1-substituted-4-heteroaryl-piperidine.

11. A compound according to any of the preceding claims selected from the group comprising:
6-[(2S)-2-methyl-4-(2-methylphenyl)piperazin-1-yl]-1-[2-(pyrrolidin-1-yl)ethyl]-1 H-pyrrolo[2,3-b]pyridine,
6-{4-[3-(methylsulfonyl)phenyl]piperazin-1-yl}-1-[2-(pyrrolidin-1-yl)ethyl]-1H-pyrrolo[2,3-b]pyridine,
6-[4-(4-acetylphenyl)piperazin-1-yl]-1-[trans-3-(methylamino)cyclobutyl]-1H-pyrrolo[2,3-b]pyridine-3-carbonitrile,
5-(4-{1-[2-(6-azabicyclo[3.2.0]hept-6-yl)ethyl]-1H-pyrrolo[2,3-b]pyridin-6-yl}piperazin-1-yl)imidazo[1,2-a]pyridine,
6-{4-[4-(methylsulfonyl)phenyl]piperazin-1-yl}-1-[2-(pyrrolidin-1-yl)ethyl]-1H-pyrrolo[2,3-b]pyridine-3-carbonitrile,
N-methyl-3-[6-[4-(4-pyridin-2-ylphenyl)piperazin-1-yl]pyrrolo[2,3-b]pyridin-1-yl]cyclobutan-1-amine,
N-methyl-3-[6-[4-(4-pyridin-2-ylphenyl)piperazin-1-yl]pyrrolo[2,3-b]pyridin-1-yl]cyclobutan-1-amine.

12. A compound according to any of claims 1 to 11 for use as a medicament.

13. A pharmaceutical composition comprising an effective amount of a compound according to claims 1 to 11 in combination with a pharmaceutically acceptable diluent or carrier.

14. A compound according to any of claims 1 to 11 for use in the treatment of inflammatory conditions driven by STING activation.

15. A synthesis intermediate, or an acid addition salt, a racemic mixture or its corresponding enantiomer and/or optical isomers thereof, of general formula II 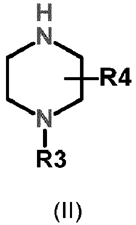 wherein
- R4 represents (C1-6)alkyl, (C1-6)alkoxy or oxo; and
- R3 represents optionally substituted aryl or heteroaryl.

16. A synthesis intermediate, or an acid addition salt, a racemic mixture or its corresponding enantiomer and/or optical isomers thereof, of general formula III 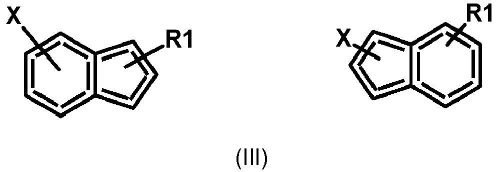 wherein
- X represents a halogen (Br, Cl, I) suitable for cross-coupling with intermediates of formula (II) or cross-coupling with an aryl or heteroaryl boronic acid derivative ; and
- R1 represents alkyl or cycloalkyl amines optionally substituted including (C1-3) aminoalkyl, (C3-7) aminocycloalkyl, (C1-3)alkylimidazole, (C1-3)alkyl isoindoline, (C1-3)alkylpiperazine, (C1-3)alkylpiperidine, (C1-3)alkyl imidazopiperazine, (C1-3)alky(C4-7)aminocycloalkyl, (C1-3)alky(C4-7)aminodicycloalkyl.

17. A synthesis intermediate, or an acid addition salt, a racemic mixture or its corresponding enantiomer and/or optical isomers thereof, of general formula IV 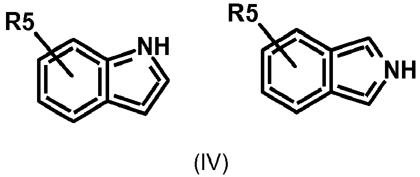
- wherein R5 can be either X or R2; X represents a halogen (Br, Cl, I) suitable for cross-coupling with intermediates of formula (II) or cross-coupling with an aryl or heteroaryl boronic acid derivative ; and R2 represents aryl, heteroaryl, heterobicyclic, (C4-7) aminocycloalkyl, cycloalkyl, heterocycloalkyl, (C6-8) diaminocycloalkyl, morpholino, (C4-7)cylcoalkylmethyl, piperzinyl, piperdinyl. R2 is optionally substituted with groups including hydroxyl, (C1-6)alkyl, acetyl, halogen, cyano, C1-6 alkyl, trifluoromethyl, difluromethyl, (C2-6) alkenyl, hydroxy, (C1-6) alkoxy, difluoromethoxy, trifluoromethoxy, trifluoroethoxy, (C1-6) alkylthio, (C1-6) alkylsulphonyl, amino, (C1-6) alkylamino, di(C1-6)alkylamino, (C1-6)alkoxy(C1-6)alkyl-amino, N-[(C1-6)alkyl]-N-[hydroxy(C1-6)alkyl]amino, (C2-6) alkylcarbonylamino, (C2-6) alkoxycarbonylamino, (C1-6) alkylsulphonylamino, formyl, (C2-6) alkylcarbonyl, carboxy, (C2-6) alkoxycarbonyl, aminocarbonyl, (C1-6) alkylaminocarbonyl, di(C1-6)alkylaminocarbonyl, aminosulphonyl, (C1-6) alkylaminosulphonyl or di(C1-6)alkylaminosulphonyl; (C3-7)heterocycloalkyl or (C3-7)spiroheterocycloalkyl, either of which groups may be optionally substituted by one or more substituents.

18. A synthesis intermediate, or an acid addition salt, a racemic mixture or its corresponding enantiomer and/or optical isomers thereof, of general formula V 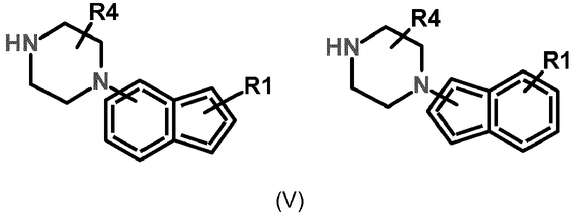 wherein
- R4 represents (C1-6)alkyl, (C1-6)alkoxy or oxo ; and
- R1 represents alkyl or cycloalkyl amines optionally substituted including (C1-3) aminoalkyl, (C3-7) aminocycloalkyl, (C1-3)alkylimidazole, (C1-3)alkyl isoindoline, (C1-3)alkylpiperazine, (C1-3)alkylpiperidine, (C1-3)alkyl imidazopiperazine, (C1-3)alky(C4-7)aminocycloalkyl, (C1-3)alky(C4-7)aminodicycloalkyl;

19. A synthesis intermediate, or an acid addition salt, a racemic mixture or its corresponding enantiomer and/or optical isomers thereof, of general formula VI 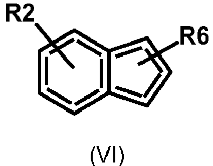 wherein
- R6 represents an alkyl or cycloalkyl substituent bearing a functional group suitable for displacement by an amine, for example 4-methylbenzenesulfonate; and
- R2 represents aryl, heteroaryl, heterobicyclic, (C4-7) aminocycloalkyl, cycloalkyl, heterocycloalkyl, (C6-8) diaminocycloalkyl, morpholino, (C4-7)cylcoalkylmethyl, piperzinyl, piperdinyl. R2 is optionally substituted with groups including hydroxyl, (C1-6)alkyl, acetyl, halogen, cyano, C1-6 alkyl, trifluoromethyl, difluromethyl, (C2-6) alkenyl, hydroxy, (C1-6) alkoxy, difluoromethoxy, trifluoromethoxy, trifluoroethoxy, (C1-6) alkylthio, (C1-6) alkylsulphonyl, amino, (C1-6) alkylamino, di(C1-6)alkylamino, (C1-6)alkoxy(C1-6)alkyl-amino, N-[(C1-6)alkyl]-N-[hydroxy(C1-6)alkyl]amino, (C2-6) alkylcarbonylamino, (C2-6) alkoxycarbonylamino, (C1-6) alkylsulphonylamino, formyl, (C2-6) alkylcarbonyl, carboxy, (C2-6) alkoxycarbonyl, aminocarbonyl, (C1-6) alkylaminocarbonyl, di(C1-6)alkylaminocarbonyl, aminosulphonyl, (C1-6) alkylaminosulphonyl or di(C1-6)alkylaminosulphonyl; (C3-7)heterocycloalkyl or (C3-7)spiroheterocycloalkyl, either of which groups may be optionally substituted by one or more substituents.

20. A synthesis intermediate, or an acid addition salt, a racemic mixture or its corresponding enantiomer and/or optical isomers thereof, of general formula VII 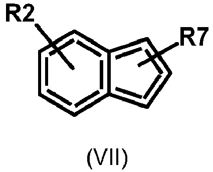 wherein
- R7 represents an primary or secondary amine ; and
- R2 represents aryl, heteroaryl, heterobicyclic, (C4-7) aminocycloalkyl, cycloalkyl, heterocycloalkyl, (C6-8) diaminocycloalkyl, morpholino, (C4-7)cylcoalkylmethyl, piperzinyl, piperdinyl. R2 is optionally substituted with groups including hydroxyl, (C1-6)alkyl, acetyl, halogen, cyano, C1-6 alkyl, trifluoromethyl, difluromethyl, (C2-6) alkenyl, hydroxy, (C1-6) alkoxy, difluoromethoxy, trifluoromethoxy, trifluoroethoxy, (C1-6) alkylthio, (C1-6) alkylsulphonyl, amino, (C1-6) alkylamino, di(C1-6)alkylamino, (C1-6)alkoxy(C1-6)alkyl-amino, N-[(C1-6)alkyl]-N-[hydroxy(C1-6)alkyl]amino, (C2-6) alkylcarbonylamino, (C2-6) alkoxycarbonylamino, (C1-6) alkylsulphonylamino, formyl, (C2-6) alkylcarbonyl, carboxy, (C2-6) alkoxycarbonyl, aminocarbonyl, (C1-6) alkylaminocarbonyl, di(C1-6)alkylaminocarbonyl, aminosulphonyl, (C1-6) alkylaminosulphonyl or di(C1-6)alkylaminosulphonyl; (C3-7)heterocycloalkyl or (C3-7)spiroheterocycloalkyl, either of which groups may be optionally substituted by one or more substituents.
